# EUROPEAN PATENT APPLICATION

(11) **EP 2 586 440 A2**
(43) Date of publication of application: **01.05.2013**
(21) Application number: 12158058.3
(22) Date of filing: 24.06.2003
(51) Int. Cl.: A61K 31/33, A61K 31/34, A61K 31/35, A61K 31/38, A61K 31/41, A61K 31/54, A61K 31/555

(54) **Methods for preventing and treating microbial infections by modulating transcription factors**

(30) Priority: 24.06.2002 US 391345 P; 25.10.2002 US 421218 P; 26.11.2002 US 429142 P; 31.03.2003 US 458935 P
(62) Divisional of application: 03742158.3
(71) Applicant: Paratek Pharmaceuticals, Inc., Boston, MA 02111 (US)
(72) Inventor: Alekshun, Michael, N., Marlboro, New Jersey 07746 (US); Levy, Stuart, B., Boston, Massachusetts 02116 (US)
(74) Representative: Nash, David Allan

(57) **Abstract**

The invention relates to the use of a compound that modulates the expression or activity of a microbial transcription factor in one of the following methods:
a method for preventing or treating infection of a subject by a microbe, which method comprises administering the compound to a subject at risk of developing an infection such that infection of the subject is prevented or treated;
a method for reducing virulence of a microbe, which method comprises administering the compound to a subject at risk of developing an infection with the microbe such that virulence of the microbe is reduced;
a method for reducing virulence in a microbe, which method comprises administering the compound to a subject having a microbial infection such that virulence of the microbe is reduced.

The invention also relates to a method comprising:
(a) infecting or inoculating a nonhuman animal with a microbe, wherein the ability of the microbe to establish an infection in the nonhuman animal requires that the microbe colonize the animal;
(b) administering a compound that modulates the expression or activity of the microbial transcription factor to the nonhuman animal; and
(c1) determining the level of infection of the nonhuman animal, wherein the ability of the compound to reduce the level of infection of the animal indicates that the compound is effective at inhibiting microbial virulence; or
(c2) determining the effect of the test compound on the ability of the microbe to colonize the animal, such that a compound for treating microbial infection is identified; or
(c3) determining the effect of the test compound on the ability of the microbe to colonize the animal, such that a compound for reducing microbial virulence is identified.

## Description

### Related Applications

This application claims priority to USSN 60/458,935, entitled "Methods for Preventing and Treating Microbial Infections by Modulating Transcription Factors," filed on March 31, 2003; USSN 60/429,142, entitled "Methods for Preventing and Treating Microbial Infections by Modulating Transcription Factors," filed on November 26,2002; USSN 60/421,218, entitled "Methods for Preventing and Treating Microbial Infections by Modulating Transcription Factors," filed on October 25, 2002; and USSN 60/391,345, entitled "Methods of Preventing and Treating Bacterial Infections by Inhibiting Virulance Factors," filed June 24, 2002. This application is also related to USSN 60/423,319, entitled "Transcription Factor Modulating Compounds and Method of Use Thereof," filed on November 1, 2002 and USSN 60/425,916, "Transcription Factor Modulating Compounds and Method of Use Thereof" filed on November 13, 2002. This application is also related to USSN 10/139,591, entitled "Transcription Factor Modulating Compounds and Methods of Use Thereof," filed on May 6, 2002. This application is also related to USSN 09/316,504, entitled "MarA Family Helix-Turn-Helix Domains and Their Methods of Use," filed on May 21, 1999. This application is also related to USSN 09/801,563, entitled "NIMR Compositions and Their Methods of Use," filed on March 8, 2001. The entire contents of these applications are hereby incorporated herein by reference.

### Background

Most antibiotics currently used and in development to treat bacterial infections impose selective pressure on microorganisms and have led to the development of widespread antibiotic resistance. Therefore, the development of an alternative approach to treating and/or preventing microbial infections would be of great benefit.

### Summary of the Invention

The instant invention identifies microbial transcription factors, e.g., transcription factors of the AraC-XylS family, as virulence factors in microbes and shows that inhibition of these factors reduces the virulence of microbial cells. Because these transcription factors control virulence, rather than essential cellular processes, the development of resistance to compounds that modulate the expression and/or activity of microbial transcription factors is much less likely.

Accordingly, in one aspect, the invention is directed to a method for preventing infection of a subject by a microbe comprising: administering a compound that modulates the expression and/or activity of a microbial transcription factor to a subject at risk of developing an infection such that infection of the subject is prevented.

In one embodiment, the transcription factor is a member of the AraC-XylS family of transcription factors.

In one embodiment, the transcription factor is a member of the MarA family of transcription factors.

In another embodiment, the method further comprises administering an antibiotic.

In another aspect, the invention pertains to a method for preventing urinary tract infection of a subject by a microbe comprising: administering a compound that modulates the expression and/or activity of a microbial transcription factor to a subject at risk of developing a urinary tract infection such that infection of the subject is prevented.

In yet another aspect, the invention pertains to a method for reducing virulence of a microbe comprising: administering a compound that modulates the expression and/or activity of a microbial transcription factor to a subject at risk of developing an infection with the microbe such that virulence of the microbe is reduced.

In one embodiment, the transcription factor is a member of the AraC-XylS family of transcription factors.

In another embodiment, the transcription factor is a member of the MarA family of transcription factors.

In yet another embodiment, the method further comprises administering an antibiotic.

In another aspect, the invention pertains to a method for treating a microbial infection in a subject comprising: administering a compound that modulates the expression and/or activity of a transcription factor to a subject having a microbial infection such that infection of the subject is treated.

In one embodiment, the transcription factor is a member of the AraC-Xy1S family of transcription factors.

In another embodiment, the transcription factor is a member of the MarA family of transcription factors.

In still another embodiment, the invention further comprises administering an antibiotic.

In another aspect, the invention pertains to a method for treating a urinary tract infection in a subject comprising: administering a compound that modulates the expression and/or activity of a transcription factor to a subject having a urinary tract infection such that infection of the subject is treated.

In one embodiment, the transcription factor is a member of the AraC-XylS family of transcription factors.

In one embodiment, the transcription factor is a member of the MarA family of transcription factors.

In another embodiment, the method further comprises administering an antibiotic.

In another aspect, the invention pertains to a method for reducing virulence in a microbe comprising: administering a compound that inhibits the expression and/or activity of a transcription factor to a subject having a microbial infection such that virulence of the microbe is reduced.

In one embodiment, the transcription factor is a member of the AraC-XylS family of transcription factors.

In another embodiment, the transcription factor is a member of the MarA family of transcription factors.

In yet another embodiment, the method further comprises administering an antibiotic.

In another aspect, the invention pertains to a method for evaluating the effectiveness of a compound that modulates the expression and/or activity of a microbial transcription factor at inhibiting microbial virulence comprising: infecting a non-human animal with a microbe, wherein the ability of the microbe to establish an infection in the non-human animal requires that the microbe colonize the animal; administering the compound that modulates the expression and/or activity of the microbial transcription factor to the non-human animal; and determining the level of infection of the non-human animal, wherein the ability of the compound to reduce the level of infection of the animal indicates that the compound is effective at inhibiting microbial virulence.

In one embodiment, the transcription factor is a member of the AraC-XylS family of transcription factors.

In another embodiment, the transcription factor is a member of the MarA family of transcription factors.

In yet another embodiment, the method further comprises administering an antibiotic.

In still another embodiment, the level of infection of the non-human animal is determined by measuring the ability of the microbe to colonize the tissue of the non-human animal.

In another embodiment, the level of infection of the non-human animal is determined by enumerating the number of microbes present in the tissue of the non-human animal.

In another aspect, the invention pertains to a method for identifying a compound for treating microbial infection, comprising: innoculating a non-human animal with a microbe, wherein the ability of the microbe to establish an infection in the non-human animal requires that the microbe colonize the animal; administering a compound which reduces the expression and/or activity of a microbial transcription factor to the animal, and determining the effect of the test compound on the ability of the microbe to colonize the animal, such that a compound for treating microbial infection is identified.

In one embodiment, the transcription factor is a member of the AraC-XylS family of transcription factors.

In another embodiment, the transcription factor is a member of the MarA family of transcription factors.

In still another embodiment, the level of infection of the non-human animal is determined by measuring the ability of the microbe to colonize the tissue of the non-human animal.

In another embodiment, the level of infection of the non-human animal is determined by enumerating the number of microbes present in the tissue of the non-human animal.

In another aspect, method for identifying a compound for reducing microbial virulence, comprising: inoculating a non-human animal with a microbe, wherein the ability of the microbe to establish an infection in the non-human animal requires that the microbe colonize the animal; administering a compound which reduces the expression and/or activity of a microbial transcription factor to the animal, and determining the effect of the test compound on the ability of the microbe to colonize the animal, such that a compound for reducing microbial virulence is identified.

In another embodiment, the transcription factor is a member of the AraC-XylS family of transcription factors.

In still another embodiment, the transcription factor is a member of the MarA family of transcription factors.

In yet another embodiment, the level of infection of the non-human animal is determined by measuring the ability of the microbe to colonize the tissue of the non-human animal.

In another embodiment, the level of infection of the non-human animal is determined by enumerating the number of microbes present in the tissue of the non-human animal.

In another aspect, the invention pertains to a method for identifying transcription factors which promote microbial virulence comprising: creating a microbe in which a transcription factor to be tested is misexpressed; introducing the microbe into a non-human animal; wherein the ability of the microbe to establish an infection in the non-human animal requires that the microbe colonize the animal; and determining the ability of the microbe to colonize the animal, wherein a reduced ability of the microbe to colonize the animal as compared to a wild-type microbial cell identifies the transcription factor as a transcription factor which promotes microbial virulence.

In another embodiment, the transcription factor is a member of the AraC-XylS family of transcription factors.

In another embodiment, the transcription factor is a member of the MarA family of transcription factors.

In another embodiment, the level of infection of the non-human animal is determined by measuring the ability of the microbe to colonize the tissue of the non-human animal.

In another embodiment, the level of infection of the non-human animal is determined by enumerating the number of microbes present in the tissue of the non-human animal.

In another aspect, the invention pertains to a method for reducing the ability of a microbe to adhere to an abiotic surface comprising: contacting the abiotic surface or the microbe with a compound that modulates the activity of a transcription factor such that the ability of the microbe to adhere to the abiotic surface is reduced.

In one embodiment, the transcription factor is a member of the AraC-XylS family of transcription factors.

In another embodiment, the transcription factor is a member of the MarA family of transcription factors.

In yet another embodiment, the method further comprises contacting the abiotic surface or the microbe with a second agent that is effective at controlling the growth of the microbe.

In still another embodiment, the abiotic surface is selected from the group consisting of: stents, catheters, and prosthetic devices.

In one aspect, the invention pertains to a pharmaceutical composition comprising a compound that modulates the activity or expression of a microbial transcription factor and a pharmaceutically acceptable carrier, wherein the compound reduces microbial virulence.

In another aspect, the invention pertains to a pharmaceutical composition comprising a compound that modulates the activity or expression of a microbial transcription factor and an antibiotic in a pharmaceutically acceptable carrier.

### Brief Description of the Figures

*Figures 1A-E* are a multiple sequence alignment of PROSITE PS01124 AraC family polypeptides.

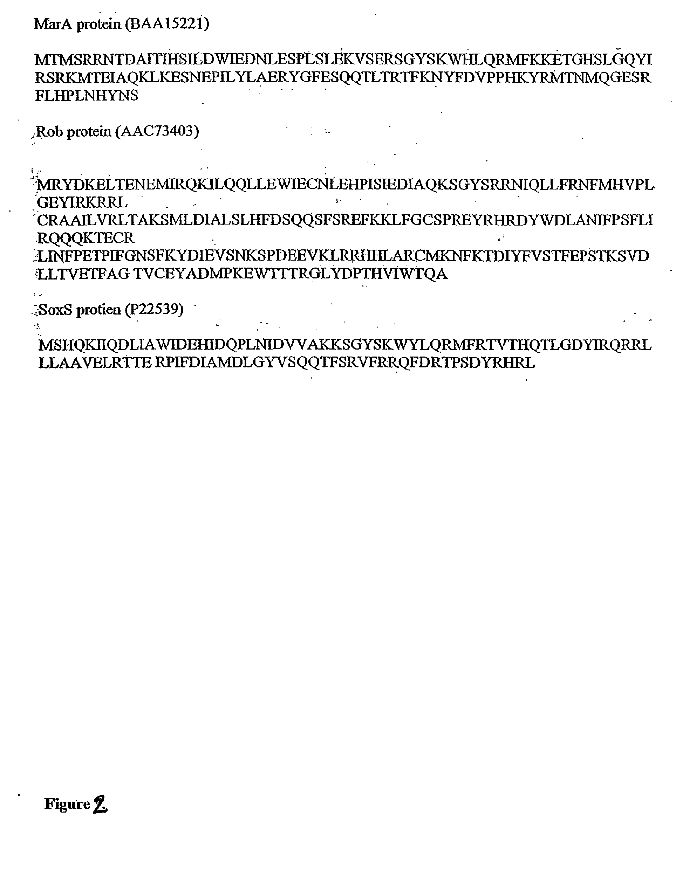

*Figure* 2 depicts the amino acid sequence of MarA, Rob, and SoxS from E. *coli* and the corresponding accession numbers.
*Figure* 3 depicts representative activities of a set of Mar inhibitors in a mobility shift assay. Lanes 1-6 all contain 0.1 nM (³³P)DNA and lanes 2-6 all contain 5 nM SoxS. Lanes 1 and 2, no compound; lanes 3-6, 50 µg/ml Compound A, Compound B, Compound C, and Compound D, respectively. Compound A and Compound B represent two different synthetic batches of the same compound. A, free DNA; B, SoxS-complex DNA.
*Figure 4* depicts the effects of a soxS, rob and marA deletion (triple knockout) from a clinical isolate on virulence in an ascending pyelonephritis infection model.
*Figure 5* depicts the effect of a single rob deletion from a clinical isolate and on restoring rob expression on virulence *in vivo* in an ascending pyelonephritis infection model.
*Figure* 6 depicts the effect of a single soxS deletion from a clinical isolate and on restoring soxS expression on virulence *in vivo* in an ascending pyelonephritis infection model as well as the effect of restoring marA expression in the triple knock out.
*Figure* 7 depicts the effect of soxS deletion from a clinical isolate on virulence *in vivo* in an ascending pyelonephritis infection model.
*Figure 8* depicts the effect of rob deletion from a clinical isolate on virulence *in vivo* in an ascending pyelonephritis infection model.
*Figures* 9A-B depict the virulence of multi-drug resistant E.coli in an ascending pylelonephritis mouse model of infection. Panel A depicts wild type KM-D E.coli and Panel B depicts E. Coli SRM which is isogenic but lacks *MarA, SoxS* and *rob.*
*Figure 10* depicts the activity of Compund 1 against E. coli C189 (a clinical cystitis isolate) in an ascending pyelonephritis mouse model.

### Detailed Description

The instant invention identifies microbial transcription factors, e.g., transcription factors of the AraC-XylS family, as virulence factors in microbes and shows that inhibition of these factors reduces the virulence of microbial cells. Because these transcription factors control virulence, rather than essential cellular processes, modulation of these factors should not promote resistance.

Some major families of transcription factors found in bacteria include the helix-turn-helix transcription factors (HTH) (Harrison, S. C., and A. K. Aggarwal 1990. Annual Review of Biochemistry. 59:933-969) such as AraC, MarA, Rob, SoxS and LysR; winged helix transcription factors (Gajiwala, K. S., and S. K. Burley 2000. 10:110-116), e.g., MarR, Sar/Rot family, and OmpR (Huffman, J. L., and R. G. Brennan 2002. Curr Opin Struct Biol. 12:98-106, Martinez-Hackert, E., and A. M. Stock 1997. Structure. 5:109-124); and looped-hinge helix transcription factors (Huffman, J. L., and R. G. Brennan 2002 Curr Opin Struct Biol. 12:98-106), e.g. the AbrB protein family.

The AraC-XylS family of transcription factors comprises many members. MarA, SoxS, Rma, and Rob are examples of proteins within the AraC-XylS family of transcription factors. These factors belong to a subset of the AraC-XylS family that have historically been considered to play roles in promoting resistance to multiple antibiotics and have not been considered to be virulence factors. In fact, the role of marA in virulence has been tested using a marA null mutant of Salmonella enterica serovar Typhimurium (S. typhimurium) in a mouse infection model (Sulavik et al. 1997. J. Bacteriology 179:1857) and no such role has been found. In another model (using co-infection experiments or crude statistics) only a weak effect of a marA null mutant in chickens has been demonstrated (Randall et al. 2001. J. Med. Microbiol. 50:770). In contrast to this earlier work, this invention is based, at least in part, on the finding that the ability of microbes to cause infection in a host can be inhibited by inhibiting the expression and/or activity of microbial transcription factors, e.g., the AraC-XylS family of transcription factors or MarA family of transcription factors. Thus, the instant invention validates the use of microbial transcription factors as therapeutic targets.

### I. Definitions

Before further description of the invention, certain terms employed in the specification, examples and appended claims are, for convenience, collected here.

As used herein, the term "modulates" includes both up- and down modulation.

As used herein, the term "infectivity" or "virulence" includes the ability of a pathogenic microbe to colonize a host, a first step required in order to establish growth in a host. Infectivity or virulence is required for a microbe to be a pathogen. In addition, a virulent microbe is one which can cause a severe infection. Exemplary virulence factors include: factors involved in outermembrane protein expression, microbial toxins, factors involved in biofilm formation, factors involved in carbohydrate transport and metabolism, factors involved in cell envelope synthesis, and factors involved in lipid metabolism.

As used herein, the term "pathogen" includes both obligate and opportunistic organisms. The ability of a microbe to resist antibiotics is also important in promoting growth in a host, however, in one embodiment, antibiotic resistance is not included in the terms "infectivity" or "virulence" as used herein. Accordingly, in one embodiment, the instant invention pertains to methods of reducing the infectivity or virulence of a microbe without affecting (e.g., increasing or decreasing) antibiotic resistance. Preferably, as used herein, the term "infectivity or virulence" includes the ability of an organism to establish itself in a host by evading the host's barriers and immunologic defenses.

The term "transcription factor" includes proteins that are involved in gene regulation in both prokaryotic and eukaryotic organisms. In one embodiment, transcription factors can have a positive effect on gene expression and, thus, may be referred to as an "activator" or a "transcriptional activation factor." In another embodiment, a transcription factor can negatively effect gene expression and, thus, may be referred to as "repressors" or a "transcription repression factor."

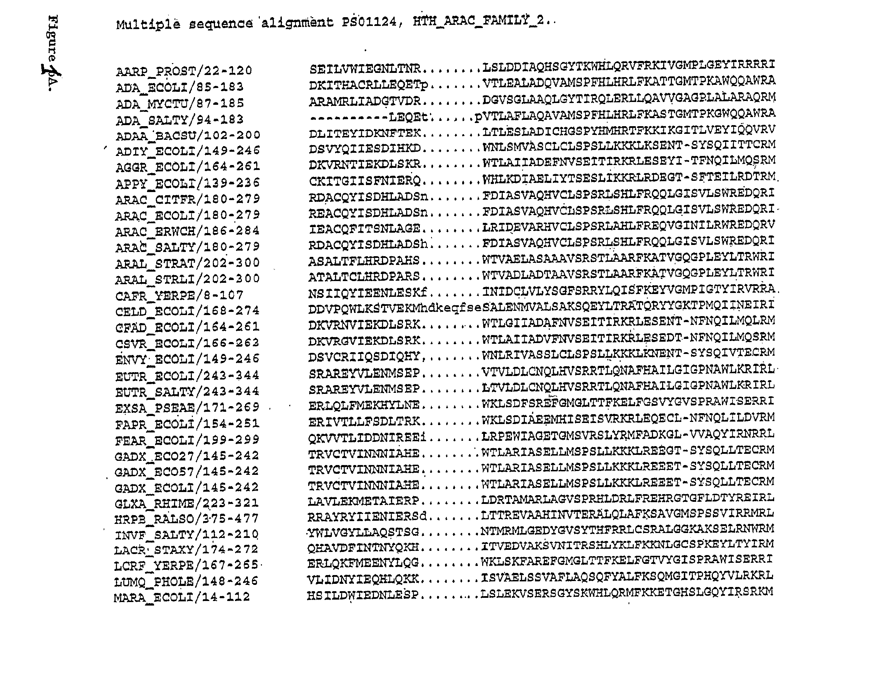

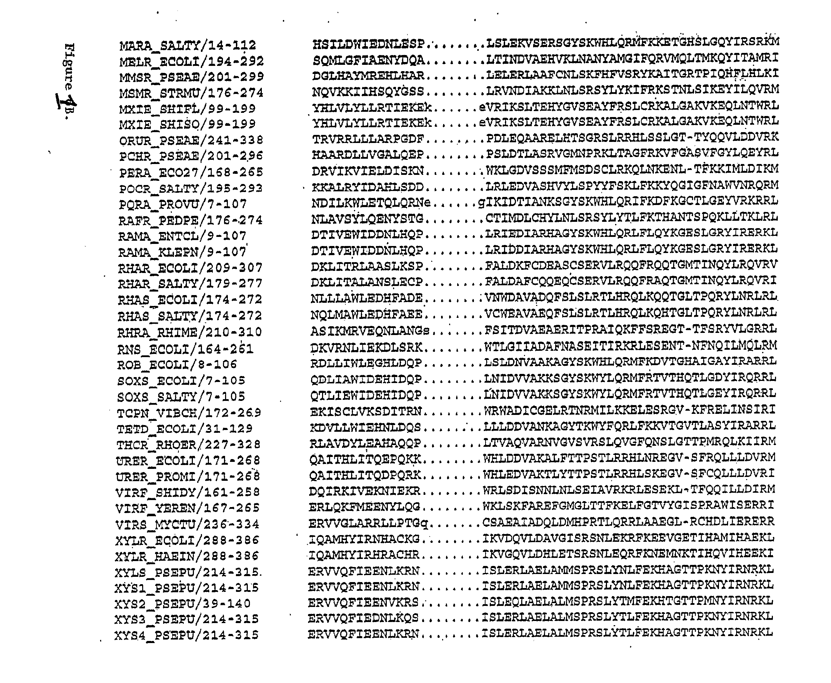

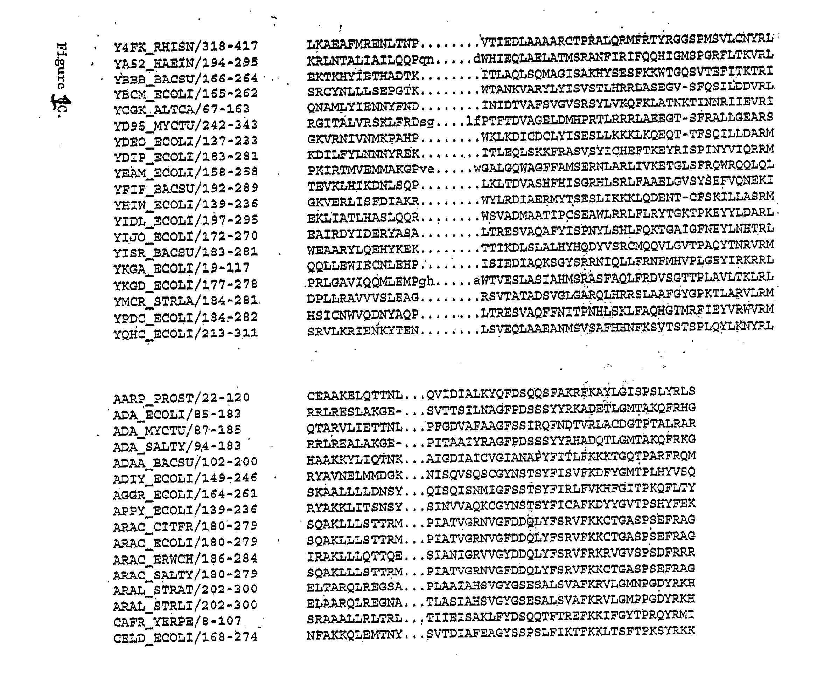

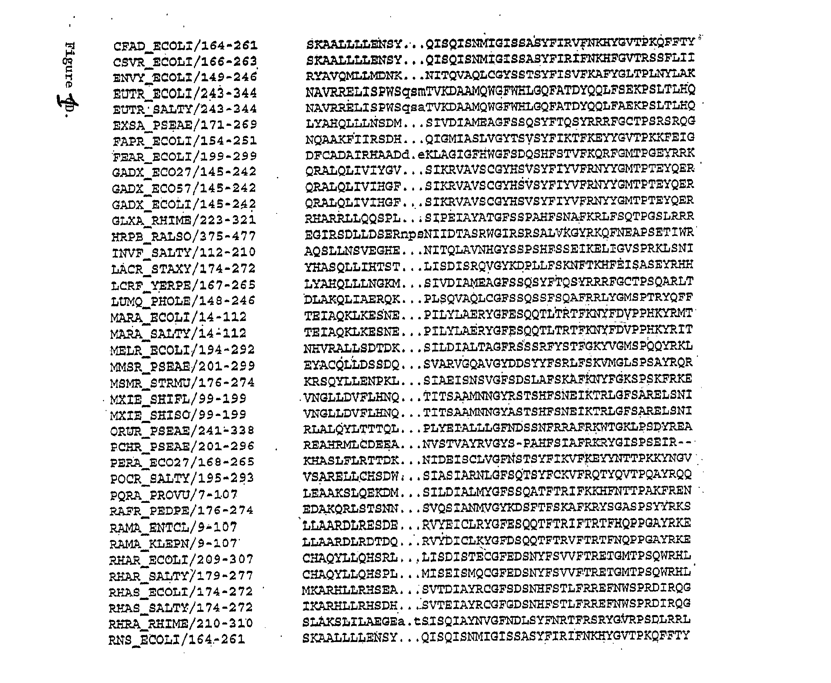

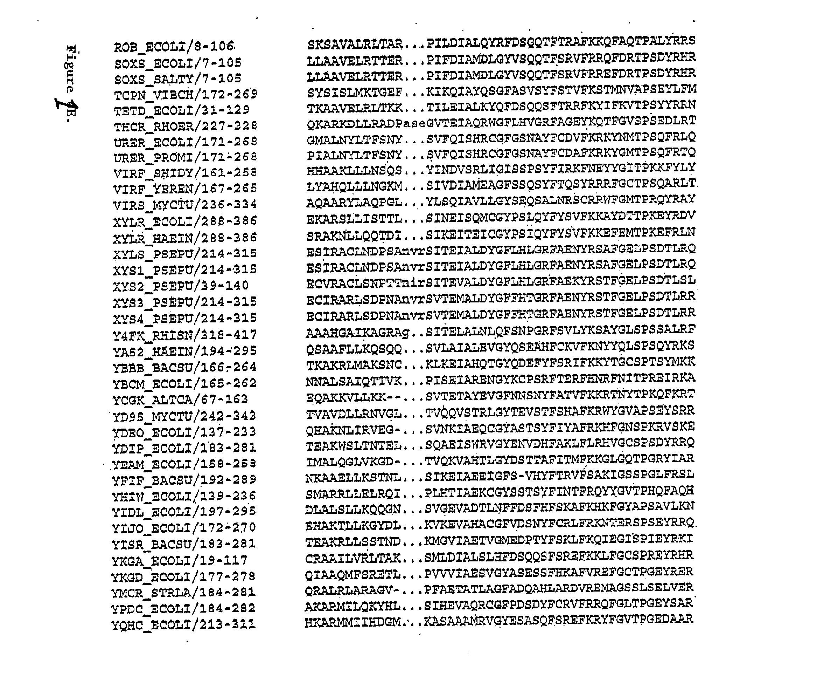

The term "AraC family polypeptide," "AraC-XylS family polypeptide" include an art recognized group of prokaryotic transcription factors which contains hundreds of different proteins (Gallegos et al., (1997) Micro. Mol. Biol. Rev. 61: 393; Martin and Rosner, (2001) Curr. Opin. Microbiol. 4:132). AraC family polypeptides include proteins defined in the PROSITE (PS) database as profile PS01124. The AraC family polypeptides also include polypeptides described in PS0041, HTH AraC Family 1, and PS01124, and HTH AraC Family 2. Multiple sequence alignments for exemplary AraC-XylS family polypeptides are shown in Figure 1. Exemplary AraC family polypeptides are also shown in Table 1. In an embodiment, the AraC family polypeptides are generally comprised of, at the level of primary sequence, a conserved stretch of about 100 amino acids, which are believed to be responsible for the DNA binding activity of these proteins (Gallegos et al., (1997) Micro. Mol. Biol. Rev. 61: 393; Martin and Rosner, (2001) Curr. Opin. Microbiol. 4: 132). AraC family polypeptides also may include two helix turn helix DNA binding motifs (Martin and Rosner, (2001) Curr. Opin. Microbiol. 4: 132; Gallegos et al., (1997) Micro. Mol. Biol. Rev. 61: 393; Kwon et al., (2000) Nat. Struct. Biol. 7: 424; Rhee et al., (1998) Proc. Natl. Acad. Sci. U.S.A. 95: 10413). The term includes MarA family polypeptides and HTH proteins.

An exemplary signature pattern which defines the AraC family polypeptides is shown, e.g., on PROSITE and is represented by the sequence: [KRQ]-[LIVMA]-X(2)-[GSTALIV]-{FYWPGDN}X(2)-[LIVMSA]-X(4,9)-[LIVMF]-X(2)-[LIVMSTA]-X(2)-[GSTACIL]-X(3)-[GANQRF]-[LIVMFY]-X(4,5)-[LFY]-X(3)-[FYIVA]-{FYWHCM}-X(3)-[GSADENQKR]-X-[NSTAPKL]-[PARL], where X is any amino acid.

In one embodiment, the invention pertains to a method for modulating an AraC family polypeptide, by contacting the AraC family polypeptide with a test compound which interacts with a portion of the polypeptide involved in DNA binding. Transcription factors of the AraC family can be active as monomers or dimers. In one embodiment, a transcription factor of the invention belongs to the AraC family and is active as a monomer. In another embodiment, a transcription factor of the invention belongs to the AraC family and is active as a dimer.

In one embodiment, a transcription factor of the instant invention excludes one or more of: VirF (LcrF), V38K, BvgA/BvgS, PhoP/PhoQ, EnvZ/OmpR, ToxR/ToxS, ToxT, AggR, ExsA, PerA, RNS, LysR, SpvR, IrgB, LasR, SdiA, VirB, AlgR, or LuxR.

AraC family members belong to a larger group of transcription factors which comprise helix-turn-helix domains. "Helix-turn-helix domains" are known in the art and have been implicated in DNA binding (Ann Rev. of Biochem. 1984. 53:293). An example of the consensus sequence for a helix-turn domain can be found in Brunelle and Schleif (1989. J. Mol. Biol. 209:607). The domain has been illustrated by the sequence XXXPhoAlaXXPhoGlyPhoXXXXPhoXXPhoXX, where X is any amino acid and Pho is a hydrophobic amino acid.

The helix-turn-helix domain was the first DNA-binding protein motif to be recognized. Although originally the HTH domain was identified in bacterial proteins, the HTH domain has since been found in hundreds of DNA-binding proteins from both eukaryotes and prokaryotes. It is constructed from two alpha helices connected by a short extended chain of amino acids, which constitutes the "turn." In one embodiment, a transcription factor of the invention comprises at least one helix-turn-helix domain.

In one embodiment, a transcription factor of the invention is a Mar A family polypeptide. The language "MarA family polypeptide" includes the many naturally occurring HTH proteins, such as transcription regulation proteins which have sequence similarities to MarA and which contain the AraC signature pattern. MarA family polypeptides have two "helix-turn-helix" domains. This signature pattern was derived from the region that follows the first, most amino terminal, helix-turn-helix domain (HTH1) and includes the totality of the second, most carboxy terminal helix-turn-helix domain (HTH2). (See PROSITE PS00041).

The MarA family of proteins ("MarA family polypeptides") represent one subset of AraC-XylS family polypeptides and include proteins like MarA, SoxS, Rob, Rma, AarP, PqrA, etc. The MarA family polypeptides, generally, are involved in regulating resistance to antibiotics, organic solvents, and oxidative stress agents (Alekshun and Levy, (1997) Antimicrob. Agents. Chemother. 41: 2067). Like other AraC-XylS family polypeptides, MarA-like proteins also generally contain two HTH motifs as exemplified by the MarA and Rob crystal structures (Kwon et al., (2000) Nat. Struct. Biol. 7: 424; Rhee et al., (1998) Proc. Natl. Acad. Sci. U.S.A. 95: 10413). Members of the MarA family can be identified by those skilled in the art and will generally be represented by proteins with homology to amino acids 30-76 and 77-106 of MarA (SEQ ID. NO. 1).

Preferably, a MarA family polypeptide or portion thereof comprises a first MarA family HTH domain (HTH1) (Brunelle, 1989, J Mol Biol; 209(4):607-22). In another embodiment, a MarA polypeptide comprises the second MarA family HTH domain (HTH2) (Caswell, 1992, Biochem J.; 287:493-509.). In a preferred embodiment, a MarA polypeptide comprises both the first and second MarA family HTH domains.

Exemplary MarA family polypeptides are shown, e.g., in Table 2, Figure 1, and at Prosite (PS00041) and include, e.g.,: AarP, Ada, AdaA, AdiY, AfrR, AggR, AppY, AraC, CfaR, CelD, CfaD, CsvR, D90812, EnvY, ExsA, FapR, HrpB, InF, InvF, LcrF, LumQ, MarA, MelR, MixE, MmsR, MsmR, OrfR, Orf_f375, PchR, PerA, PocR, PqrA, RafR, RamA, RhaR, RhaS, Rns, Rob, SoxS, S52856, TetD, TcpN, ThcR, TmbS, U73857, U34257, U21191, UreR, VirF, XylR, XylS, Xys1, 2, 3, 4, Ya52, YbbB, YfiF, YisR, YzbC, YijO, BfaA, PerA, ctxA, YbtA, VirF (LcrF), V38K, BvgA/BvgS, PhoP/PhoQ, EnvZ/OmpR, ToxR/ToxS, ToxT, AggR, ExsA, PerA, RNS, LysR, SpvR, IrgB, LasR, SdiA, VirB, AlgR, LuxR, BfpT, GadX, MxiE, CfaR, fapR, CsvR, Rns, invF, Hi1C, SprA, SirC, HilD, VC1825, or VCA0231.

In particularly preferred embodiments, a MarA family polypeptide is selected from the group consisting of: MarA, RamA, AarP, Rob, SoxS, and PqrA. The nucleotide and amino acid sequences of the *E. coli* Rob molecule are shown in SEQ ID NO:3 and 4, respectively.

**TABLE 2. Some Bacterial MarA homologs^{a}**

| Gram-negative bacteria | | Gram-positive bacteria |
|---|---|---|
| *Escherichia coli* | *Kiebsiella pneumoniae* | *Lactobacillus helveticus* |
| **MarA (1)** | **RamA (27)** | **U34257 (38)** |
| **OrfR (2, 3)** | | |
| **SoxS (4, 5)** | *Haemophilus influenzae* | *Azorhizobium caulinodans* |
| AfrR (6) | Ya52 (28) | S52856 (39) |
| AraC (7) | | |
| CelD (8) | *Yersinia spp.* | *Streptomyces spp.* |
| D90812 (9) | CafR (29) | **U21191 (40)** |
| FapR (10, 11) | LcrF (30) or VirF (30) | AraL (41) |
| MelR (12) | | |
| ORF *ƒ*375 (13, 14) | *Providencia stuartii* | *Streptococcus mutans* |
| RhaR (15, 16, 17) | **AarP (31)** | MsmR (42) |
| RhaS (18) | | |
| Rob (19) | *Pseudomonas spp.* | *Pediococcus pentosaceus* |
| U73857 (20) | MmsR (32) | RafR (43) |
| XylR (21) | TmbS (33) | |
| YijO (22) | XylS (34) | *Photobacterium leiognathi* |
| | Xys1,2,3,4 (35, 36) | LumQ (44) |
| *Proteus vulgaris* | | |
| **PqrA (23)** | Cyanobacteria | *Bacillus subtilis* |
| | *Synechocystis* spp. | AdaA (45) |
| *Salmonella Typhimurium* | **LumQ** (37) | YbbB (46) |
| **MarA (24)** | PchR (37) | YfiF (47) |
| InvF (25) | | YisR (48) |
| PocR (26) | | YzbC (49) |

| | | |
|---|---|---|
| ^{a} The smaller MarA homologs, ranging in size from 87 (U34257) to 138 (OrfR) amino acid residues, are represented in boldface. References are given in parentheses and are listed below. | | |

### References for Table 2:

(1) S.P. Cohen, et al. 1993. J. Bacteriol. 175:1484-1492
(2) G.M. Braus, et al. 1984. J. Bacteriol. 160:504-509
(3) K. Schollmeier, et al., 1984. J. Bacteriol. 160:499-503
(4) C.F. Amabile-Cuevas, et al., 1991. Nucleic Acids Res. 19:4479-4484
(5) J. Wu, et al., 1991. J. Bacteriol. 173:2864-2871
(6) M.K. Wolf, et al., 1990. Infect. Immun. 58:1124-1128
(7) C.M. Stoner, et al. 1982. J. Mol. Biol. 153:649-652
(8) L.L. Parker, et al., 1990. Genetics 123:455-471
(9) H. Mori, 1996. Unpublished data taken from the NCBI databases
(10) P. Klaasen, et al., 1990. Mol. Microbiol. 4:1779-1783
(11) M. Ahmed, et al., 1994. J. Biol. Chem 269-28506-28513
(12) C. Webster, et al., 1989. Gene 83:207-213
(13) G. Plunkett, III. 1995. Unpublished
(14)C Garcia-Martin, et al., 1992. J. Gen. Microbiol. 138:1109-1116
(15) G. Plunkett, III., et al. 1993. Nucleic Acids Res. 21:3391-3398
(16) C. G. Tate, et al. 1992. J. Biol. Chem. 267:6923-6932
(17) J.F. Tobin et al., 1987. J. Mol. Biol. 196:789-799
(18) J. Nishitani, 1991. Gene 105:37-42
(19) R.E. Benz, et al., 1993. Zentralbl. Bakteriol. Parasitenkd. Infektionskr. Hyg. Abt.1 Orig. 278:187-196
(20) M. Duncan, *et al.,* 1996. Unpublished data
(21) H.J. Sofia, et al., 1994. Nucleic Acids Res. 22:2576-2586
(22) F.R. Blattner, et al., 1993. Nucleic Acids Res. 21:5408-5417
(23) H. Ishida, et al., 1995. Antimicrob. Agents Chemother. 39:453-457
(24) M.C. Sulavik, et al., 1997. J. Bacteriol. 179:1857-1866
(25) K. Kaniga, et al., 1994. Mol. Microbiol. 13:555-568
(26) J.R. Roth, et al. 1993. J. Bacteriol. 175:3303-3316
(27) A.M. George, et al., 1983. J. Bacteriol. 155:541-548
(28) R.D. Fleischmann, et al., 1995. Science 269:469-512
(29) E.E. Galyov, et al., 1991. FEBS Lett. 286:79-82
(30) N.P. Hoe, et al., 1992. J. Bacteriol. 174:4275-4286
(31) G. Cornelis, et al., 1989. J. Bacteriol. 171:254-262
(32) D.R. Macinga, et al., 1995. J. Bacteriol. 177:3407-3413
(33) M.I. Steele, et al., 1992. J. Biol. Chem. 267:13585-13592
(34) G. Deho, *et al.,* 1995. Unpublished data
(35) N. Mermod, et al., 1984. EMBO J. 3:2461-2466
(36) S.J. Assinder, et al., 1992. Nucleic Acids Res. 20:5476
(37) S.J. Assinder, et al., 1993. J. Gen. Microbiol. 139:557-568
(38) E.G. Dudley, et al., 1996. J. Bacteriol. 178:701-704
(39) D. Geelen, *et al.,* 1995. Unpublished data
(40) J. Konnanec, et al., 1995. Gene 165:77-80
(41) C.W. Chen, et al., 1992. J. Bacteriol. 174:7762-7769
(42) R.R. Russell, et al., 1992. J. Biol. Chem, 267:4631-4637
(43) K.K. Leenhouts, *et al.,* 1995. Unpublished data
(44) J.W. Lin, et al., 1995. Biochem. Biophys. Res. Commun. 217:684-695
(45) F. Morohoshi, et al. 1990. Nucleic Acids Res. 18:5473-5480
(46) M. Rosenberg, et al., 1979. Annu. Rev. Genet. 13:319-353
(47) H. Yamamoto, et al., 1996. Microbiology 142:1417-1421
(48) L.B. Bussey, et al., 1993. J. Bacteriol. 175:6348-6353
(49) P.G. Quirk, et al., 1994. Biochim. Biophys. Acta 1186:27-34

Members of transcription factor families share common properties, e.g., certain structural and functional characteristics are shared among the family members. Accordingly, it will be understood by one of ordinary skill in the art that the structural relatedness inquiries described below (e.g., based on primary nucleic acid or amino acid sequence homology (or on the presence of certain signature domains) or on hybridization as an indicator of such nucleic acid homology), or based on three-dimensional correspondence of amino acids) can be used to identify members of the various transcription factor families.

Transcription factors belonging to particular families are "structurally related" to one or more known family members, e.g., members of the MarA family of transcription factors are structurally related to MarA. This relatedness can be shown by sequence or structural similarity between two polypeptide sequences or between two nucleotide sequences that specify such polypeptides. Sequence similarity can be shown, e.g., by optimally aligning sequences using an alignment program for purposes of comparison and comparing corresponding positions. To determine the degree of similarity between sequences, they will be aligned for optimal comparison purposes (e.g., gaps may be introduced in the sequence of one protein for nucleic acid molecule for optimal alignment with the other protein or nucleic acid molecules). The amino acid residues or bases and corresponding amino acid positions or bases are then compared. When a position in one sequence is occupied by the same amino acid residue or by the same base as the corresponding position in the other sequence, then the molecules are identical at that position. If amino acid residues are not identical, they may be similar. As used herein, an amino acid residue is "similar" to another amino acid residue if the two amino acid residues are members of the same family of residues having similar side chains. Families of amino acid residues having similar side chains have been defined in the art (see, for example, Altschul et al. 1990. J. Mol. Biol. 215:403) including basic side chains (e.g., lysine, arginine, histidine), acidic side chains (*e.g.,* aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan). The degree (percentage) of similarity between sequences, therefore, is a function of the number of identical or similar positions shared by two sequences (i.e., % homology = # of identical or similar positions/total # of positions x 100). Alignment strategies are well known in the art; see, for example, Altschul et al. *supra* for optimal sequence alignment.

Transcription factors belonging to certain families may also share some amino acid sequence similarity with a known member of that family. The nucleic acid and amino acid sequences of exemplary members of transcription factor are available in the art. For example, the nucleic acid and amino acid sequence of MarA can be found, e.g., on GeneBank (accession number M96235 or in Cohen et al. 1993. J. Bacteriol. 175:1484, or in SEQ ID NO:1 and SEQ ID NO:2.

The nucleic acid and/or amino acid sequences of a known member of a transcription factor family can be used as "query sequences" to perform a search against databases (e.g., either public or private) to, for example, identify other family members having related sequences. Such searches can be performed, e.g., using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to MarA family nucleic acid molecules. BLAST protein searches can be performed with the XBLAST program, score = 50, wordlength = 3 to obtain amino acid sequences homologous to transcription factors of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (e.g., XBLAST and NBLAST) can be used.

Transcription factor family members can also be identified as being similar based on their ability to specifically hybridize to nucleic acid sequences specifying a known member of a transcription factor family. Such stringent conditions are known to those skilled in the art and can be found *e.g.,* in Current Protocols in Molecular Biology, John Wiley & Sons, N.Y. (1989), 6.3.1-6.3.6. A preferred, non-limiting example of stringent hybridization conditions are hybridization in 6X sodium chloride/sodium citrate (SSC) at about 45°C, followed by one or more washes in 0.2 X SSC, 0.1% SDS at 50-65°C. Conditions for hybridizations are largely dependent on the melting temperature Tm that is observed for half of the molecules of a substantially pure population of a double-stranded nucleic acid. Tm is the temperature in °C at which half the molecules of a given sequence are melted or single-stranded. For nucleic acids of sequence 11 to 23 bases, the Tm can be estimated in degrees C as 2(number of A+T residues) + 4(number of C+G residues). Hybridization or annealing of nucleic acid molecules should be conducted at a temperature lower than the Tm, e.g., 15°C, 20°C, 25°C or 30°C lower than the Tm. The effect of salt concentration (in M of NaCl) can also be calculated, see for example, Brown, A., "Hybridization" pp. 503-506, in The Encyclopedia of Molec. Biol., J. Kendrew, Ed., Blackwell, Oxford (1994).

Preferably, the nucleic acid sequence of a transcription factor family member identified in this way is at least about 10%, 20%, more preferably at least about 30%, more preferably at least about 40% identical and preferably at least about 50%, or 60% identical to a query nucleotide sequence. In preferred embodiments, the nucleic acid sequence of a family member is at least about 70%, 80%, preferably at least about 90%, more preferably at least about 95% identical with a query nucleotide sequence. Preferably, family members have an amino acid sequence at least about 20%, preferably at least about 30%, more preferably at least about 40% identical and preferably at least about 50%, or 60% or more identical with a query amino acid sequence. In preferred embodiments, the nucleic acid sequence of a family member is at least about 70%, 80%, more preferably at least about 90%, or more preferably at least about 95% identical with a query nucleotide sequence.

However, it will be understood that the level of sequence similarity among microbial regulators of gene transcription, even though members of the same family, is not necessarily high. This is particularly true in the case of divergent genomes where the level of sequence identity may be low, *e.g.,* less than 20% *(e.g., B. burgdorferi* as compared e.g., to *B. subtilis*)*.* Accordingly, structural similarity among transcription factor family members can also be determined based on "three-dimensional correspondence" of amino acid residues. As used herein, the language "three-dimensional correspondence" is meant to includes residues which spatially correspond, *e.g.,* are in the same position of a known transcription factor family member as determined, *e.g.,* by x-ray crystallography, but which may not correspond when aligned using a linear alignment program. The language "three-dimensional correspondence" also includes residues which perform the same function, *e.g.,* bind to DNA or bind the same cofactor, as determined, *e.g.,* by mutational analysis. Such analysis can be performed using comparison programs that are publicly available.

The term "transcription factor modulating compound" or transcription factor modulator" includes compounds which modulate transcription, i.e., which affect the expression and/or activity of one or more transcription factors, such that the expression and/or activity of the transcription factor is modulated, e.g., enhanced or inhibited. The term includes e.g., AraC family modulating compounds, winged helix modulating compounds, looped-hinge helix modulating compounds and MarA family modulating compounds. In one embodiment, the transcription factor modulating compound is an inhibiting compound of a microbial transcription factor, e.g., a prokaryotic transcription factor or a eukaryotic transcription activation factor. In another embodiment, the modulating compound preferentially modulates a transcription factor present in a microbial cell, while not modulating a transcription factor in a host organism harboring the microbial cell. In one embodiment, the modulating compound modulates a prokaryotic transcription factor and not a eukaryotic transcription factor. Exemplary eukaryotic cell transcription factors are taught in the art (e.g., Warren. 2002. Current Opinion in Structural Biology. 12:107).

In one embodiment, a compound is an HTH protein modulating compound. The term "HTH protein modulating compound" or "HTH protein modulator" includes compounds which interact with one or more proteins comprising an HTH domain such that the activity of the HTH protein is modulated, e.g., enhanced or, inhibited. In one embodiment, the HTH protein modulating compound is a MarA family polypeptide modulating compound. In one embodiment, the activity of the HTH protein is enhanced when it interacts with the HTH protein modulating compound. In a preferred embodiment, the activity of the HTH protein is decreased upon an interaction with the HTH protein modulating compound. Values and ranges included and/or intermediate of the values set forth herein are also intended to be within the scope of the present invention.

The term "MarA family polypeptide modulating compound" or "MarA family modulating compound" include compounds which interact with one or more MarA family polypeptides such that the activity of the MarA family peptide is enhanced or inhibited, preferably inhibited. In an embodiment, the MarA family polypeptide modulating compound is an inhibiting compound. In a further embodiment, the MarA family inhibiting compound is an inhibitor of MarA, Rob, and/or SoxS.

The term "polypeptide(s)" refers to a peptide or protein comprising two or more amino acids joined to each other by peptide bonds or modified peptide bonds. "Polypeptide(s)" includes both short chains, commonly referred to as peptides, oligopeptides and oligomers and longer chains generally referred to as proteins. Polypeptides may contain amino acids other than the 20 gene encoded amino acids. "Polypeptide(s)" include those modified either by natural processes, such as processing and other post-translational modifications, but also by chemical modification techniques. Such modifications are well described in basic texts and in more detailed monographs, as well as in a voluminous research literature, and they are well known to those of skill in the art. It will be appreciated that the same type of modification may be present in the same or varying degree at several sites in a given polypeptide. Also, a given polypeptide may contain many types of modifications.

Modifications can occur anywhere in a polypeptide, including the peptide backbone, the amino acid side-chains, and the amino or carboxyl termini. Modifications include, for example, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, crosslinking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, glycosylation, lipid attachment, sulfation, gamma-carboxylation of glutamic acid residues, hydroxylation and ADP-ribosylation, selenoylation, sulfation, transfer-RNA mediated addition of amino acids to proteins, such as arginylation, and ubiquitination. See, for instance, Proteins--Structure And Molecular Properties, 2nd Ed., T. E. Creighton, W. H. Freeman and Company, New York (1993) and Wold, F., Posttranslational Protein Modifications: Perspectives and Prospects, pgs. 1-12 in Posttranslational Covalent Modification Of Proteins, B. C. Johnson, Ed., Academic Press, New York (1983); Seifter et al., Meth. Enzymol. 182:626-646 (1990) and Rattan et al., Protein Synthesis: Posttranslational Modifications and Aging, Ann. N.Y. Acad. Sci. 663: 48-62 (1992). Polypeptides may be branched or cyclic, with or without branching. Cyclic, branched and branched circular polypeptides may result from post-translational natural processes and may be made by entirely synthetic methods, as well.

As used herein, the term "winged helix" includes dimeric transcription factors in which each monomer comprises a helix-turn-helix motif followed by one or two β-hairpin wings (Brennan. 1993. Cell. 74:773; Gajiwala and Burley. 2000. Curr. Opin. Struct. Biol. 10:110). The classic winged helix motif comprises two wings, three α helices, and three β strands in the sequence H1-B1-H2-T-H3-B2-W 1-B3-W2 (where H is a helix, B is a β strand, T is a turn, and W is a wing), although some variation in structure has been demonstrated (Huffman and Brennan. 2002. Current Opinion in Structural Biology. 12:98).

As used herein the term "looped-hinge helix" included transcription factors, such as AbrB which, in the absence of DNA, have revealed a dimeric N-terminal region consisting of a four-stranded β sheet and a C-terminal DNA-binding region comprising one α helix and a "looped hinge" (see, e.g., Huffman and Brennan. 2002 Current Opinion in Structural Biology 12:98). Residues corresponding to R23 and R24 of AbrB are critical for DNA recognition and contribute to the electropositive nature of the DNA-binding region.

Preferred polypeptides (and the nucleic acid molecules that encode them) are "naturally occurring." As used herein, a "naturally-occurring" molecule refers to a molecule having an amino acid or a nucleotide sequence that occurs in nature (*e.g*., a natural polypeptide). In addition, naturally or non-naturally occurring variants of the polypeptides and nucleic acid molecules which retain the same functional activity, (such as, the ability to bind to target nucleic acid molecules (*e.g*., comprising a marbox) or to polypeptides (*e.g*. RNA polymerase) with a naturally occurring polypeptide are provided for and can be used in the instant assays. Such immunologic cross-reactivity can be demonstrated, *e.g.,* by the ability of a variant to bind to a transcription factor responsive element. Such variants can be made, *e.g.,* by mutation using techniques that are known in the art. Alternatively, variants can be chemically synthesized.

As used herein the term "variant(s)" includes nucleic acid molecules or polypeptides that differ in sequence from a reference nucleic acid molecule or polypeptide, but retain its essential properties. Changes in the nucleotide sequence of the variant may, or may not, alter the amino acid sequence of a polypeptide encoded by the reference nucleic acid molecule. Nucleotide or amino acid changes may result in amino acid substitutions, additions, deletions, fusions and truncations in the polypeptide encoded by a naturally occurring reference sequence. A typical variant of a polypeptide differs in amino acid sequence from a reference polypeptide. Generally, differences are limited so that the sequences of the reference polypeptide and the variant are closely similar overall and, in many regions, identical. A variant and reference polypeptide may differ in amino acid sequence by one or more substitutions, additions, and/or deletions in any combination.

A variant of a nucleic acid molecule or polypeptide may be naturally occurring, such as an allelic variant, or it may be a variant that is not known to occur naturally. Non-naturally occurring variants of nucleic acid molecules and polypeptides may be made from a reference nucleic acid molecule or polypeptide by mutagenesis techniques, by direct synthesis, and by other recombinant methods known to skilled artisans. Alternatively, variants can be chemically synthesized. For instance, artificial or mutant forms of autologous polypeptides which are functionally equivalent, (*e.g*., have the ability to interact with a transcription factor responsive element) can be made using techniques which are well known in the art.

Mutations can include, *e.g.,* at least one discrete point mutation which can give rise to a substitution, or by at least one deletion or insertion. For example, mutations can also be made by random mutagenesis or using cassette mutagenesis. For the former, the entire coding region of a molecule is mutagenized by one of several methods (chemical, PCR, doped oligonucleotide synthesis) and that collection of randomly mutated molecules is subjected to selection or screening procedures. In the latter, discrete regions of a polypeptide, corresponding either to defined structural or functional determinants are subjected to saturating or semi-random mutagenesis and these mutagenized cassettes are re-introduced into the context of the otherwise wild type allele. In one embodiment, PCR mutagenesis can be used. For example, Megaprimer PCR can be used (O.H. Landt, 1990. Gene 96:125-128).

The language "activity of a transcription factor" includes the ability of a transcription factor to interact with DNA, *e.g.,* to bind to a transcription factor responsive promoter, or to initiate transcription from such a promoter.

The language "activity of a MarA family polypeptide" includes the ability of the MarA family polypeptide to interact with DNA, *e.g.,* to bind to a MarA family polypeptide responsive promoter, or to initiate transcription from such a promoter. MarA functions both as a transcriptional activator (*e.g*., upregulating genes such as inaA, galT, micF, etc.) and as a repressor (*e.g*., downregulating genes such as fecA, purA, guaB, etc.) (Alekshun, 1997, Antimicrob. Agents Chemother. 41:2067-2075; Barbosa & Levy, J. Bact. 2000, Vol. 182, p. 3467-3474; Pomposiello et al. J. Bact. 2001, Vol 183, p. 3890-3902).

The language "transcription factor responsive element" includes a nucleic acid sequence which can interact with a transcription factor (e.g., promoters or enhancers or operators) which are involved in initiating transcription of an operon in a microbe. Transcription factor responsive elements responsive to various transcription factors are known in the art and additional responsive elements can be identified by one of ordinary skill in the art. For example, microarray analysis can be used to identify genes that are regulated by a transcription factor of interest. For interest, genes regulated by a transcription factor would be expressed at higher levels in wild type cells than in cells which are deleted for the transcription factor. In addition, genes responsive to a given transcription factor would comprise one or more target sequences responsive to the transcription factor in their promoter regions (Lyons et al. 2000. PNAS 97:7957). Exemplary responsive elements include: araBAD, araE, araFGH (responsive to AraC); melBAD (responsive to MelR); rhaSR (responsive to RhaR); rahBAD, rhaT (responsive to RhaS); Pm (responsive to XylS); fumC, inaA, micF, nfo, pai5, sodA, tolC, acrAB, fldA, fpr, mar, poxB, ribA, and zwf (responsive to MarA, SoxS, Rob); and coo, rns (responsive to Rns).

The language "marA family polypeptide responsive element" includes a nucleic acid sequence which can interact with marA, *e.g.,* promoters or enhancers which are involved in regulating transcription of a nucleic acid sequence in a microbe. MarA responsive elements comprise approximately 16 base pair marbox sequence, the sequence critical for the binding of MarA to its target. In addition, a secondary site, the accessory marbox, upstream of the primary marbox contributes to basal and derepressed mar transcription. A marbox may be situated in either the forward or backward orientation. (Martin, 1999, Mol. Microbiol. 34:431-441). In the *marRAB* operon, the marbox is in the backward orientation and is thus located on the sense strand with respect to *marRAB* (Martin, 1999, Mol. Microbiol. 34:431-441). Subtle differences within the marbox sequence of particular promoters may account for differential regulation by MarA and other related, *e.g.,* SoxS and Rob, transcription factors (Martin, 2000, Mol Microbiol; 35(3):623-34). In one embodiment, MarA family responsive elements are promoters that are structurally or functionally related to a *marA* promoter, e.g., interact with MarA or a protein related to MarA.

Preferably, the *marA* family polypeptide responsive element is a *marRAB* promoter. For example, in the *mar* operon, several promoters are *marA* family polypeptide responsive promoters as defined herein, *e.g.,* the 405-bp *ThaI* fragment from the *marO* region is a *marA* family responsive promoter (Cohen et al. 1993. J. Bact. 175:7856). In addition, MarA has been shown to bind to a 16 bp MarA binding site (referred to as the "marbox" within *marO (*Martin et al. 1996. J. Bacteriol. 178:2216). MarA also affects transcription from the *acrAB; micF; mlr 1,2,3; slp; nfo; inaA; fpr; sodA; soi-17,19; zwf; fumC; or rpsF* promoters (Alekshun and Levy. 1997. Antimicrobial Agents and Chemother. 41:2067). Other *marA* family responsive promoters are known in the art and include: *araBAD, araE, araFGH and araC,* which are activated by AraC; Pm, which is activated by XylS; *melAB* which is activated by MelR; and *oriC* which is bound by Rob.

The language "MarA family polypeptide responsive promoter" also includes portions of the above promoters which are sufficient to activate transcription upon interaction with a MarA family member protein. The portions of any of the MarA family polypeptide-responsive promoters which are minimally required for their activity can be easily determined by one of ordinary skill in the art, e.g., using mutagenesis. Exemplary techniques are described by Gallegos et al. (1996, J. Bacteriol. 178:6427). A "MarA family polypeptide responsive promoter" also includes non-naturally occurring variants of MarA family polypeptide responsive promoters which have the same function as naturally occurring MarA family promoters. Preferably such variants have at least 30% or greater, 40% or greater, or 50% or greater, nucleotide sequence identity with a naturally occurring MarA family polypeptide responsive promoter. In preferred embodiments, such variants have at least about 70% nucleotide sequence identity with a naturally occurring MarA family polypeptide responsive promoter. In more preferred embodiments, such variants have at least about 80% nucleotide sequence identity with a naturally occurring MarA family polypeptide responsive promoter. In particularly preferred embodiments, such variants have at least about 90% nucleotide sequence identity and preferably at least about 95% nucleotide sequence identity with a naturally occurring MarA family polypeptide responsive promoter. In yet other embodiments nucleic acid molecules encoding variants of MarA family polypeptide responsive promoters are capable of hybridizing under stringent conditions to nucleic acid molecules encoding naturally occurring MarA family polypeptide responsive promoters.

In one embodiment, the methods described herein can employ molecules identified as responding to the transcription factors of the invention, i.e., molecules in a regulon whose expression is controlled by the transcription factor. For example, compounds that modulate transcription of genes that are directly modulated by a microbial transcription factor (e.g., a marA family transcription factor) can be used to modulate virulence of a microbe or modulate infection by a microbe. In another embodiment, such genes can be identified as important in controlling virulence using the methods described herein. As used herein, the term "regulon" includes two or more loci in two or more different operons whose expression is regulated by a common repressor or activator protein.

The term "interact" includes close contact between molecules that results in a measurable effect, e.g., the binding of one molecule with another. For example, a transcription factor can interact with a transcription factor responsive element and alter the level of transcription of DNA. Likewise, compounds can interact with a transcription factor and alter the activity of a transcription factor.

The term "inducible promoter" includes promoters that are activated to induce the synthesis of the genes they control. As used herein, the term "constitutive promoter" includes promoters that do not require the presence of an inducer, *e.g.,* are continuously active.

The term "microbe" includes microorganisms expressing or made to express a transcription factor, e.g., an HTH containing transcription factor, an AraC family polypeptide, or a marA family polypeptide. "Microbes" are of some economic importance, *e.g.,* are environmentally important or are important as human pathogens. For example, in one embodiment microbes cause environmental problems, *e.g.,* fouling or spoilage, or perform useful functions such as breakdown of plant matter. In another embodiment, microbes are organisms that live in or on mammals and are medically important. Preferably microbes are unicellular and include bacteria, fungi, or protozoa. In another embodiment, microbes suitable for use in the invention are multicellular, *e.g.,* parasites or fungi. In preferred embodiments, microbes are pathogenic for humans, animals, or plants. Microbes may be used as intact cells or as sources of materials for cell-free assays and/or as targets in a therapeutic method. In one embodiment, the microbes include prokaryotic organisms. In other embodiments, the microbes include eukaryotic organisms. Tables 1 and 3 provides a partial list of bacterial that comprise MarA homologs.

**Table 3. A partial list of species that have MarA homologues.**

| MarA | |
|---|---|
| *E. coli* | |
| | UPEC (uropathogenic) |
| | EPEC (enteropathogenic) |
| | ETEC (enterotoxigenic) |
| | EHEC (enterohemorrhagic) |
| | EAEC (enteroaggregative) |
| | EIEC (enteroinvasive) |
| | ETEC (enterotoxigenic) |
| | DHEC (diarrhea-associated hemolytic) |
| | CTD (cytolethal distending toxin-producing) |
| *Salmonella enterica* | |
| | Cholerasuis (septicemia) |
| | Enteritidis enteritis |
| | Typhimurium enteritis |
| | resistant) Typhimurium (multi-drug |
| | Typhimurium |
| | Typhimurium DT 104 |
| | Typhi |
| *Yersinia enterocolitica* | |
| *Yersinia pestis* | |
| *Yersinia pseudotuberculosis* | |
| *Pseudomonas aeruginosa* | |
| *Enterobacter* spp. | |
| *Klebsiella* sp. | |
| *Proteus* spp. | |
| *Bacillus anthracis* | |
| *Burkholderia pseudomallei* | |
| *Brucella suis* | |
| *Vibrio cholerae* | |
| *Citrobacter sp.* | |
| *Shigella* sp. | |
| *S. flexneri* | |
| *S. sonnei* | |
| *S. dysenteriae* | |
| *Providencia stuartii* | |
| *Neisseria meningitidis* | |
| *Mycobacterium tuberculosis* | |
| *Mycobacterium leprae* | |
| *Staphylococcus aureus* | |
| *Streptococcus pyogenes* | |
| *Enterococcus faecalis* | |
| *Bordetella pertussis* | |
| *Bordetella bronchiseptica* | |

In one embodiment, the assays described herein can employ indicators, such as selective markers and reporter genes. The term selective marker includes polypeptides that serve as indicators, e.g., provide a selectable or screenable trait when expressed by a cell. The term "selective marker" includes both selectable markers and counterselectable markers. As used herein the term "selectable marker" includes markers that result in a growth advantage when a compound or molecule that fulfills the test parameter of the assay is present. The term "counterselectable marker" includes markers that result in a growth disadvantage unless a compound or molecule is present which disrupts a condition giving rise to expression of the counterselectable marker. Exemplary selective markers include cytotoxic gene products, gene products that confer antibiotic resistance, gene products that are essential for growth, gene products that confer a selective growth disadvantage when expressed in the presence of a particular metabolic substrate (e.g., the expression of the URA3 gene confers a growth disadvantage in the presence of 5-fluoroorotic acid).

As used herein the term "reporter gene" includes any gene which encodes an easily detectable product which is operably linked to a regulatory sequence, e.g., to a transcription factor responsive promoter. By operably linked it is meant that under appropriate conditions an RNA polymerase may bind to the promoter of the regulatory region and proceed to transcribe the nucleotide sequence such that the reporter gene is transcribed. In preferred embodiments, a reporter gene consists of the transcription factor responsive promoter linked in frame to the reporter gene. In certain embodiments, however, it may be desirable to include other sequences, e.g, transcriptional regulatory sequences, in the reporter gene construct. For example, modulation of the activity of the promoter may be effected by altering the RNA polymerase binding to the promoter region, or, alternatively, by interfering with initiation of transcription or elongation of the mRNA. Thus, sequences which are herein collectively referred to as transcriptional regulatory elements or sequences may also be included in the reporter gene construct. In addition, the construct may include sequences of nucleotides that alter translation of the resulting mRNA, thereby altering the amount of reporter gene product.

Examples of reporter genes include, but are not limited to CAT (chloramphenicol acetyl transferase) (Alton and Vapnek (1979), Nature 282: 864-869) luciferase, and other enzyme detection systems, such as beta-galactosidase; firefly luciferase (deWet et al. (1987), Mol. Cell. Biol. 7:725-737); bacterial luciferase (Engebrecht and Silverman (1984), PNAS 1: 4154-4158; Baldwin et al. (1984), Biochemistry 23: 3663-3667); PhoA, alkaline phosphatase (Toh et al. (1989) Eur. J. Biochem. 182: 231-238, Hall et al. (1983) J. Mol. Appl. Gen. 2: 101), human placental secreted alkaline phosphatase (Cullen and Malim (1992) Methods in Enzymol. 216:362-368) and green fluorescent protein (U.S. patent 5,491,084; WO96/23898).

In certain embodiments of the invention it will be desirable to obtain "isolated or recombinant" nucleic acid molecules transcription factors or mutant forms thereof. The term "isolated or recombinant" includes nucleic acid molecules which have been, e.g., (1) amplified in vitro by, for example, polymerase chain reaction (PCR); (2) recombinantly produced by cloning, or (3) purified, as by cleavage and gel separation; or (4) synthesized by, for example, chemical synthesis. Such a nucleic acid molecule is isolated from the sequences which naturally flank it in the genome and from cellular components.

In yet other embodiments of the invention, it will be desirable to obtain a substantially purified or recombinant transcription factors. Such polypeptides, for example, can be purified from cells which have been engineered to express an isolated or recombinant nucleic acid molecule which encodes a transcription factor. For example, as described in more detail below, a bacterial cell can be transformed with a plasmid which encodes a transcription factor. The transcription factor can then be purified from the bacterial cells and used, for example, in the cell-free assays described herein or known in the art.

As used herein, the term "antibiotic" includes antimicrobial agents isolated from natural sources or chemically synthesized. The term "antibiotic" refers to antimicrobial agents for use in human therapy. Preferred antibiotics include: tetracyclines, fluoroquinolones, chloramphenicol, penicillins, cephalosporins, puromycin, nalidixic acid, and rifampin.

The term "test compound" includes any reagent or test agent which is employed in the assays of the invention and assayed for its ability to influence the activity of a transcription factor, e.g., an AraC family polypeptide, an HTH protein, and/or a MarA family polypeptide, e.g., by binding to the polypeptide or to a molecule with which it interacts. More than one compound, e.g., a plurality of compounds, can be tested at the same time for their ability to modulate the activity of a transcription factor, e.g., an AraC family polypeptide, an HTH protein, or a MarA family polypeptide, activity in a screening assay. The term "screening assay" preferably refers to assays which test the ability of a plurality of compounds to influence the readout of choice rather than to tests which test the ability of one compound to influence a readout. In one embodiment, high throughput screening can be used to assay for the activity of a compound. In one embodiment, the test compound is a MarA family modulating compound.

Exemplary test compounds which can be screened for activity include, but are not limited to, peptides, non-peptidic compounds, nucleic acids, carbohydrates, small organic molecules (e.g., polyketides), and natural product extract libraries. The term "non-peptidic test compound" includes compounds that are comprised, at least in part, of molecular structures different from naturally-occurring L-amino acid residues linked by natural peptide bonds. However, "non-peptidic test compounds" also include compounds composed, in whole or in part, of peptidomimetic structures, such as D-amino acids, non-naturally-occurring L-amino acids, modified peptide backbones and the like, as well as compounds that are composed, in whole or in part, of molecular structures unrelated to naturally-occurring L-amino acid residues linked by natural peptide bonds. "Non-peptidic test compounds" also are intended to include natural products.

In one embodiment, small molecules can be used as test compounds. The term "small molecule" is a term of the art and includes molecules that are less than about 7500, less than about 5000, less than about 1000 molecular weight or less than about 500 molecular weight. In one embodiment, small molecules do not exclusively comprise peptide bonds. In another embodiment, small molecules are not oligomeric. Exemplary small molecule compounds which can be screened for activity include, but are not limited to, peptides, peptidomimetics, nucleic acids, carbohydrates, small organic molecules (e.g., polyketides) (Cane et al. 1998. Science 282:63), and natural product extract libraries. In another embodiment, the compounds are small, organic non-peptidic compounds. In a further embodiment, a small molecule is not biosynthetic. For example, a small molecule is preferably not itself the product of transcription or translation.

The term "antagonist" includes transcription factor modulating compounds (e.g., AraC family polypeptide modulating compounds, HTH protein modulating compounds, MarA family polypeptide modulating compounds, etc.) which inhibit the activity of a transcription factor by binding to and inactivating the transcription factor (e.g., an AraC family modulating compound, an MarA family polypeptide modulating compound, etc.), e.g., by binding to a nucleic acid target with which the transcription factor interacts (e.g., for MarA, a marbox), by disrupting a signal transduction pathway responsible for activation of a particular regulon (e.g., for Mar, the inactivation of MarR or activation of MarA synthesis), and/or by disrupting a critical protein-protein interaction (e.g., MarA-RNA polymerase interactions that are required for MarA to function as a transcription factor.) Antagonists may include, for example, naturally (e.g., TrpR-tryptophan and LacI-lactose) or chemically synthesized compounds such as small cell permeable organic molecules, nucleic acid interchelators, peptides, *etc.*

The term "agonist" includes transcription factor modulating compounds (e.g., AraC family polypeptide modulating compounds, HTH protein modulating compounds, MarA family polypeptide modulating compounds, etc.) which promote the activity of a transcription factor by binding to and activating the transcription factor (e.g., an AraC family modulating compound, an MarA family polypeptide modulating compound, etc.), by binding to a nucleic acid target with which the transcription factor interacts (e.g., for MarA, a marbox), by facilitating a signal transduction pathway responsible for activation of a particular regulon (e.g., for Mar, the inactivation of MarR or activation of MarA synthesis), and/or by facilitating a critical protein-protein interaction (e.g., MarA-RNA polymerase interactions that are required for MarA to function as a transcription factor.) Agonists may include, for example, naturally or chemically synthesized compounds such as small cell permeable organic molecules, nucleic acid interchelators, peptides, *etc.*

It will be understood by one of ordinary skill in the art that transcription factors can activate or repress transcription. Accordingly, a modulator (e.g., an agonist or antagonist) may increase or decrease transcription depending upon the activity of the unmodulated transcription factor.

### II Polypeptides Comprising Microbial Transcription Factors or Transcription Factor Domains

Polypeptides comprising transcription factors or transcription factor domains can be naturally occurring proteins or, e.g., can be fusion proteins comprising a portion of at least one transcription factor (e.g., a domain that retains an activity of the full-length polypeptide, e.g., which is capable of binding to a transcription factor responsive element or which retains their indicator function, e.g., a helix-turn-helix domain) and a non-transcription factor protein.

Nucleic acid molecules encoding polypeptides transcription factors or functional domains thereof can be expressed in cells using vectors. Almost any conventional delivery vector can be used. Such vectors are widely available commercially and it is within the knowledge and discretion of one of ordinary skill in the art to choose a vector which is appropriate for use with a given microbial cell. The sequences encoding these polypeptides can be introduced into a cell on a self-replicating vector or may be introduced into the chromosome of a microbe using homologous recombination or by an insertion element such as a transposon.

Almost any conventional delivery vector can be used. Such vectors are widely available commercially and it is within the knowledge and discretion of one of ordinary skill in the art to choose a vector which is appropriate for use with a given microbial cell. The sequences encoding these domains can be introduced into a cell on a self-replicating vector or may be introduced into the chromosome of a microbe using homologous recombination or by an insertion element such as a transposon.

These nucleic acids can be introduced into microbial cells using standard techniques, for example, by transformation using calcium chloride or electroporation. Such techniques for the introduction of DNA into microbes are well known in the art.

In one embodiment, a nucleic acid molecule which has been amplified *in vitro* by, for example, polymerase chain reaction (PCR); recombinantly produced by cloning, or) purified, as by cleavage and gel separation; or synthesized by, for example, chemical synthesis can be used to produce MarA family polypeptides (George, A. M. & Levy, S. B. (1983)J. Bacteriol. 155, 541-548; Cohen, S. P. et al. (1993) J Infect. Dis. 168, 484-488; Cohen, S. P et al. (1993) J Bacteriol. 175, 1484-1492; Sulavick, M. C. et al. (1997) J. Bacteriol. 179, 1857-1866).

Host cells can be genetically engineered to incorporate nucleic acid molecules of the invention. In one embodiment nucleic acid molecules specifying transcription factors can be placed in a vector. The term "vector" refers to a nucleic acid molecule capable of transporting another nucleic acid molecule to which it has been linked. The term "expression vector" or "expression system" includes any vector, (*e.g*., a plasmid, cosmid or phage chromosome) containing a gene construct in a form suitable for expression by a cell (*e.g*., linked to a promoter). In the present specification, "plasmid" and "vector" are used interchangeably, as a plasmid is a commonly used form of vector. Moreover, the invention is intended to include other vectors which serve equivalent functions. A great variety of expression systems can be used to produce the polypeptides of the invention. Such vectors include, among others, chromosomal, episomal and virus-derived vectors, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids.

Appropriate vectors are widely available commercially and it is within the knowledge and discretion of one of ordinary skill in the art to choose a vector which is appropriate for use with a given host cell. The sequences encoding a transcription factor, such as, for example, MarA family polypeptides, can be introduced into a cell on a self-replicating vector or may be introduced into the chromosome of a microbe using homologous recombination or by an insertion element such as a transposon.

The genes specifying these proteins can be amplified using PCR and bacterial genomic DNA. These PCR products can then be cloned into pET15b (Novagen, Madison, WI), to incorporate a 6-His tag in each protein, and proteins will be expressed and purified according to standard methods.

The expression system constructs may contain control regions that regulate expression. "Transcriptional regulatory sequence" is a generic term to refer to DNA sequences, such as initiation signals, enhancers, operators, and promoters, which induce or control transcription of polypeptide coding sequences with which they are operably linked. It will also be understood that a recombinant gene encoding a transcription factor gene, e.g., an HTH protein gene or an AraC family polypeptide, e.g., MarA family polypeptide, can be under the control of transcriptional regulatory sequences which are the same or which are different from those sequences which control transcription of the naturally-occurring transcription factor gene. Exemplary regulatory sequences are described in Goeddel; Gene Expression Technology: Methods in Enzymology 185, Academic Press, San Diego, CA (1990). For instance, any of a wide variety of expression control sequences, that control the expression of a DNA sequence when operatively linked to it, may be used in these vectors to express DNA sequences encoding the polypeptide.

Generally, any system or vector suitable to maintain, propagate or express nucleic acid molecules and/or to express a polypeptide in a host may be used for expression in this regard. The appropriate DNA sequence may be inserted into the expression system by any of a variety of well-known and routine techniques, such as, for example, those set forth in Sambrook et al., Molecular Cloning, A Laboratory Manual, (supra).

Exemplary expression vectors for expression of a gene encoding a polypeptide and capable of replication in a bacterium, e.g., a gram positive, gram negative, or in a cell of a simple eukaryotic fungus such as a *Saccharomyces* or, *Pichia,* or in a cell of a eukaryotic organism such as an insect, a bird, a mammal, or a plant, are known in the art. Such vectors may carry functional replication-specifying sequences (replicons) both for a host for expression, for example a *Streptomyces,* and for a host, for example, *E. coli,* for genetic manipulations and vector construction. See, e.g., U.S. 4,745,056. Suitable vectors for a variety of organisms are described in Ausubel, F. et al., Short Protocols in Molecular Biology, Wiley, New York (1995), and for example, for *Pichia,* can be obtained from Invitrogen (Carlsbad, CA).

Useful expression control sequences, include, for example, the early and late promoters of SV40, adenovirus or cytomegalovirus immediate early promoter, the *lac* system, the *trp* system, the TAC or TRC system, T7 promoter whose expression is directed by T7 RNA polymerase, the major operator and promoter regions of phage lambda, the control regions for fd coat polypeptide, the promoter for 3-phosphoglycerate kinase or other glycolytic enzymes, the promoters of acid phosphatase, *e.g.,* Pho5, the promoters of the yeast α-mating factors, the polyhedron promoter of the baculovirus system and other sequences known to control the expression of genes of prokaryotic or eukaryotic cells or their viruses, and various combinations thereof. A useful translational enhancer sequence is described in U.S. 4,820,639.

In one embodiment, an inducible promoter will be employed to express a polypeptide of the invention. For example, in one embodiment, trp (induced by tryptophan), tac (induced by lactose), or tet (induced by tetracycline) can be used in bacterial cells, or GAL1 (induced by galactose) can be used in a host cellcell.

In another embodiment, a constitutive promoter can be used to express a polypeptide of the invention.

It should be understood that the design of the expression vector may depend on such factors as the choice of the host cell to be transformed and/or the type of polypeptide desired to be expressed. Representative examples of appropriate hosts include bacterial cells, such as gram positive, gram negative cells; fungal cells, such as yeast cells and Aspergillus cells; insect cells such as Drosophila S2 and Spodoplera Sf9 cells; animal cells such as CHO, COS, HeLa, C127, 3T3, BHK, 293 and Bowes melanoma cells; and plant cells.

In one embodiment, cells used to express heterologous polypeptides of the invention, comprise a mutation which renders one or more endogenous transcription factors, such as a AraC family polypeptide or a MarA family polypeptide, nonfunctional or causes one or more endogenous polypeptide to not be expressed. Manipulation of the genetic background in this manner allows for screening for compounds that modulate specific transcription factors, such as MarA family members or AraC family members, or more than one transcription factors.

In other embodiments, mutations may also be made in other related genes of the host cell, such that there will be no interference from the endogenous host loci. In yet another embodiment, a mutation may be made in a chromosomal gene to create a heterotroph.

Introduction of a nucleic acid molecule into the host cell ("transformation") can be effected by methods described in many standard laboratory manuals, such as Davis et al., Basic Methods In Molecular Biology, (1986) and Sambrook et al., Molecular Cloning: A Laboratory Manual, 2nd Ed., Cold Spring Harbor Laboratory Press, Cold Spring Harbor, N.Y. (1989). Examples include calcium phosphate transfection, DEAE-dextran mediated transfection, transvection, microinjection, cationic lipid-mediated transfection, electroporation, transduction, scrape loading, ballistic introduction and infection.

Purification of polypeptides, *e.g.,* recombinantly expressed polypeptides, can be accomplished using techniques known in the art. For example, if the polypeptide is expressed in a form that is secreted from cells, the medium can be collected. Alternatively, if the polypeptide is expressed in a form that is retained by cells, the host cells can be lysed to release the polypeptide. Such spent medium or cell lysate can be used to concentrate and purify the polypeptide. For example, the medium or lysate can be passed over a column, *e.g.,* a column to which antibodies specific for the polypeptide have been bound. Alternatively, such antibodies can be specific for a second polypeptide which has been fused to the first polypeptide (*e.g.*, as a tag) to facilitate purification of the first polypeptide. Other means of purifying polypeptides are known in the art.

### III. Methods for Identifying Antiinfective Compounds Which Modulate an Activity of a Transcription Factor

Transcription factor agonists and antagonists can be assayed in a variety of ways. For example, in one embodiment, the invention provides for methods for identifying a compound which modulates an transcription factor, e.g., by measuring the ability of the compound to interact with an transcription factor nucleic acid molecule or an transcription factor polypeptide or the ability of a compound to modulate the activity and/or expression of an transcription factor polypeptide.

Furthermore, the ability of a compound to modulate the binding of an transcription factor polypeptide or transcription factor nucleic acid molecule to a molecule to which they normally bind, e.g., a nucleic acid, cofactor, or protein molecule can be tested.

In one embodiment, a transcription factor and its cognate DNA sequence can be present in a cell free system, e.g., a cell lysate and the effect of the compound on that interaction can be measured using techniques known in the art.

In a preferred embodiment, the assay system is a cell-based system. Compounds identified using the subject methods are useful, *e.g.,* in reducing microbial virulence and, thereby, and in reducing the ability of the microbe to cause infection in a host.

The ability of the test compound to modulate the expression and/or activity of a transcription factor can be determined in a variety of ways. Exemplary methods which can be used in the instant assays are known in the art and are described, e.g., in 5,817,793 and WO 99/61579. Other exemplary methods are described in more detail below.

In one embodiment, the invention provides for methods of identifying a test compound which modulates the expression and/or activity of a transcription factor, (e.g., an HTH protein, a MarA family polypeptide, an AraC family polypeptide, etc.) by contacting a cell expressing a transcription factor (or portion thereof) with a test compound under conditions which allow interaction of the test compound with the cell.

Cell-based assays can be performed in a relatively high-throughput manner using automatic liquid dispensers and robotic instrumentation. Optionally, controls can be included to identify compounds that are inhibitory to cell growth. Also, MIC assays, achieved using robotic instrumentation and a standard panel of different gram-positive and gram-negative organisms, can be performed on any compounds identified using standard methods. Preferably, a transcription factor modulatory compound has no intrinsic antibacterial activity.

For *in vitro* assays, control molecules, e.g.,non-MarA or AraC proteins can optionally be included to detect non-specific interactions, e.g., DNA interchelators, of compounds.

Preferably, the compounds identified using the instant assays are effective at modulating at least one transcription factor. In one embodiment, the compounds are effective at modulating more than one transcription factor. In one embodiment, the compound is effective at modulating more than one related transcription factor. In another embodiment, the compound is effective at modulating more than one unrelated transcription factor. In another embodiment, a compound specifically modulates one transcription factor.

The assays of the invention can be combined. For example, compounds can be identified in a preliminary cell-free screening assay. Promising compounds can be further tested in cell based and/or animal assays.

### 1. Whole Cell Assays

In one embodiment of the invention, the subj ect screening assays can be performed using whole cells. In one embodiment of the invention, the step of determining whether a compound reduces the activity or expression of a transcription factor comprises contacting a cell expressing a transcription factor with a compound and measuring the ability of the compound to modulate the activity and/or expression of a transcription factor.

In another embodiment, modulators of transcription factor expression are identified in a method wherein a cell is contacted with a candidate compound and the expression of transcription factor mRNA or protein in the cell is determined. The level of expression of transcription factor mRNA or protein in the presence of the candidate compound is compared to the level of expression of transcription factor mRNA or polypeptide in the absence of the candidate compound. The candidate compound can then be identified as a modulator of transcription factor expression based on this comparison. For example, when expression of transcription factor mRNA or protein is greater (e.g., statistically significantly greater) in the presence of the candidate compound than in its absence, the candidate compound is identified as a stimulator of transcription factor mRNA or protein expression. Alternatively, when expression of transcription factor mRNA or protein is less (e.g., statistically significantly less) in the presence of the candidate compound than in its absence, the candidate compound is identified as an inhibitor of transcription factor mRNA or protein expression. The level of transcription factor mRNA or protein expression in the cells can be determined by methods described herein for detecting transcription factor mRNA or protein.

To measure expression of a transcription factor, transcription of a transcription factor gene can be measured in control cells which have not been treated with the compound and compared with that of test cells which have been treated with the compound. For example, cells which express endogenous transcription factors or which are engineered to express or overexpress recombinant transcription factors can be caused to express or overexpress a recombinant transcription factor in the presence and absence of a test modulating agent of interest, with the assay scoring for modulation in transcription factor responses by the target cell mediated by the test agent. For example, as with the cell-free assays, modulating agents which produce a change, e.g., a statistically significant change in transcription factor -dependent responses (either an increase or decrease) can be identified.

Recombinant expression vectors that can be used for expression of transcription factor are known in the art (see discussions above). In one embodiment, within the expression vector the transcription factor -coding sequences are operatively linked to regulatory sequences that allow for constitutive or inducible expression of transcription factor in the indicator cell(s). Use of a recombinant expression vector that allows for constitutive or inducible expression of transcription factor in a cell is preferred for identification of compounds that enhance or inhibit the activity of transcription factor. In an alternative embodiment, within the expression vector the transcription factor coding sequences are operatively linked to regulatory sequences of the endogenous transcription factor gene (i.e., the promoter regulatory region derived from the endogenous gene). Use of a recombinant expression vector in which transcription factor expression is controlled by the endogenous regulatory sequences is preferred for identification of compounds that enhance or inhibit the transcriptional expression of transcription factor.

In one embodiment, the level of transcription can be determined by measuring the amount of RNA produced by the cell. For example, the RNA can be isolated from cells which express a transcription factor and that have been incubated in the presence or absence of compound. Northern blots using probes specific for the sequences to be detected can then be performed using techniques known in the art. Numerous other, art-recognized techniques can be used. For example, western blot analysis can be used to test for transcription factor. For example, in another embodiment, transcription of specific RNA molecules can be detected using the polymerase chain reaction, for example by making cDNA copies of the RNA transcript to be measured and amplifying and measuring them. In another embodiment, RNAse protection assays, such as S1 nuclease mapping or RNase mapping can be used to detect the level of transcription of a gene. In another embodiment, primer extension can be used.

In yet other embodiments, the ability of a compound to induce a change in transcription or translation of a transcription factor can be accomplished by measuring the amount of transcription factor produced by the cell. Polypeptides which can be detected include any polypeptides which are produced upon the activation of a transcription factor responsive promoter, including, for example, both endogenous sequences and reporter gene sequences. In one embodiment, the amount of polypeptide made by a cell can be detected using an antibody against that polypeptide. In other embodiments, the activity of such a polypeptide can be measured.

In one embodiment, other sequences which are regulated by a transcription factor can be detected. In one embodiment, sequences not normally regulated by a transcription factor can be assayed by linking them to a promoter that is regulated by the transcription factor.

In preferred embodiments, to provide a convenient readout of the transcription from a promoter, such a promoter is linked to a reporter gene, the transcription of which is readily detectable. For example, a bacterial cell, e.g., an *E. coli* cell, can be transformed as taught in Cohen et al. 1993. J. Bacteriol. 175:7856.

Examples of reporter genes include, but are not limited to, CAT (chloramphenicol acetyl transferase) (Alton and Vapnek (1979), Nature 282: 864-869) luciferase, and other enzyme detection systems, such as beta-galactosidase; firefly luciferase (deWet et al. (1987), Mol. Cell. Biol. 7:725-737); bacterial luciferase (Engebrecht and Silverman (1984), PNAS 1: 4154-4158; Baldwin et al. (1984), Biochemistry 23: 3663-3667); PhoA, alkaline phosphatase (Toh et al. (1989) Eur. J. Biochem. 182: 231-238, Hall et al. (1983) J. Mol. Appl. Gen. 2: 101), human placental secreted alkaline phosphatase (Cullen and Malim (1992) Methods in Enzymol. 216:362-368) and green fluorescent polypeptide (U.S. patent 5,491,084; WO96/23898).

In one embodiment, the expression of a selectable marker that confers a selective growth disadvantage or lethality is placed under the direct control of a transcription factor responsive element in a cell expressing the transcription factor.

In one embodiment, the transcription factor is plasmid encoded. In one embodiment, the genetic background of the host organism is manipulated, e.g., to delete one or more chromosomal transcription factor genes or transcription factor homolog genes.

In one embodiment, expression of a transcription factor is controlled by a highly regulated and inducible promoter. For example, in one embodiment, a promoter such as *inaA, galT, micF trp, tac,* or *tet* in bacterial cells or *GAL1* in yeast cells can be used.

For example, to monitor the activity of the MarA (AraC) family members in whole cells, gene promoter luciferase *(luc)* fusion assays can be performed with the following constructs: *bfpT* [to measure BfpT (PerA) and GadX activity], *invF* [to monitor HilC and HilD function], *sicA* [to measure InvF activity], *mxiC* [to monitor VirF and MxiE function], and *ctxA, tcpA,* and *acfA* [to measure ToxT activity]. *V. cholerae* strain AC-V1225 bears a transcriptional *ctxA-lacZ* fusion and can be used to monitor CtxA expression in whole cells. This strain can be grown under inducing conditions with Mar inhibitors and measure β-galactosidase activity in the treated bacteria. In order to do this assay as a high-throughput screen in a 96-well plate format, the technique of Griffith et al. will be used (Griffith, K.L, et al. (2002) Biochem. Biophys. Res. Commun.290:397-402). In the assays, whole cells will be grown in serial 10-fold dilutions of a Mar inhibitor. Compounds that negatively affect MarA (AraC) family member activity will be detected by decreased expression of the *luc* reporter gene.

In another embodiment, expression of the transcription factor is constitutive.

In one embodiment, a selective marker encoding a cytotoxic gene product (e.g., ccdB) is employed.

In another embodiment, a selective marker is a gene that confers antibiotic resistance (e.g., kan, cat, or bla).

In another embodiment, a selective marker is an essential gene (e.g., purA or guaB in a purine or guanine heterotroph).

In still another embodiment, a selective marker is a gene that confers a selective growth disadvantage in the presence of a particular metabolic substrate (e.g., the expression of URA3 in the presence of 5-fluoroorotic acid [5-FOA] in yeast).

In yet another embodiment, the ability of a compound to modulate the binding of a transcription factor to a transcription factor binding molecule (e.g., DNA or protein) can be determined. Transcription factor binding polypeptides can be identified using techniques which are known in the art. For example, in the case of binding polypeptides that interact with transcription factors, interaction trap assays or two hybrid screening assays can be used.

In one embodiment, compounds that modulate transcription factors (e.g., HTH proteins, AraC family polypeptides, or MarA family polypeptides) are identified using a one-hybrid screening assay. As used herein, the term "one-hybrid screen" as used herein includes assays that detect the disruption of protein-nucleic acid interactions. These assays will identify agents that interfere with the binding of a transcription factor (e.g., an HTH protein, a AraC family polypeptide, or a MarA family polypeptide) to a particular target, e.g., DNA containing, for MarA, a marbox, at the level of the target itself, e.g., by binding to the target and preventing the transcriptional activation factor from interacting with or binding to this site.

In another embodiment, compounds of the invention are identified using a two-hybrid screening assay. As used herein the term "two-hybrid screen" as used herein includes assays that detect the disruption of protein-protein interactions. Such two hybrid assays can be used to interfere with crucial protein-transcription factor interactions (e.g., HTH protein interactions, AraC family polypeptide interactions, MarA family polypeptide interactions). One example would be to prevent RNA polymerase-MarA family polypeptide interactions that are necessary for the MarA family polypeptide to function as a transcription factor (either positive acting or negative acting).

In yet another embodiment, compounds of the invention are identified using a three-hybrid screening assay. As used herein the term "three-hybrid screen" as used herein includes assays that will detect the disruption of a signal transduction pathway(s) required for the activation of a particular regulon of interest. In one embodiment, the three-hybrid screen is used to detect disruption of a signal transduction pathway(s) required for the activation of the Mar regulon, e.g, synthesis of MarA (Li and Park. J. Bact. 181:4824). The assay can be used to identify compounds that may be responsible for activating transcription factor expression, e.g., Mar induction by antibiotics may proceed in this manner.

In one embodiment of the assay, the expression of a selective marker (e.g., ccdB, cat, bla, kan, guaB, URA3) is put under the direct control of a promoter responsive to the transcription factor (e.g., inaA, galT, micF). In the absence of the transcription factor the expression of the selective marker would be silent. For example, in the case of regulation of the cytotoxic gene ccdB, the gene would be silent and the cells would survive. Synthesis of a transcription factor from an inducible plasmid in a suitable host would result in the activation of the activatable promoter responsive to the transcription factor and expression of the selective marker. In the case of ccdB, the gene would be expressed and result in cell death. Compounds that inhibit a transcriptional activator would be identified as those that permit cell survival under conditions of expression of the activator.

In another embodiment, e.g., where the expression of an activatable promoter responsive to the transcription factor regulates a gene such as URA3, a different result could be obtained. In this case, in the absence of the transcription factor and thus, in the absence of URA3 expression, cells would grow in the presence of a 5-FOA. Upon activation of expression of the transcription factor and, thus, synthesis of URA3, cells would die following the conversion of 5-FOA to a toxic metabolite by URA3.

In another embodiment, a selectable marker is put under the direct control of a repressible promoter responsive to the transcription factor (e.g., fecA). In this example, under conditions of constitutive transcription factor synthesis, e.g., in a constitutive mutant, the expression of the selectable marker would be silent. In the case of ccdB, this would mean that cells would remain viable. Following inactivation of the transcription factor, the selectable marker would be turned on, resulting in cell death.

In another embodiment, a purine or guanine heterotroph can be constructed by the inactivation of the chromosomal guaB or purA genes in E. *coli.* The guaB or purA gene would then be cloned into a suitable vector, under the control of its natural promoter. This construct would then be transformed into the heterotrophic host. The heterotroph will not grow if transcription factor expression is constitutive and if cells are grown on media lacking purines or guanine. This can be attributed to transcription factor mediated repression of guaB or purA synthesis. Candidate inhibiting compounds of a transcription factor can be identified as compounds that restored growth, i.e., relieved repression mediated by the transcription factor of guaB and purA expression.

In one embodiment, in order to identify compounds that modulate activity of a transcription factor from a pathogen, a transcription factor from a non-pathogen or organism that is less pathogenic can be used. For example, *E. coli* has been used previously as a surrogate to assess *Yersinia* spp. gene promoter function and sequence comparisons demonstrate that the *psn* promoter regions were found to be identical in UPEC (strain *E. coli* CFT703), *Y. pestis,* and *Y. pseudotuberculosis.* Accordingly, the *E. coli* CFT703 *psn* promoter can be cloned using PCR into a luciferase *(luc)* reporter plasmid and used in whole cell screening assays.

In preferred embodiments, controls may be included to ensure that any compounds which are identified using the subject assays do not merely appear to modulate the activity of a transcription factor, because they inhibit protein synthesis. For example, if a compound appears to inhibit the synthesis of a protein being translated from RNA which is transcribed upon activation of a transcription factor responsive element, it may be desirable to show that the synthesis of a control, e.g., a protein which is being translated from RNA which is not transcribed upon activation of a transcription factor responsive element, is not affected by the addition of the same compound. For example, the amount of the transcription factor being made and compared to the amount of an endogenous protein being made. In another embodiment the microbe could be transformed with another plasmid comprising a promoter which is not responsive to the transcription factor and a protein operably linked to that promoter. The expression of the control protein could be used to normalize the amount of protein produced in the presence and absence of compound.

In another embodiment, the effect of the compound on the enzymatic acitivity of molecules whose activity is modulated by the transcription factor can be measured. For example, the effects of YbtA inhibition on YopH activity in whole cells can be measured. YopH is a tyrosine phosphatase and Yersinia spp. Virulence factor that is secreted by a TTSS in the pathogen. An assay can be used to measure the effects of inhibiting the activity of LcrF (VirF), a MarA (AraC) family member, on YopH activity in whole cells. The activity of YopH on *p*-nitrophenyl phosphate (pNPP, an indicator of phosphatase activity) results in the formation of a colored substrate that can be measured spectrophotometrically. *Y. pseudotuberculosis* can be incubated in the presence and absence of a Mar inhibitor and controls included to measure the inhibitory effects of the compounds themselves on the phosphatase activity of YopH. The *in vitro* expression of Yops from *Yersinia* spp. can be induced at 37°C and in the absence of calcium. Overnight cultures of *Y. pseudotuberculosis* can be diluted into fresh LB medium containing either sodium oxalate (a divalent metal ion chelator, low calcium containing media) or excess calcium (to repress YopH expression) and grown at 27 °C. Subsequently, aliquots of these cells can be placed into wells containing either a Mar inhibitor or compound solvent (DMSO) as a control. The culture temperature can be shifted to 37 °C (to induce YopH expression in the low calcium containing media) and cells grown for a period of time. The inhibitory effects of compounds on cell growth can be measured separately in identical plates. Prefereably, compounds which do not possess intrinsic antibacterial activity are selected.

The assay plates can be centrifuged and aliquots of the supernatants were added to an assay buffer containing *p*-nitrophenyl phosphate, an indicator of phosphatase activity. After mixing, the OD at 410 nM can be determined. A control can be included to measure the inhibitory effects of the compounds themselves on the phosphatase activity of YopH . Compounds having such an effect can be excluded from further analysis. This assay has been used to identify a number of compounds that inhibit the activity (expression or secretion) of YopH presumably at the level of LcrF (VirF).

In another embodiment, the affect of the compound on the ability of a microbe to form a biofilm can be measured using standard techniques (e.g., O'Toole et al. 1999 Methods Enzymol 310:91)

In another embodiment, the ability of a microbe to penetrate into and/or to adhere to tissue culture cells in the presence and absence of the test compound can be measured. To monitor the penetration (*Salmonella* and *Shigella)* into and adherence (*E. coli, Salmonella,* and *Shigella)* of pathogenic bacteria to tissue culture cells in the presence or absence of the Mar inhibitors, assays can be performed in 96-well microtiter plates e.g., as previously described for *Salmonella* spp. (Darwin et al. (1999) J. Bacteriol. 181:4949-54), *Shigella* spp. (Andrews, et al. (1992) Infect.. Immun. 60:3287-95), and *E*. *coli* (Gomez-Duarte et al. (1995) Infect. Immun. 63:1767-76)). Entry and replication in epithelial cells such as HeLa, Henle-407, or MDCK can be measured by a gentamicin (GM) protection assay. Assays monitoring invasion for different pathogens are essentially the same but are performed with minor modifications. For example, S. *typhimurium* are engulfed by mouse macrophage and a number of epithelial cell lines. Intracellular bacteria are able to replicate (in epithelial cells) and cause cytotoxicity (in macrophages). Both phenomena require secretion of bacterial proteins through a TTSS and protein secretion is controlled by least three MarA (AraC) proteins (HilC, HilD, and InvF), which function in a regulatory cascade. Inhibition of these activators reduces uptake and cytotoxicity. Cells, e.g., HEp-2 cells (ATCC CCL23) can be grown and maintained accordingly. 2x10⁵ HEp-2 cells can be seeded into microtiter plates in order to obtain 90% confluent monolayers within 24 hours. Single colonies of wild type S. *typhimurium* can be grown overnight in standing LB broth containing 0.3 M NaCl, diluted, added to the wells containing the tissue culture cells at a multiplicity of infection (MOI) of ∼10-20, and the cells can be incubated for 1 hr at 37°C to allow for bacterial penetration. Subsequently, the monolayers will be washed with phosphate buffered saline (PBS), incubated with 100 µg/ml GM (to kill extracellular but not intracellular bacteria), washed again with PBS, and then lysed using PBS+0.5% Triton X-100. Serial dilutions of the lysates will be made to obtain viable bacterial counts on LB or McConkey agar plates or by using the most probable number method. Percent invasion will be calculated as follows: 100 X (number of GM^{R} bacteria/total number of input bacteria). The adherence assays are performed in a manner similar to the invasion assays except that multiple washes are included at the first stage of bacteria-tissue culture cell interaction and GM is excluded.

In another embodiment, the ability of certain microbial cells to bind to congo red can be used as a measure of their virulence. Shigella spp. *virF* null mutants are non-invasive in tissue culture cells *in vitro* and are defective for their ability to bind the dye Congo red (CR). The CR binding phenotype is routinely used as a diagnostic for clinical *Shigella* isolates, i.e., bacteria unable to bind CR (Cbr⁻ cells) are non-invasive in the Sereny test *in vivo.* This test is a reliable predictor of virulence of this organism. A simple screen can be used to identify transcription factors (e.g.,VirF) inhibitors in whole cells by exploiting the CR binding phenotype. Briefly, S. *flexneri* 2a can be grown confluent on tryptic soy broth agar plates containing 0.025% CR (Sigma Chemical Co., St. Louis, MO). Various Mar inhibitors at a concentration of 50 ug/ml will be robotically spotted onto these plates in order to identify compounds that yield Cbr⁻ cells. Serial dilutions of compounds that produce the Cbr⁻ phenotype will be analyzed in subsequent assays in order to determine IC₅₀/EC₅₀ values.

In another embodiment, an apyrase zymogram assay can be used. It has been recently determined that S. *flexneri* and EIEC lacking *virF* are deficient for apyrase activity. Thus, the zymogram technique can be used to measure loss of *apy* activity in whole cell lysates as previously described (Berlutti et al. (1998) Infect. Immun. 66:4957-4964) of S. *flexneri* grown in the presence of the Mar inhibitors. Briefly, cells will be grown overnight in nutrient rich broth, washed, concentrated to OD₆₀₀ ≈40, and then disrupted via sonication. Cell debris will be removed with centrifugation and the lysates will be subjected to SDS-PAGE. The denaturing gels will then be soaked in renaturation buffer (50 mM Tris-HCl [pH 7.0], 1% [vol/vol] Triton X-100) to restore apyrase activity and equilibrated with 100 mM Tris-HCl [pH 7.5] for one hour and then 100 mM Tris-HCl-10 mM EDTA-1 mM ATP for 30 min. at 10°C. Subsequently, the gels will be soaked in a fresh 4:1 (vol/vol) solution of acidified ammonium molybdate (5 mM ammonium molybdate, 0.12 M sulfuric acid) and ascorbic acid (10%, wt/vol) to visualize apyrase activity.

In another embodiment, a S. typhimurium TTSS assay can be used. S. *typhimurium* secretes SptP through a TTSS and the expression of both SptP and the TTSS is regulated by InvF. The TTSS is presumably induced upon contact with host cells during infection and culture conditions that promote secretion of SptP into the culture medium have been identified. Optimal conditions are growth at 37°C with low aeration in LB media containing 0.3 M NaCl (Fu, Y., et al. (1999) Nature 401:293-7). The phosphatase activity of SptP has been measured biochemically in lysed cells using a ³²P-labelled peptide (Fu, Y., et al. (1999) Nature 401:293-7; Kaniga, K., et al. (1996) Mol Microbiol. 21:633-41) and will be used to monitor InvF function *in vitro.*

Briefly, cells will be grown in media to promote SptP secretion and the phosphatase activity of the protein will be monitored as described for *Y. enterocolitica* YopH and using a chemiluminescent (e.g., CSPD) or colorimetric (e.g., pNPP) substrate. Depending on the level of SptP secreted, these assays may be performed with cell lysates and ³²P-labelled peptide substrate as described (Fu, Y., et al. (1999) Nature 401:293-7; Kaniga, K., et al. (1996) Mol Microbiol. 21:633-41). In these assays, lysates will be prepared, incubated with the labeled peptide substrate, the phosphatase reaction will terminated with trichloroacetic acid, and acid soluble ³²P will be measured in a multi-channel scintillation counter in 96-well microtiter plates.

In another embodiment, a *Vibrio* enzyme-linked immunosorbent assay (ELISA) can be performed. The MarA (AraC) family member ToxT activates expression of several genes in the ToxR virulon including *ctxA* and *ctxB* encoding the subunits of cholera toxin (CT). CT production is dependent on ToxT as mutants in both the classical and El Tor biotype backgrounds lacking the helix-turn-helix DNA binding domain of ToxT *(toxT_{HTH})* fail to produce CT. The CT subunit B binds avidly to GM1-gangliosides on the surface of target cells *in vivo* and a GM1-based ELISA assay has been developed to detect CT *in V. cholerae* culture supernatants. This assay can be used to monitor *in vitro* ToxT function.

Briefly, bacteria can be grown in the presence of Mar inhibitors under conditions known to promote cholera toxin production: classical strain 0395 will be grown in LB (pH 6.5) shaking at 30°C and El Tor strain E7946 can be grown under AKI conditions. The wells of microtiter plates can be coated with purified GM1-ganglioside (Sigma Chemical Co., St. Louis, MO) and the plates will be washed and blocked with BSA prior to incubation with *V. cholerae* culture supernatants. Cholera toxin subunit B bound to the plate can be labeled with a mouse primary antibody (US Biological, Swampscott, MA) followed by labeling with an anti-mouse secondary antibody conjugated to horseradish peroxidase (Cell Signaling Technology, Beverly, MA). The horseradish peroxidase can then be detected using a chemiluminescent substrate and the signal can be detected using a plate reader. A series of diluted purified CT (Sigma Chemical Co., St. Louis, MO) will be used to determine the amount of CT in the culture samples. Additional controls can include ToxT null mutants of *V. cholerae* 0395 (0395::*toxT*_{HTH}) and *V. cholerae* E7946 (E7946:: *toxT*_{HTH}).

CT is composed of two subunits, CtxA and CtxB, and the expression of both is governed by ToxT, a MarA (AraC) family *member. V. cholerae toxT* null mutants, in both the classical (0395) and El Tor biotype backgrounds, fail to produce CT and are avirulent in an infant mouse model of infection.

CtxB binds GM1-ganglioside on the surface of target cells *in vivo* with high affinity and a GM1-based ELISA assay has been developed to detect CT *in V. cholerae* culture supernatants. This assay can be used to monitor *in vitro* ToxT function in wild type and *toxT* null mutants. Briefly, bacteria can be grown under conditions known to promote cholera toxin production [O395, LB broth (pH 6.5) at 30°C and El Tor, AKI media (1.5% Bacto Peptone, 0.4% yeast extract, 0.5% NaCl, and 0.3% sodium bicarbonate) standing at 37°C then followed by shaking at 37°C]. Culture supernatants can be added to microtiter plates coated with purified GM1-ganglioside (Sigma Chemical Co., St. Louis, MO) and blocked with BSA. CtxB bound to the plate was detected by first labeling with a mouse primary antibody (US Biological, Swampscott, MA) and then by labeling with an anti-mouse secondary antibody conjugated to horseradish peroxidase (Cell Signaling Technology, Beverly, MA). The horseradish peroxidase can be detected using a chemiluminescent substrate and the signal detected using a plate reader.

Wild type *V. cholerae* yields a robust signal while the *toxT* null mutant fails to elicit a response. The amount of CT in the culture samples was then quantitated using serial dilutions of purified CT (Sigma Chemical Co., St. Louis, MO). As illustrated, wild type *V. cholerae* yields ∼225 ng/ml CT while the *toxT* null mutant yields background levels of CT.

In another exemplary embodiment, a cytotoxicity assay can be used to investigate the ability of compounds to decrease virulence. For example, macrophage cytotoxicity can be measured by the release of the cytoplasmic housekeeping enzyme lactate dehydrogenase (LDH) using a commercially available kit (Promega, Madison, WI). The experiment can be conducted by first diluting a fresh overnight culture of an organism, e.g., *Y. pseudotuberculosis,* into LB containing sodium oxalate (inducing conditions) and growing 1 hr at 37 °C to induce synthesis of transcription factors and the secretion machinery. The bacterial cells are then washed in DMEM and added to a nearly confluent monolayer of macrophage cells, at a multiplicity of infection of 50 bacterial cells/macrophage cell. Test compounds are added at the appropriate concentrations and incubation is continued at 37 °C in a humidified 5% CO₂ atmosphere. After 5-6 hrs, LDH in the culture medium is measured using a colorimetric assay. Several controls can be included in the assay: a negative control of uninfected macrophage cells, a maximum release control in which uninfected cells have been lysed with detergent, and controls to show that the bacterial cells lack LDH activity. A reduction in the ability of microbial cells to cause toxicity the presence of a compound indicates that the compound modulates the expression and/or activity of a transcription factor.

### 2. Cell-Free Assays

The subject screening methods can also involve cell-free assays, e.g., using high-throughput techniques. For example, to screen for agonists or antagonists, a synthetic reaction mix comprising a transcription factor molecule and a labeled substrate or ligand of such polypeptide is incubated in the absence or the presence of a candidate molecule that may be an agonist or antagonist. In one embodiment, the reaction mix can further comprise a cellular compartment, such as a membrane, cell envelope or cell wall, or a combination thereof. The ability of the test compound to agonize or antagonize the transcription factor is reflected in decreased binding of the transcription factor to a transcription factor binding polypeptide or in a decrease in transcription factor activity.

In many drug screening programs which test libraries of modulating agents and natural extracts, high throughput assays are desirable in order to maximize the number of modulating agents surveyed in a given period of time. Assays which are performed in cell-free systems, such as maybe derived with purified or semipurified proteins, are often preferred as "primary" screens in that they can be generated to permit rapid development and relatively easy detection of an alteration in a molecular target which is mediated by a test modulating agent. Moreover, the effects of cellular toxicity and/or bioavailability of the test modulating agent can be generally ignored in the *in vitro* system.

In one embodiment, the ability of a compound to modulate the activity of a transcription factor is accomplished using isolated transcription factors or transcription factor nucleic acid molecule in a cell-free system. In such an assay, the step of measuring the ability of a compound to modulate the activity of the transcription factor is accomplished, for example, by measuring direct binding of the compound to a transcription factor or transcription factor nucleic acid molecule or the ability of the compound to alter the ability of the transcription factor to bind to a molecule to which the transcription factor normally binds (e.g., protein or DNA).

In yet another embodiment, an assay of the present invention is a cell-free assay in which a transcription factor or portion thereof is contacted with a test compound and the ability of the test compound to bind to the transcription factor or biologically active portion thereof is determined. Determining the ability of the test compound to modulate the activity of a transcription factor can be accomplished, for example, by determining the ability of the transcription factor to bind to a transcription factor target molecule by one of the methods described above for determining direct binding. Determining the ability of the transcription factor to bind to a transcription factor target molecule can also be accomplished using a technology such as real-time Biomolecular Interaction Analysis (BIA). Sjolander, S. and Urbaniczky, C. (1991) Anal. Chem. 63:2338-2345 and Szabo et al. (1995) Curr. Opin. Struct. Biol. 5:699-705. As used herein, "BIA" is a technology for studying biospecific interactions in real time, without labeling any of the interactants (e.g., BIAcore). Changes in the optical phenomenon of surface plasmon resonance (SPR) can be used as an indication of real-time reactions between biological molecules.

In yet another embodiment, the cell-free assay involves contacting a transcription factor or biologically active portion thereof with a known compound which binds the transcription factor to form an assay mixture, contacting the assay mixture with a test compound, and determining the ability of the test compound to interact with the transcription factor, wherein determining the ability of the test compound to interact with the transcription factor comprises determining the ability of the transcription factor to preferentially bind to or modulate the activity of a transcription factor target molecule.

The cell-free assays of the present invention are amenable to use of both soluble and/or membrane-bound forms of proteins (e.g., transcription factors or transcription factor binding polypeptides). In the case of cell-free assays in which a membrane-bound form of a polypeptide is used it may be desirable to utilize a solubilizing agent such that the membrane-bound form of the polypeptide is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton^{®} X-100, Triton^{®} X-114, Thesit^{®}, Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

For example, compounds can be tested for their ability to directly bind to a transcription factor nucleic acid molecule or a transcription factor or portion thereof, e.g., by using labeled compounds, e.g., radioactively labeled compounds. For example, a transcription factor sequence can be expressed by a bacteriophage. In this embodiment, phage which display the transcription factor would then be contacted with a compound so that the polypeptide can interact with and potentially form a complex with the compound. Phage which have formed complexes with compounds can then be separated from those which have not. The complex of the polypeptide and compound can then be contacted with an agent that dissociates the bacteriophage from the compound. Any compounds that bound to the polypeptide can then be isolated and identified.

Readouts which involve fluorescence resonance energy transfer (FRET) can also be employed in the instant assays. FRET occurs when one fluorophore, the donor, absorbs a photon and transfers the absorbed energy non-radiatively to another fluorophore, the acceptor. The acceptor then emits the energy at its characteristic wavelength. The donor and acceptor molecules must be in close proximity, less than approximately 10 nm, for efficient energy transfer to occur (see Methods Enzymol. 211, 353-388 (1992); Methods Enzymol. 246, 300-334 (1995)). The proximity requirement can be used to construct assays sensitive to small separations between the donor-acceptor pair. FRET typically requires a single excitation wavelength and two emission wavelengths, and an analysis consisting of the ratio of the donor and acceptor emission intensities. FRET donor acceptor pairs can be constructed for both bead-based assays and cell-based assays. Several green fluorescent protein (GFP) mutants displaying enhanced fluorescence and altered emission wavelengths can be paired for FRET cell-based assays by fusing the GFP FRET donor to one protein, e.g., a transcription factor and the GFP FRET acceptor to a promoter sequence to which the transcription factor binds.

For example, time resolved-fluorescence resonance energy transfer (TR-FRET) technique (e.g., Hillisch et al. 2001. Curr Opin Struct Biol 11:201) to measure the *in vitro* DNA binding activity of various MarA (AraC) family members. With this technique, a biotinylated double-stranded DNA molecule is incubated with a MarA (AraC) protein fused to 6-histidine (6-His) residues, which facilitates purification and immunoprecipitation using nickel agarose and anti-6-His antibodies, respectively. A europium-labeled anti-6His antibody binds the protein and a streptavidin conjugated allophycocyanin (APC) complex binds the DNA. The europium molecule is excited at 340 nm and if it is in close proximity to the APC (10-100Å) there will be a FRET from the 615 nm emission of europium to APC. The energy emitted from the excited APC is then recorded at 665 nm. (The europium and APC are termed FRET pairs.) Compounds that inhibit the binding of protein to DNA, and therefore result in the physical separation of the FRET pairs, are identified by a reduced emission at 665 nm. This assay is particularly well suited to investigate the function of MarA (AraC) family members from *Yersinia* spp.

Luminescence can be read, e.g., using a Victor V plate reader (PerkinElmer Life Sciences, Wellesley, MA). Compounds that inhibit the binding of the protein to the DNA result in a loss of protein from the plate at the first wash step and are identified by a reduced luminescence signal. The concentration of compound necessary to reduce signal by 50% (EC₅₀/IC₅₀) can be calculated using serial dilutions of the inhibitory compounds.

The fluorescence marker can be attached to a member of the binding pair (e.g., the transcription factor or the DNA molecule) either directly or indirectly. For example, one can covalently attach the marker to a molecule of interest. Methods of forming a linkage between an oligonucleotide and or protein are known to those of skill in the art. One suitable method involves incorporating into the marker (preferably in the loop portion) an amino-dT residue. This can then be conjugated using a chemical linker to a functional group (e.g., an amine group) on the molecule of interest (see, e.g., Partis et al. (1983) J. Prot. Chem. 2: 263-277). Alternatively, the marker can be attached to the molecule of interest indirectly by noncovalent means. For example, the molecular beacon can be attached to a binding moiety (e.g., an antibody) that binds to the binding pair member of interest.

Other methods of assaying the ability of proteins to bind to DNA, e.g., DNA footprinting, and nuclease protection are also well known in the art and can be used to test the ability of a compound to bind to a transcription factor nucleotide sequence.

In another embodiment, the invention provides a method for identifying compounds that modulate antibiotic resistance by assaying for test compounds that bind to transcription factor nucleic acid molecules and interfere, e.g., with gene transcription.

In another embodiment, a transcription factor nucleic acid molecule and a transcription factor binding polypeptide that normally binds to that nucleotide sequence are contacted with a test compound to identify compounds that block the interaction of a transcription factor nucleic acid molecule and a transcription factor binding polypeptide. For example, in one embodiment, the transcription factor nucleotide sequence and/or the transcription factor binding polypeptide are contacted under conditions which allow interaction of the compound with at least one of the transcription factor nucleic acid molecule and the transcription factor binding polypeptide. The ability of the compound to modulate the interaction of the transcription factor nucleotide sequence with the transcription factor binding polypeptide is indicative of its ability to modulate a transcription factor activity.

Determining the ability of the transcription factor to bind to or interact with a transcription factor binding polypeptide can be accomplished, e.g., by direct binding. In a direct binding assay, the transcription factor could be coupled with a radioisotope or enzymatic label such that binding of the transcription factor to a transcription factor target molecule can be determined by detecting the labeled transcription factor in a complex. For example transcription factors can be labeled with ¹²⁵I, ³⁵S ¹⁴C, or ³H, either directly or indirectly, and the radioisotope detected by direct counting of radioemmission or by scintillation counting. Alternatively, transcription factor molecules can be enzymatically labeled with, for example, horseradish peroxidase, alkaline phosphatase, or luciferase, and the enzymatic label detected by determination of conversion of an appropriate substrate to product.

In one embodiment, the ability of a compound to bind to a transcription factor nucleic acid molecule can be measured. For example, gel shift assays or restriction enzyme protection assays can be used. Gel shift assays separate polypeptide-DNA complexes from free DNA by non-denaturing polyacrylamide gel electrophoresis. In such an experiment, the level of binding of a compound to DNA can be determined and compared to that in the absence of compound. Compounds which change the level of this binding are selected in the screen as modulating the activity of a transcription factor. In another embodiment, a qualitative assay of the activity of a candidate transcription factor modulating compound by measuring their ability to interrupt DNA-protein interactions *in vitro* can be used. Briefly, 5 nM of a MarA (AraC) family member (or a concentration where ~50% of a radiolabeled (³³P) double-stranded DNA probe is bound to the protein) is incubated for 30 min at room temperature either in the absence (DMSO (solvent) alone) or presence of a test compound. Subsequently, 0.1 nM of the (³³P) labeled DNA probe is added and the mixture is allowed to equilibrate for 15 min at room temperature. The mixture is then resolved on a non-denaturing polyacrylamide gel and the gel is analyzed by autoradiography.

Typically, it will be desirable to immobilize either transcription factor, a transcription factor binding polypeptide or a compound to facilitate separation of complexes from uncomplexed forms, as well as to accommodate automation of the assay. Binding of transcription factor to an upstream or downstream binding polypeptide, in the presence and absence of a candidate agent, can be accomplished in any vessel suitable for containing the reactants. Examples include microtitre plates, test tubes, and micro-centrifuge tubes. In one embodiment, a fusion protein can be provided which adds a domain that allows the polypeptide to be bound to a matrix.

For example, glutathione-S-transferase/ transcription factor (GST/transcription factor) fusion proteins can be adsorbed onto glutathione sepharose beads (Sigma Chemical, St. Louis, MO) or glutathione derivatized microtitre plates, which are then combined with the cell lysates, e.g. an ³⁵S-labeled, and the test modulating agent, and the mixture incubated under conditions conducive to complex formation, e.g., at physiological conditions for salt and pH, though slightly more stringent conditions may be desired. Following incubation, the beads are washed to remove any unbound label, and the matrix immobilized and radiolabel determined directly (e.g. beads placed in scintilant), or in the supernatant after the complexes are subsequently dissociated. Alternatively, the complexes can be dissociated from the matrix, separated by SDS-PAGE, and the level of transcription factor -binding polypeptide found in the bead fraction quantitated from the gel using standard electrophoretic techniques.

Other techniques for immobilizing proteins on matrices are also available for use in the subject assay. For instance, either a transcription factor or polypeptide to which it binds can be immobilized utilizing conjugation of biotin and streptavidin. For instance, biotinylated transcription factor molecules can be prepared from biotin-NHS (N-hydroxy-succinimide) using techniques well known in the art (e.g., biotinylation kit, Pierce Chemicals, Rockford, IL), and immobilized in the wells of streptavidin-coated 96 well plates (Pierce Chemical). Alternatively, antibodies reactive with transcription factor but which do not interfere with binding of upstream or downstream elements can be derivatized to the wells of the plate, and transcription factor trapped in the wells by antibody conjugation. As above, preparations of a transcription factor -binding polypeptide and a test modulating agent are incubated in the transcription factor -presenting wells of the plate, and the amount of complex trapped in the well can be quantitated. Exemplary methods for detecting such complexes, in addition to those described above for the GST-immobilized complexes, include immunodetection of complexes using antibodies reactive with the transcription factor binding polypeptide, or which are reactive with transcription factor and compete with the binding polypeptide; as well as enzyme-linked assays which rely on detecting an enzymatic activity associated with the binding polypeptide, either intrinsic or extrinsic activity. In the instance of the latter, the enzyme can be chemically conjugated or provided as a fusion protein with the transcription factor binding polypeptide. To illustrate, the transcription factor can be chemically cross-linked or genetically fused with horseradish peroxidase, and the amount of protein trapped in the complex can be assessed with a chromogenic substrate of the enzyme, e.g. 3,3'-diamino-benzadine terahydrochloride or 4-chloro-1-napthol. Likewise, a fusion protein comprising the protein and glutathione-S-transferase can be provided, and complex formation quantitated by detecting the GST activity using 1-chloro-2,4-dinitrobenzene (Habig et al (1974) J Biol Chem 249:7130).

For processes which rely on immunodetection for quantitating one of the proteins trapped in the complex, antibodies against the polypeptide, such as anti-transcription factor antibodies, can be used. Alternatively, the polypeptide to be detected in the complex can be "epitope tagged" in the form of a fusion protein which includes, in addition to the transcription factor sequence, a second polypeptide for which antibodies are readily available (e.g. from commercial sources). For instance, the GST fusion proteins described above can also be used for quantification of binding using antibodies against the GST moiety. Other useful epitope tags include myc-epitopes (e.g., see Ellison et al. (1991) J Biol Chem 266:21150-21157) which includes a 10-residue sequence from c-myc, as well as the pFLAG system (International Biotechnologies, Inc.) or the pEZZ-protein A system (Pharamacia, NJ).

It is also within the scope of this invention to determine the ability of a compound to modulate the interaction between transcription factor and its target molecule, without the labeling of any of the interactants. For example, a microphysiometer can be used to detect the interaction of transcription factor with its target molecule without the labeling of either transcription factor or the target molecule. McConnell, H. M. et al. (1992) Science 257:1906-1912. As used herein, a "microphysiometer" (e.g., Cytosensor) is an analytical instrument that measures the rate at which a cell acidifies its environment using a light-addressable potentiometric sensor (LAPS). Changes in this acidification rate can be used as an indicator of the interaction between compound and receptor.

The invention also pertains to the use of molecular design techniques to design transcription factor modulating compounds, e.g., HTH protein modulating compounds, AraC family modulating compounds, MarA family modulating compounds, or MarA modulating compounds, which are capable of binding or interacting with one or more transcription factors (e.g., of a prokaryotic or eukaryotic organism). The invention pertains to both the transcription factor modulating compounds identified by the methods as well as the modeling methods, and compositions comprising the compounds identified by the methods.

In an embodiment, the invention pertains to a method of identifying transcription factor modulating compounds. The method includes obtaining the structure of a transcription factor of interest, and using GLIDE to identify a scaffold which has an interaction energy score of-20 or less (e.g., -40 or less, e.g., -60 or less) with a portion of the transcription factor.

### 3. Structure Based Drug Design

The invention also pertains, at least in part, to a computational screening of small molecule databases for chemical entities or compounds that can bind in whole, or in part, to a transcription factor, such as a HTH protein, an AraC family polypeptide, a MarA family polypeptide, e.g., MarA. In this screening, the quality of fit of such entities or compounds to the binding site may be judged either by shape complementarity or by estimated interaction energy (Meng, E. C. et al., 1992, J. Coma. Chem., 13:505-524). Such a procedure allows for the screening of a very large library of potential transcription factor modulating compounds for the proper molecular and chemical complementarities with a selected protein or class or proteins.

Transcription factor modulating compounds identified through computational screening can later be passed through the *in vivo* assays described herein as further screens. For example, a transcription factor inhibiting compound identified through computational screening could be tested for its ability to promote cell survival in a cell system containing a counterselectable marker under the control a transcription factor activated promoter. The promotion of cell survival in the foregoing assay would be indicative of a compound that inhibits the of the transcription factor. Other suitable assays are known in the art.

The crystal structures of both MarA (PDB m code 1BL0) and its homolog Rob (PDB ID code 1DY5) are available in the Protein Data Bank. These structures were used to identify sites on the proteins that could be targeted by small molecule chemical inhibiting compounds. A total of at least eight potential small molecule binding sites on MarA (Table 4) and four sites on Rob (Table 5) were identified as potential small molecule binding sites. The invention pertains, at least in part, to MarA modulating compounds which interact with any one of the following sites of MarA (based on the sequence given in SEQ ID NO. 2).

**Table 4**

| Site Number | Residues (based on full length MarA) | Site Label |
|---|---|---|
| 1 | 42 to 50 | R46 Major Groove |
| 2 | 54 to 62 | L56 HTH core |
| 3 | 55 to 65 | R61 Minor Groove |
| 4 | 15 to 25 | W19 |
| 5 | 14 to 25 | E21 |
| 6 | 24 to 35 | L28 |
| 7 | 76 to 83 | P78 |
| 8 | 106 to 112 | R110 |

The GLIDE docking method was then used to fit combinatorial chemistry scaffolds into these sites and an interaction energy was calculated for each. Eight scaffolds were predicted to bind to site 1, encompassing amino acids tryptophan 42 to lysine 50, with an interaction energy score of-60 or less. These scaffolds are shown below: 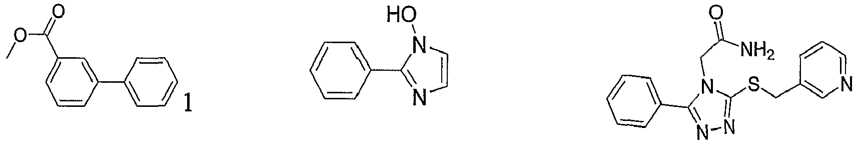 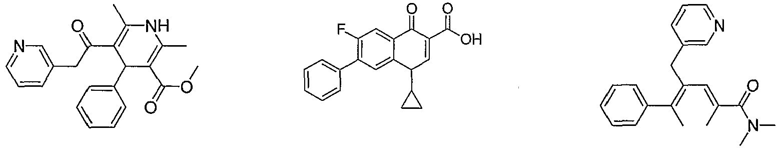 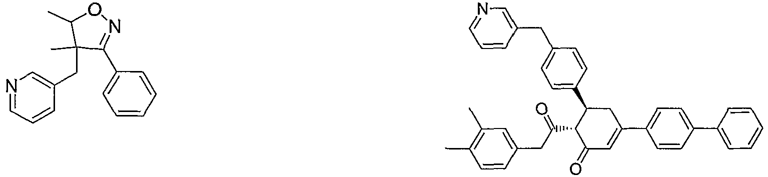 Three scaffolds were identified for site 2 of MarA (e.g., residues histidine 54 to serine 62). 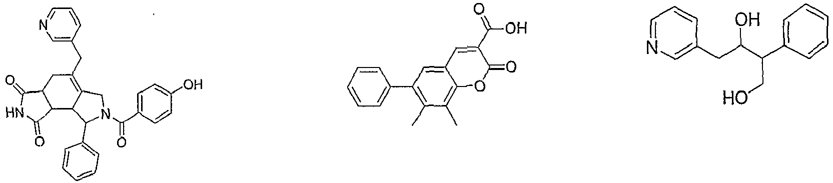 Four scaffolds were identified for MarA site 3, (e.g., residues serine 55 to methionine 65): 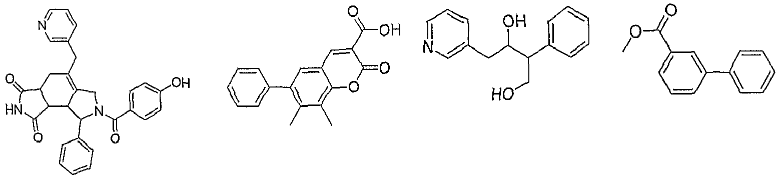 Six scaffolds were identified for site 6 (e.g., residues leucine 24 to glutamate 35). 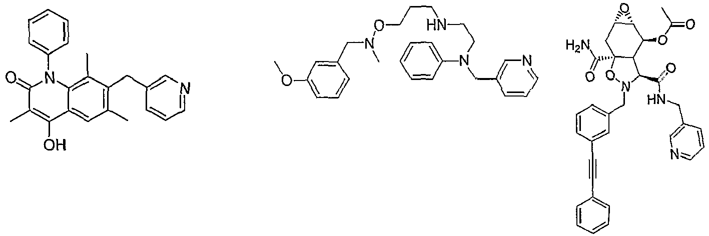 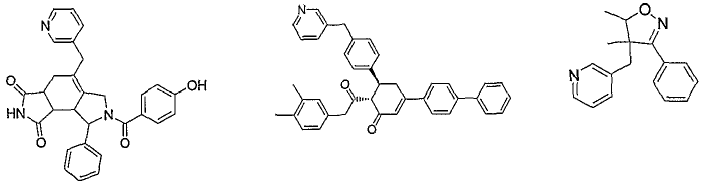 These scaffolds were then used to search the CambridgeSoft ACX-SC database of over 600,000 non-proprietary chemical structures and the number of chemicals similar to the scaffolds was determined.

The term "scaffold" includes the compounds identified by the computer modeling program. These compounds may or may not be themselves transcription factor modulating compounds. An ordinarily skilled artisan will be able to analyze a scaffold obtained from the computer modeling program and modify the scaffold such that the resulting compounds have enhanced chemical properties over the initial scaffold compound, e.g., are more stable for administration, less toxic, have enhanced affinity for a particular transcription factor, etc. The invention pertains not only to the scaffolds identified, but also the transcription factor modulating compounds which are developed using the scaffolds.

Table 5 lists portions of Rob which were identified as possible interaction sites for a modulating compound. The invention pertains, at least in part, to any compounds modeled to bind to these regions of Rob. The numbering corresponds to that given in SEQ ID NO. 4.

**Table 5**

| Site Number | Residues (based on full length Rob) | Site Label |
|---|---|---|
| 1 | 37 to 45 | R40 Major Groove |
| 2 | 43 to 54 | 150 HTH Core |
| 3 | 51 to 60 | R55 Minor Groove |
| 4 | 10 to 20 | W13 |

These scaffolds were identified as possible modulating compounds which with site 1 of Rob (residues 37-45), a MarA family polypeptide.

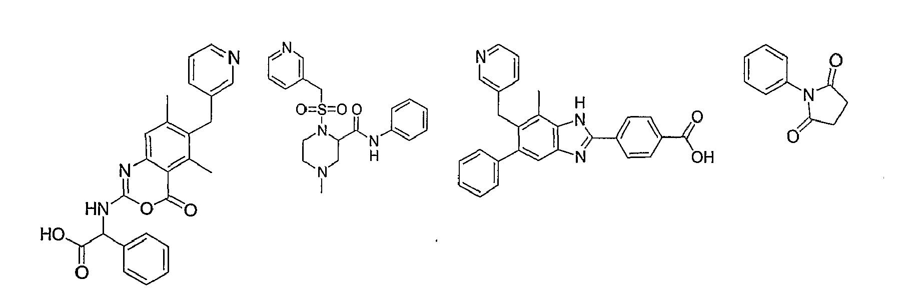

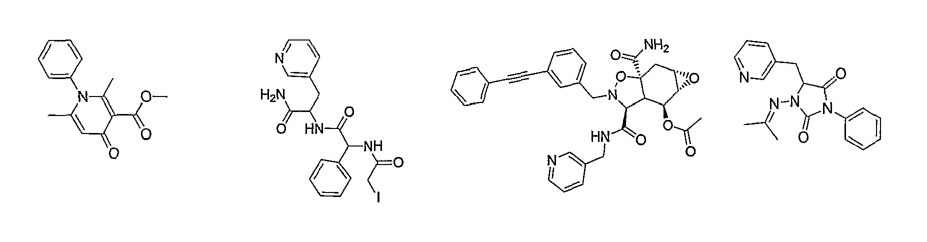

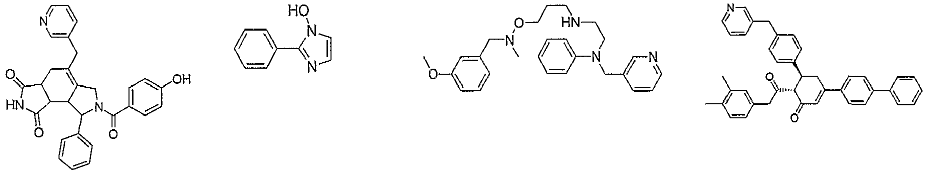

These scaffolds were identified as small molecules that may interact with site 2 of Rob (residues 43-52), a MarA family polypeptide.

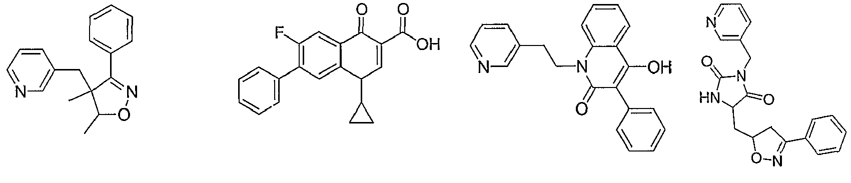

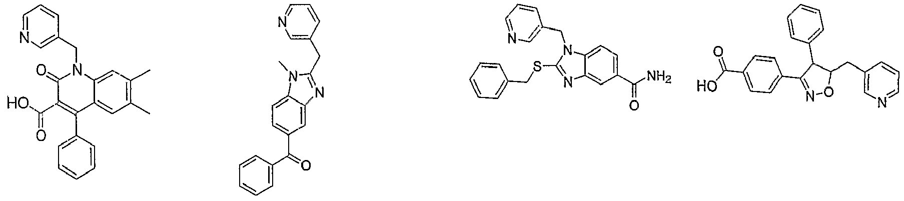

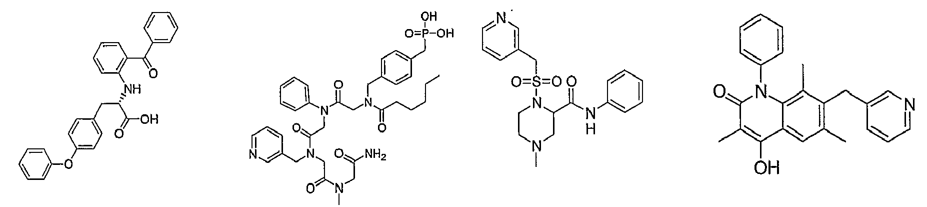

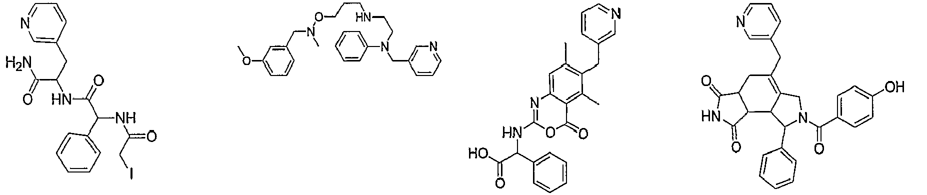

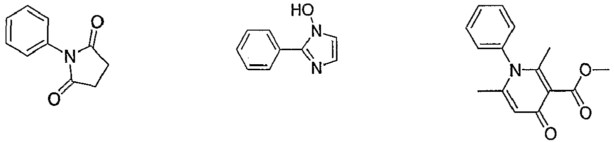

The design of compounds that bind to, modulate, or inhibit transcription factors, generally involves consideration of two factors. First, the compound must be capable of physically and structurally associating with a particular transcription factor. Non-covalent molecular interactions important in the association of a transcription factor with a modulating compound include hydrogen bonding, van der Waals and hydrophobic interactions.

Second, the modulating compound must be able to assume a conformation that allows it to associate with the selected transcription factor. Although certain portions of the inhibiting compound will not directly participate in this association with the transcription factor, those portions may still influence the overall conformation of the molecule. This, in tum, may have a significant impact on potency. Such conformational requirements include the overall three-dimensional structure and orientation of the chemical entity or compound in relation to all or a portion of the binding site, e.g., active site or accessory binding site of a particular transcription factor such as MarA, or the spacing between functional groups of a compound comprising several chemical entities that directly interact with the particular transcription factor.

In a further embodiment, the potential modulating effect of a chemical compound on a selected transcription factor (e.g., a HTH protein, a AraC family polypeptide, a MarA family polypeptide, e.g., MarA) is analyzed prior to its actual synthesis and testing by the use of computer modeling techniques. If the theoretical structure of the given compound suggests insufficient interaction and association between it and the selected transcription factor, synthesis and testing of the compound is avoided. However, if computer modeling indicates a strong interaction, the molecule may then be synthesized and tested for its ability to bind to the selected transcription factor and modulate the transcription factor's activity.

A transcription factor modulating compound or other binding compound (e.g., an HTH protein modulating compound, an AraC family polypeptide modulating compound, or a MarA family inhibiting compound, e.g., a MarA inhibiting compound) may be computationally evaluated and designed by screening and selecting chemical entities or fragments for their ability to associate with the individual small molecule binding sites or other areas of a transcription factor.

One skilled in the art may use one of several methods to screen chemical entities or fragments for their ability to associate with a selected transcription factor and more particularly with the individual small molecule binding sites of the particular transcription activation factor. This process may begin by visually inspecting the structure of the transcription factor on a computer screen based on the atomic coordinates of the transcription factor crystals. Selected chemical entities may then be positioned in a variety of orientations, or docked, within an individual binding site of the transcription factor. Docking may be performed using software such as Quanta and Sybyl, followed by energy minimization with standard molecular mechanics forcefields or dynamics with programs such as CHARMM (Brooks, B. R. et al., 1983, J. Comp. Chem., 4:187-217) or AMBER (Weiner, S. J. et al., 1984, J. Am. Chem. Soc., 106:765-784).

Specialized computer programs may also assist in the process of selecting molecules that bind to a selected transcription factor, (e.g., an HTH protein, an AraC family polypeptide, or a MarA family polypeptide, e.g., MarA). The programs include, but are not limited to:
1.GRID (Goodford, P. J., 1985, "A Computational Procedure for Determining Energetically Favorable Binding Sites on Biologically Important Macromolecules" J. Med. Chem., 28:849-857 GRID is available from Oxford University, Oxford, UK.
2.AUTODOCK (Goodsell, D. S. and A. J. Olsen, 1990, "Automated Docking of Substrates to Proteins by Simulated Annealing" Proteins: Structure. Function, and Genetics, 8:195-202. AUTODOCK is available from Scripps Research Institute, La Jolla, Calif. AUTODOCK helps in docking inhibiting compounds to a selected transcription factor in a flexible manner using a Monte Carlo simulated annealing approach. The procedure enables a search without bias introduced by the researcher.
3.MCSS (Miranker, A. and M. Karplus, 1991, "Functionality Maps of Binding Sites: A Multiple Copy Simultaneous Search Method." Proteins: Structure, Function and Genetics, 11:29-34). MCSS is available from Molecular Simulations, Burlington, Mass.
4.MACCS-3D (Martin, Y. C., 1992, J. Med. Chem., 35:2145-2154) is a 3D database system available from MDL Information Systems, San Leandro, Calif.
5.DOCK (Kuntz, I. D. et al., 1982, "A Geometric Approach to Macromolecule-Ligand Interactions" J. Mol. Biol., 161:269-288). DOCK is available from University of California, San Francisco, Calif. DOCK is based on a description of the negative image of a space-filling representation of the molecule (i.e. the selected transcription factor) that should be filled by the inhibiting compound. DOCK includes a force-field for energy evaluation, limited conformational flexibility and consideration of hydrophobicity in the energy evaluation.
6.MCDLNG (Monte Carlo De Novo Ligand Generator) (D. K. Gehlhaar, et al. 1995. J. Med. Chem. 38:466-472). MCDLNG starts with a structure (i.e. an X-ray crystal structure) and fills the binding site with a close packed array of generic atoms. A Monte Carlo procedure is then used to randomly: rotate, move, change bond type, change atom type, make atoms appear, make bonds appear, make atoms disappear, make bonds disappear, etc. The energy function used by MCDLNG favors the formation of rings and certain bonding arrangements. Desolvation penalties are given for heteroatoms, but heteroatoms can benefit from hydrogen bonding with the binding site.

In an embodiment of the invention, docking is performed by using the Affinity program within InsightII (Molecular Simulations Inc., 1996, San Diego, Calif., now Accelrys Inc.). Affinity is a suite of programs for automatically docking a ligand (i.e. a transcription factor modulating compound) to a receptor (i.e. a transcription factor). Commands in Affinity automatically find the best binding structures of the ligand to the receptor based on the energy of the ligand/receptor complex. As described below,

Affinity allows for the simulation of flexible-flexible docking.
Affinity consists of two commands, GridDocking and fixedDocking, under the new pulldown Affinity in the Docking module of the Insight II program. Both commands use the same, Monte Carlo type procedure to dock a guest molecule (i.e. HTH protein modulating compound) to a host (i.e., a transcription factor). They also share the feature that the "bulk" of the receptor (i.e. transcription factor), defined as atoms not in the binding (active) site specified, is held rigid during the docking process, while the binding site atoms and ligand atoms are movable. The commands differ, however, in their treatment ofnonbond interactions. In GridDocking, interactions between bulk and movable atoms are approximated by the very accurate and efficient molecular mechanical/grid (MM/Grid) method developed by Luty et al. 1995. J. Comp. Chem. 16:454, while interactions among movable atoms are treated exactly. GridDocking also includes the solvation method of Stouten et al. 1993. Molecular Simulation 10:97. On the other hand, the fixedDocking command computes nonbond interactions using methods in the Discover program (cutoff methods and the cell multipole method) and it does not include any solvation terms.
Affinity does not, generally, require any intervention from the user during the docking. It automatically moves the ligand (i.e. modulating compound), evaluates energies, and checks if the structure is acceptable. Moreover, the ligand and the binding site of the receptor (i.e. the selected transcription modulator) are flexible during the search.

Most of the docking methods in the literature are based on descriptors or empirical rules (for a review see Kuntz et al. 1994. Acc. Chem. Res. 27:117. These include DOCK (Kuntz et al. 1982. J. Mol. Biol. 161:269., Shoichet et al. 1992. J. Compt. Chem. 13:380., Oshiro et al. 1995. J. Comp. Aided Molec. Design 9:113.), CAVEAT (Bartlett et al. 1989. "Chemical and Biological Problems in Molecular Recognition" Royal Society of Chemistry: Cambridge, pp. 182-196., Lauri & Bartlett. 1994. J. Comput. Aided Mol. Design 8:51), FLOG (Miller et al. 1994. J. Comp. Aided Molec. Design 8:153), and PRO_LIGAND (Clark et al. 1995. J. Comp. Aided Molec. Design 9:13), to name a few. Affinity differs from these methods in several aspects.

First, it uses full molecular mechanics in searching for and evaluating docked structures. In contrast descriptor-based methods use empirical rules which usually take into account only hydrogen bonding, hydrophobic interactions, and steric effects. This simplified description of ligand/receptor interaction is insufficient in some cases. For example, Meng et al. 1992. J. Compt. Chem. 13:505 studied three scoring methods in evaluating docked structures generated by DOCK. They found that only the forcefield scores from molecular mechanics correctly identify structures closest to experimental binding geometry, while scoring functions that consider only steric factors or only electrostatic factors are less successful. Note that in the study by Meng et al. 1992.J. Compt. Chem. 13:505*,* docking was still performed using descriptors. Affinity, on the other hand, uses molecular mechanics in both docking and scoring and is therefore more consistent.

Second, in Affinity, while the bulk of the receptor is fixed, the defined binding site is free to move, thereby allowing the receptor to adjust to the binding of different ligands or different binding modes of the same ligand. By contrast, almost all of the descriptor-based methods fix the entire receptor.

Third, the ligand itself is flexible in Affinity which permits different conformations of a ligand (i.e. transcription factor modulating compound) to be docked to a receptor (*i.e*. transcription factor). Recently Oshiro et al. (1995 J. Comp. Aided Molec. Design 9;113) extended DOCK to handle flexible ligands. FLOG is also able to treat flexible ligand by including different conformations for each structure in the database (Miller et al. 1995. J. Comp. Aided Molec. Design. 8:153). Most other methods are limited to rigid ligands.

There are also a few energy based docking methods (Kuntz et al. 1994. Acc. Chem Res. 27:117). These methods use either molecular dynamics (notably simulated annealing) or Monte Carlo methods. For example, Caflisch et al. 1992. Proteins: Struct. Funct. and Genetics 13:223) developed a two step procedure for docking flexible ligands. In their procedure, ligand is first docked using a special energy function designed to remove bad contact between the ligand and the receptor efficiently. Then Monte Carlo minimization (Li & Scheraga. 1987. Proc. Natl. Acad. Sci. U.S.A. 84:6611) is carried out to refine the docked structures using molecular mechanics. Hart and Read. 1992. Proteins: Struct. Funct. and Genetics 13:206 also employ two steps to dock ligands. They use a score function based on receptor geometry to approximately dock ligands in the first step, and then use Monte Carlo minimization similar to that of Caflisch et al. 1992. Proteins: Struct. Funct. and Genetics 13:223 for the second step. The method by Mizutani et al. (1994. J. Mol. Biol. 243:310) is another variation of this two step method.

Affinity uses a Monte Carlo procedure in docking ligands, but there are important distinctions over the prior art methods. First, the Monte Carlo procedure in Affinity can be used in conjunction either with energy minimization (to mimic the Monte Carlo minimization method of Li & Scheraga. 1987. Proc. Natl. Acad. Sci. U.S.A. 84:6611) or with molecular dynamics (to mimic the hybrid Monte Carlo method, Clamp et al. 1994. J. Comput. Chem. 15:838, or the smart Monte Carlo method, Senderowitz et al. 1995. J. Am. Chem. Soc. 117:8211). This flexibility allows Affinity to be applied to a variety of docking problems. Second, in the initial screening of docked structures, Affinity employs energy differences obtained from molecular mechanics, while the methods discussed above use empirical rules or descriptors. Therefore, Affinity is more consistent in that it uses molecular mechanics in both initial screening and final refinement of docked structures. Third, Affinity allows the binding site of the receptor to relax, while the methods discussed above fix the entire receptor. Fourth, Affinity employs two new nonbond techniques which are both accurate and efficient to make docking practical. One is the Grid/MM method of Luty *et al.* which represents the bulk of the receptor by grids (Luty et al. 1995. J. Comp. Chem. 16:454). This method is 10-20 times faster than the no-cutoff method with almost no loss in accuracy. It also incorporates the solvation method of Stouten et al. (1993. Molecular Simulation 10:97). The other is the cell multipole method. This method is about 50% slower than the Grid/MM method, but it does not require grid setup. Thus, a typical docking calculation takes about 1-3 hours of CPU time on an Indigo R4400 workstation.

Once suitable chemical fragments have been selected, they can be assembled into a single compound or inhibiting compound. Assembly may be proceed by visual inspection of the relationship of the fragments to each other on a three-dimensional image display on a computer screen in relation to the structure coordinates of a particular transcription factor, e.g., MarA. This may be followed by manual model building using software such as Quanta or Sybyl. Useful programs to aid one of skill in the art in connecting the individual chemical fragments include:
1. 3D Database systems such as MACCS-3D (MDL Information Systems, San Leandro, Calif. This area is reviewed in Martin, Y. C., 1992, "3D Database Searching in Drug Design", J. Med. Chem., 35, pp. 2145-2154).
2.CAVEAT (Bartlett, P. A. et al, 1989, "CAVEAT: A Program to Facilitate the Structure-Derived Design of Biologically Active Molecules". In Molecular Recognition in Chemical and Biological Problems", Special Pub., Royal Chem. Soc., 78, pp. 182-196). CAVEAT is available from the University of California, Berkeley, Calif. CAVEAT suggests inhibiting compounds to MarA based on desired bond vectors.
3.HOOK (available from Molecular Simulations, Burlington, Mass.). HOOK proposes docking sites by using multiple copies of functional groups in simultaneous searches.

In another embodiment, transcription factor modulating compounds may be designed as a whole or "de novo" using either an empty active site or optionally including some portion(s) of a known inhibiting compound(s). These methods include:
1. LUDI (Bohm, H.-J., "The Computer Program LUDI: A New Method for the De Novo Design of Enzyme Inhibiting compounds", J. ComR. Aid. Molec. Design, 6, pp. 61-78 (1992)). LUDI is available from Biosym Technologies, San Diego, Calif. LUDI is a program based on fragments rather than on descriptors. LUDI proposes somewhat larger fragments to match with the interaction sites of a macromolecule and scores its hits based on geometric criteria taken from the Cambridge Structural Database (CSD), the Protein Data Bank (PDB) and on criteria based on binding data. LUDI is a library based method for docking fragments onto a binding site. Fragments are aligned with 4 directional interaction sites (lipophilic-aliphatic, lipophilic-aromatic, hydrogen donor, and hydrogen acceptor) and scored for their degree of overlap. Fragments are then connected (i.e. a linker of the proper length is attached to each terminal atom in the fragments). Note that conformational flexibility can be accounted for only by including multiple conformations of a particular fragment in the library.
2. LEGEND (Nishibata, Y. and A. Itai, Tetrahedron, 47, p. 8985 (1991)). LEGEND is available from Molecular Simulations, Burlington, Mass.
3.CoMFA (Conformational Molecular Field Analysis) (J. J. Kaminski. 1994. Adv. Drug Delivery Reviews 14:331-337.) CoMFA defines 3-dimensional molecular shape descriptors to represent properties such as hydrophobic regions, sterics, and electrostatics. Compounds from a database are then overlaid on the 3D pharmacophore model and rated for their degree of overlap. Small molecule databased that be searched include: ACD (over 1,000,000 compounds), Maybridge (about 500,000 compounds), NCI (about 500,000 compounds), and CCSD. In measuring the goodness of the fit, molecules can either be fit to the 3D molecular shape descriptors or to the active conformation of a known inhibiting compound.
4.LeapFrog (available from Tripos Associates, St. Louis, Mo.).

FlexX (© 1993-2002 GMD German National Research Center for Information Technology; Rarey, M. et al J. Mol. Biol., 261:407-489) is a fast, flexible docking method that uses an incremental construction algorithm to place ligands into and active site of the transcription factor. Ligands (e.g., transcription factor modulating compounds) that are capable of "fitting" into the active site are then scored according to any number of available scoring schemes to determine the quality of the complimentarity between the active site and ligand.

Other molecular modeling techniques may also be employed in accordance with this invention. See, e.g., Cohen, N. C. et al., "Molecular Modeling Software and Methods for Medicinal Chemistry, J. Med. Chem., 33, pp. 883-894 (1990). See also, Navia, M. A. and M. A. Murcko, "The Use of Structural Information in Drug Design", Current Opinions in Structural Biology, 2, pp. 202-210 (1992).

Candidate transcription factor modulating compounds can be evaluated for their modulating, e.g., inhibitory or stimulatory, activity using conventional techniques which may involve determining the location and binding proximity of a given moiety, the occupied space of a bound inhibiting compound, the deformation energy of binding of a given compound and electrostatic interaction energies. Examples of conventional techniques useful in the above evaluations include, but are not limited to, quantum mechanics, molecular dynamics, Monte Carlo sampling, systematic searches and distance geometry methods (Marshall, G. R., 1987, Ann. Ref. Pharmacol. Toxicol., 27:193). Examples of computer programs for such uses include, but are not limited to, Gaussian 92, revision E2 (Gaussian, Inc. Pittsburgh, Pennsylvania), AMBER version 4.0 (University of California, San Francisco), QUANTA/CHARMM (Molecular Simulations, Inc., Burlington, Mass.), and Insight 11/Discover (Biosym Technologies Inc., San Diego, Calif.). These programs may be implemented, for example, using a Silicon Graphics Indigo2 workstation or IBM RISC/6000 workstation model 550. Other hardware systems and software packages will be known and of evident applicability to those skilled in the art.

Once a compound has been designed and selected by the above methods, the efficiency with which that compound may bind to a particular transcription factor may be tested and optimized by computational evaluation. An effective transcription factor modulating compound should demonstrate a relatively small difference in energy between its bound and free states (i.e., a small deformation energy of binding). Transcription factor modulating compounds may interact with the selected transcription factor in more than one conformation that is similar in overall binding energy. In those cases, the deformation energy of binding may be taken to be the difference between the energy of the free compound and the average energy of the conformations observed when the inhibiting compound binds to the enzyme.

A compound designed or selected as interacting with a selected transcription factor, e.g., a MarA family polypeptide, e.g., MarA, Rob, or SoxS may be further computationally optimized so that in its bound state it would preferably lack repulsive electrostatic interaction with the target protein. Such non-complementary (e.g., electrostatic) interactions include repulsive charge-charge, dipole-dipole and charge-dipole interactions. Specifically, the sum of all electrostatic interactions between the modulating compound and the enzyme when the modulating compound is bound to the selected transcription factor, preferably make a neutral or favorable contribution to the enthalpy of binding.

Specific computer software is available in the art to evaluate compound deformation energy and electrostatic interaction. Examples of programs designed for such uses include: Gaussian 92, revision C [M. J. Frisch, Gaussian, Inc., Pittsburgh, Pa. ©1992]; AMBER, version 4.0 [P. A. Kollman, University of California at San Francisco, ©1994]; QUANTA/CHARMM [Molecular Simulations, Inc., Burlington, Mass. © 1994] ; and Insight II/Discover (Biosysm Technologies Inc., San Diego, Calif. ©1994). These programs may be implemented, for instance, using a Silicon Graphics workstation, IRIS 4D/35 or IBM RISC/6000 workstation model 550. Other hardware systems and software packages will be known to those skilled in the art.

Once a transcription factor modulating compound has been optimally selected or designed, as described above, substitutions may then be made in some of its atoms or side groups in order to improve or modify its binding properties. Initial substitutions are preferable conservative, i.e., the replacement group will have approximately the same size, shape, hydrophobicity and charge as the original group. Substitutions known in the art to alter conformation should be avoided. Such substituted chemical compounds may then be analyzed for efficiency of fit to the selected transcription factor by the same computer methods described above.

Computer programs can be used to identify unoccupied (aqueous) space between the van der Waals surface of a compound and the surface defined by residues in the binding site. These gaps in atom-atom contact represent volume that could be occupied by new functional groups on a modified version of the lead compound. More efficient use of the unoccupied space in the binding site could lead to a stronger binding compound if the overall energy of such a change is favorable. A region of the binding pocket which has unoccupied volume large enough to accommodate the volume of a group equal to or larger than a covalently bonded carbon atom can be identified as a promising position for functional group substitution. Functional group substitution at this region can constitute substituting something other than a carbon atom, such as oxygen. If the volume is large enough to accommodate a group larger than a carbon atom, a different functional group which would have a high likelihood of interacting with protein residues in this region may be chosen. Features which contribute to interaction with protein residues and identification of promising substitutions include hydrophobicity, size, rigidity and polarity. The combination of docking, Kᵢ estimation, and visual representation of sterically allowed room for improvement permits prediction of potent derivatives.

Once a transcription factor modulating compound has been selected or designed, computational methods to assess its overall likeness or similarity to other molecules can be used to search for additional compounds with similar biochemical behavior. In such a way, for instance, HTS derived hits can be tested to assure that they are bona fide ligands against a particular active site, and to eliminate the possibility that a particular hit is an artifact of the screening process. There are currently several methods and approaches to determine a particular compound's similarity to members of a virtual database of compounds. One example is the OPTISIM methodology that is distributed in the Tripos package, SYBYL (© 1991-2002 Tripos, Inc., St. Louis, MO). OPTISIM exploits the fact that each 3-dimensional representation of a molecule can be broken down into a set of 2-dimensional fragments and encoded into a pre-defined binary string. The result is that each compound within a particular set is represented by a unique numerical code or fingerprint that is amenable to mathematical manipulations such as sorting and comparison. OPTISIM is automated to calculate and report the percent difference in the fingerprints of the respective compounds for instance according to the using a formalism known as the Tanimoto coefficient. For instance, a compound that is similar in structure to another will share a high coefficient. Large virtual databases of commercially available compounds or of hypothetical compounds can be quickly screened to identify compounds with high Tanimoto coefficient.

Once a series of similar transcription factor modulating compounds has been identified and expanded by the methods described, their experimentally determined biological activities can be correlated with their structural features using a number of available statistical packages. In a typical project within the industry, the CoMFA (COmparative Molecular Field Analysis) and QSAR (Quantitative Structure Activity Relationship) packages within the SYBYL suite of programs (Tripos Associates, St. Louis, MO) are utilized. In CoMFA, a particular series of compounds with measured activities are co-aligned in a manner that is believed to emulate their arrangement as they interact with the active site. A 3-dimensional lattice, or grid is then constructed to encompass the collection of the so-aligned compounds. At each point on the lattice, an evaluation of the potential energy is determined and tabulated-typically potentials that simulate the electronic and steric fields are determined, but other potential functions are available. Using the statistical methods such as PLS (Partial Least Squares), correlation between the measured activities and the potential energy values at the grid-points can be determined and summed in a linear equation to produce the overall molecular correlation or QSAR model. A particularly useful feature in CoMFA is that the individual contribution for each grid-point is known; the importance of the grid points upon the overall correlation can be visualized graphically in what is referred to as a CoMFA field. When this field is combined with the original compound alignment, it becomes a powerful tool to rationalize the activities of the individual compounds from whence the model was derived, and to predict how chemical modification of a reference compound would be effected. As an example, a QSAR model was developed for a set of 92 benzodiazepines using the method described above.

Structure based drug design as described herein or known in the art can be used to identify candidate compounds or to optimize compounds identified in screening assays described herein.

The invention pertains, per se, to not only the methods for identifying the transcription factor modulating compounds, but to the compounds identified by the methods of the invention as well as methods for using the identified compounds.

### IV. Methods for Identifying Molecules That Contribute to Virulence in Microbes

In another aspect, the invention pertains to a method of determining whether a molecule, e.g., a transcription factor or a molecule whose expression is regulated by a transcription factor is a virulence factor by creating a microbe in which the transcription factor is misexpressed and introducing the microbe into a mammal, e.g., a non-human animal or a human subject (Bieber, D. et al. 1998 Science 280:2114). In one embodiment, the molecule is a transcription factor. In one embodiment, the transcription factor comprises an HTH domain. In another embodiment, the transcription factor is an AraC family member. In another embodiment, the transcription factor is a Mar A family member.

Molecules for testing can be misexpressed using standard methods known in the art. Misexpression can arise when the molecule is expressed in a form that is non-functional or when the molecule is not expressed at all by a cell. For example, in one embodiment, one or more mutations can be introduced into a gene to be tested or into a regulatory region controlling expression of the molecule. Current methods in widespread use for creating mutant proteins in a library format are error-prone polymerase chain and cassette mutagenesis, in which the specific region to be mutagenized is replaced with a synthetically mutagenized oligonucleotide.

In another embodiment, a gene can be deleted. Genetic alteration in the form of disruption or deletion can be accomplished by several means known to those skilled in the art, including homologous recombination using an antibiotic resistance marker. These methods involve disruption of a gene using restriction endonucleases such that part or all of the gene is disrupted or eliminated or such that the normal transcription and translation are interrupted, and an antibiotic resistance marker for phenotypic screening. In a preferred embodiment, in frame deletions of a specific transcription factor can be constructed using crossover PCR and allelic exchange.

Molecules identified as being important in microbial virulence in this type of assay can then be used to identify modulators of the expression and/or activity of the molecule, using methods e.g., as described herein.

In one embodiment, a test compound identified in a primary screen (e.g., in a cell-free or whole cell assay or using drug design techniques can be tested in a secondary screening assay, e.g., in an animal model.

In one embodiment, an animal model of infection is used in which the ability of the microbe to establish an infection in the non-human animal requires that the microbe colonize the animal. The microbe is then tested in the animal model for its ability to infect the animal. The lack of infection means that the animal was not colonized by the microbe and indicates that the gene is involved in the virulence process.

For example, non-human animal models which test for the ability of a microbe to colonize a host are known in the art. Although models which do not strictly require colonization (e.g., models in which non-human animals are injected with microbes and the LD50 or time to death is measured) can be used in the instant methods, such methods are not preferred. Preferred models require that the microbe be capable of colonizing a host in order to grow in the host and cause pathogenesis (Alksne, L.E. and Projan, S.J.,. 2000 Current Opinion in Biotechnology 11:625-636)

Exemplary models include models in which bacteria (e.g., a virulent strain of *E*. *coli*) are injected into the intestines of rodents or rabbits and the ability of the bacteria to cause pathology in the gut in the presence and absence of a candidate virulence factor or in the presence and absence of a test compound is measured.

In another embodiment, the ability of a strain of *Neisseria* to colonize the genitourinary tract can be measured in the presence and absence of a candidate virulence factor or in the presence and absence of a test compound.

In still another embodiment, the ability of *H. pylori* to colonize the gut can be measured in the presence and absence of a candidate virulence factor or in the presence and absence of a test compound.

In yet another embodiment, the ability of an organism, e.g., *P. aeriginosa,* to cause infection in a non-human animal burn model or a thigh wound model can be measured in the presence and absence of a candidate virulence factor or in the presence and absence of a test compound. Models which involve traumatization of the cornea can also be used.

In yet another embodiment, an in vitro model can be used to test the virulence of a microbe, e.g., by testing for the ability of a microbe to adhere to epithelial cell monolayers and elicit an inflammatory response (Tang et al. 1996. Infection and Immunity. 64:37).

In yet another embodiment, non-human animals can be coinfected with more than one strain of bacteria (see e.g., Rippere-Lampe et al. 2001. Infection and Immunity 69:3954).

In another embodiment, a non-human model of infection with *Yersinia,* (e.g., *Y. pestis* or models of *Y. pestis,* e.g., *Y. enterocolitica* or *Y. pseudotuberculosis*) can be used. In an exemplary animal model, *Y. enterocolitica* or *Y. pseudotuberculosis* can be administered orally or via intrapentoneal inoculation. Following oral ingestion, the bacteria localize to the distal ileum and proximal colon and then invade the M cells of the Peyer's patches and colonize the underlying lymph tissues. The bacteria then spread to the mesenteric lymph nodes and, eventually, to the spleen and the liver. The number of bacteria in tissues (e.g., the cecum, Peyer's patches, mesenteric lymph nodes, and spleens) can be determined (Mecsas et al. 2001. Infection and Immunity. 67:2779; Monack et al. 1998. J.Exp.Med. 188:2127).

For example, in order to evaluate the virulence *in vivo* of *Y. pseudotuberculosis* lacking LcrF (VirF), a single null mutation in *lcrF* (*virF*) will be created in strain YPIIIpIBI using previously described genetic techniques. The wild type and mutant strains will be used to infect mice as described below.

Briefly, 8- to 10-week-old BALB/c female mice can be infected orally with serial 10-fold dilutions of wild type or mutant *Y. pseudotuberculosis.* The infected mice will be monitored for a period of 30 days and the point of 50% lethality (LD₅₀) will be calculated as described previously.

Once the LD₅₀ is determined, a sub-lethal dose of both wild type and mutant *Y. pseudotuberculosis* can be used to infect mice. Five days post-infection, the mice will be sacrificed and tissues, including small intestine lumen, cecal lumen, large intestine lumen, Peyer's patches, mesenteric lymph nodes, spleen, liver, lungs, and kidneys, and blood will be examined for bacterial load according to an established protocol. The experiments will allow comparison of the infectivity of the two strains and identify more subtle changes in virulence, parameters that will be important for subsequent experiments.

In yet another exemplary animal model, *Y. pestis* can be administered subcutaneously in a murine host and the dose necessary to kill 50% of a mouse population [lethal dose 50 (LD50)] can be determined (Rossi et al. 2001. Infection and Immunity. 69:6707; Thompson et al. 1999. Infection and Immunity. 67:38779).

In still another embodiment, a non-human animal model of prostatitis can be used. Rat models of prostatitis are known in the art (see e.g., Rippere-Lampe. 2001. Infection and Immunity 69:6515). Animals can be infected with and organism (e.g., uropathogenic *Escherichia coli* via a transurethral catheter or intravesicular inoculation. Prostate glands can be removed and the number of organisms determined (e.g., by homogenizing the tissues, serially diluting them, and plating them for colony counts).

In yet another embodiment, a non-human animal model of urinary tract infection (an ascending pyelonephritis model) can be used. Such models have been previously described and can be found in the literature. For a review see Mulvy et al. ((2000) Proc. Natl. Acad. Sci. U.S.A. 97:8829-35) or Schilling, et al. ((2001) Urology 57:56-61. Specific examples can be found in Hagberg et al. ((1983) Infection and Immunity 40:273-283), Johnson et al. ((1993) Molec. Micro. 10:143-155), Mobley et al. ((1990) Infection and Immunity 58:1281-1289), and Rippere-Lampe et al. ((2001) Infection and Immunity 69:3954-64). The use of such a model is described in the instant examples.

The number of bacteria present in the non-human animal can be directly quantitated, e.g., by harvesting the affected organ and determining the level of bacterial contamination using standard techniques. In another embodiment, the growth of the microbe in the host can be determined indirectly, e.g., by quantitating pathogenic lesions in the organ(s) of a host or by measuring the level of the host's immune response to the microbe.

It will be recognized by one of ordinary skill in the art that any of these models, as well as others described herein or known in the art, can also be used to identify compounds that modulate transcription factor activity.

### V. Transcription Factor Modulating Compounds and Test Compounds

Compounds for testing in the instant methods can be derived from a variety of different sources and can be known (although not previously known to modulate the activity and/or expression of transcription factors) or can be novel. In one embodiment, libraries of compounds are tested in the instant methods to identify transcriptional activation factor modulating compounds, e.g., HTH protein modulating compounds, AraC family polypeptide modulating compounds, MarA family polypeptide modulating compounds, etc. In another embodiment, known compounds are tested in the instant methods to identify transcription factor modulating compounds (such as, for example, HTH protein modulating compounds, AraC family polypeptide modulating compounds, MarA family polypeptide modulating compounds, etc.). In an embodiment, compounds among the list of compounds generally regarded as safe (GRAS) by the Environmental Protection Agency are tested in the instant methods. In another embodiment, the transcription factors which are modulated by the modulating compounds are transcription factors of prokaryotic microbes.

In one embodiment, a plurality of test compounds are tested using the disclosed methods. In another embodiment, the compounds tested in the subject screening assays were not previously known to modulate transcription factor activity.

A recent trend in medicinal chemistry includes the production of mixtures of compounds, referred to as libraries. While the use of libraries of peptides is well established in the art, new techniques have been developed which have allowed the production of mixtures of other compounds, such as benzodiazepines (Bunin et al. 1992. J. Am. Chem. Soc. 114:10987; DeWitt et al. 1993. Proc. Natl. Acad. Sci. USA 90:6909) peptoids (Zuckermann. 1994. J. Med. Chem. 37:2678) oligocarbamates (Cho et al. 1993. Science. 261:1303), and hydantoins (DeWitt et al. supra). Rebek et al. have described an approach for the synthesis of molecular libraries of small organic molecules with a diversity of 104-105 (Carell et al. 1994. Angew. Chem. Int. Ed. Engl. 33:2059; Carell et al. Angew. Chem. Int. Ed. Engl. 1994. 33:2061).

The compounds of the present invention can be obtained using any of the numerous approaches in combinatorial library methods known in the art, including: biological libraries; spatially addressable parallel solid phase or solution phase libraries, synthetic library methods requiring deconvolution, the 'one-bead one-compound' library method, and synthetic library methods using affinity chromatography selection. The biological library approach is limited to peptide libraries, while the other four approaches are applicable to peptide, non-peptide oligomer or small molecule libraries of compounds (Lam, K.S. Anticancer Drug Des. 1997. 12:145).

Exemplary compounds which can be screened for activity include, but are not limited to, peptides, nucleic acids, carbohydrates, small organic molecules, and natural product extract libraries. In one embodiment, the test compound is a peptide or peptidomimetic. In another, preferred embodiment, the compounds are small, organic non-peptidic compounds.

Other exemplary methods for the synthesis of molecular libraries can be found in the art, for example in: Erb et al. 1994. Proc. Natl. Acad. Sci. USA 91:11422; Horwell et al. 1996 Immunopharmacology 33:68; and in Gallop et al. 1994. J. Med. Chem. 37:1233.

Libraries of compounds may be presented in solution (*e.g.,* Houghten (1992) Biotechniques 13:412-421), or on beads (Lam (1991) Nature 354:82-84), chips (Fodor (1993) Nature 364:555-556), bacteria (Ladner USP 5,223,409), spores (Ladner USP '409), plasmids (Cull et al. (1992) Proc Natl Acad Sci USA 89:1865-1869) or on phage (Scott and Smith (1990) Science 249:386-390); (Devlin (1990) Science 249:404-406); (Cwirla et al. (1990) Proc. Natl. Acad. Sci. 87:6378-6382); (Felici (1991) J. Mol. Biol. 222:301-310); (Ladner *supra.*)*.* Other types of peptide libraries may also be expressed, see, for example, U.S. Patents 5,270,181 and 5,292,646). In still another embodiment, combinatorial polypeptides can be produced from a cDNA library.

In other embodiments, the compounds can be nucleic acid molecules. In preferred embodiments, nucleic acid molecules for testing are small oligonucleotides. Such oligonucleotides can be randomly generated libraries of oligonucleotides or can be specifically designed to reduce the activity of a transcription factor, e.g., a HTH protein, a MarA family polypeptide, or an AraC family polypeptide. For example, in one embodiment, these oligonucleotides are sense or antisense oligonucleotides. In one embodiment, oligonucleotides for testing are sense to the binding site of a particular transcription factor, e.g., a MarA family polypeptide helix-turn-helix domain. Methods of designing such oligonucleotides given the sequences of a particular transcription factor polypeptide, such as a MarA family polypeptide, is within the skill of the art.
In yet another embodiment, computer programs can be used to identify individual compounds or classes of compounds with an increased likelihood of modulating a transcription factor activity, e.g., an HTH protein, a AraC family polypeptide, or a MarA family polypeptide activity. Such programs can screen for compounds with the proper molecular and chemical complementarities with a chosen transcription factor. In this manner, the efficiency of screening for transcription factor modulating compounds in the assays described above can be enhanced.

### VI. Microbes Suitable For Testing in Assays and/or Treating with the Identified Compounds

Numerous different microbes are suitable for testing in the instant assays (e.g., as sources of transcription factors for testing) or infections with these microbes can be treated with the compounds identified using the assays described herein. For use in assays they may be used as intact cells or as sources of material, *e*.g., nucleic acid molecules or polypeptides as described herein.

In one embodiment, the cells comprise a transcription factor, e.g., an AraC/XylS or a MarA family transcription factor.

In one embodiment, microbes for use in the claimed methods constitutively express a transcription factor.

In preferred embodiments, microbes for use in the claimed methods are bacteria, either Gram negative or Gram positive bacteria. More specifically, any bacteria that are shown to become resistant to antibiotics , e.g., to display a Mar phenotype are preferred for use in the claimed methods, or that are infectious or potentially infectious.

Examples of microbes suitable for testing or treating include, but are not limited to, *Pseudomonas aeruginosa, Pseudomonas fluorescens, Pseudomonas acidovorans, Pseudomonas alcaligenes, Pseudomonas putida, Stenotrophomonas maltophilia, Burkholderia cepacia, Aeromonas hydrophilia, Escherichia coli, Citrobacter freundii, Salmonella enterica* Typhimurium, *Salmonella enterica* Typhi, *Salmonella enterica* Paratyphi, *Salmonella enterica* Enteridtidis, *Shigella dysenteriae, Shigella flexneri, Shigella sonnei, Enterobacter cloacae, Enterobacter aerogenes, Klebsiella pneumoniae, Klebsiella oxytoca, Serratia marcescens, Francisella tularensis, Morgarzella morganii, Proteus mirabilis, Proteus vulgaris, Providencia alcalifaciens, Providencia rettgeri, Providencia stuartii, Acinetobacter calcoaceticus, Acinetobacter haemolyticus, Yersinia enterocolitica, Yersinia pestis, Yersinia pseudotuberculosis, Yersinia intermedia, Bordetella pertussis, Bordetella parapertussis, Bordetella bronchiseptica, Haemophilus influenza, Haemophilus parainfluenzae, Haemophilus haemolyticus, Haemophilus parahaemolyticus, Haemophilus ducreyi, Pasteurella multocida, Pasteurella haemolytica, Branhamella catarrhalis, Helicobacter pylori, Campylobacter fetus, Campylobacter jejuni, Campylobacter coli, Borrelia burgdorferi, Tribrio cholerae, Vibrio parahaemolyticus, Legionella pneumophila, Listeria monocytogenes, Neisseria gonorrhoeae, Neisseria meningitidis, Gardnerella vaginalis, Bacteroides fragilis, Bacteroides distasonis, Bacteroides* 3452A homology group, *Bacteroides vulgatus, Bacteroides ovalus, Bacteroides thetaiotaomicron, Bacteroides uniformis, Bacteroides eggerthii, Bacteroides splanchnicus, Clostridium difficile, Mycobacterium tuberculosis, Mycobacterium avium, Mycobacterium intracellulare, Mycobacterium leprae, Corynebacterium diphtheria, Corynebacterium ulcerans, Streptococcus pneumoniae, Streptococcus agalactiae, Streptococcus pyogenes, Enterococcus faecalis, Enterococcus faecium, Staphylococcus aureus, Staphylococcus epidermidis, Staphylococcus saprophyticus, Staphylococcus intermedius, Staphylococcus hyicus subsp. hyicus, Staphylococcus haemolyticus, Staphylococcus hominis,* and *Staphylococcus saccharolyticus.*

In one embodiment, microbes suitable for testing or treating are bacteria from the family *Enterobacteriaceae.* In preferred embodiments, the compound is effective against a bacteria of a genus selected from the group consisting of: *Escherichia, Proteus, Salmonella, Klebsiella, Providencia, Enterobacter, Burkholderia, Pseudomonas, Aeromonas, Haemophilus, Yersinia, Neisseria,* and *Mycobacteria.*

In yet other embodiments, the microbes to be tested are Gram positive bacteria and are from a genus selected from the group consisting of: *Lactobacillus, Azorhizobium, Streptomyces, Pediococcus, Photobacterium, Bacillus, Enterococcus, Staphylococcus, Clostridium,* and *Streptococcus.*

In other embodiments, the microbes to be tested or treated are fungi. In a preferred embodiment the fungus is from the genus *Mucor* or *Candida, e.g., Mucor racmeosus* or *Candida albicans.*

In yet other embodiments, the microbes to be tested or treated are protozoa. In a preferred embodiment the microbe is a malaria or cryptosporidium parasite.

In another embodiment, the microbe to be tested is of concern as a potential bioterrorism agent. For example, in one embodiment, one or more of the microbes selected from the group consisting of *Bacillus anthracis* (anthrax); *Clostridium botulinum; Yersinia pestis*;; *Francisella tularensis* (tularemia); *Burkholderia pseudomallei*; *Coxiella burnetti* (Q fever); *Brucella species* (brucellosis); *Burkholderia mallei* (glanders);; Epsilon toxin of *Clostridium perfringens;* Staphylococcus enterotoxin B; Typhus fever (*Rickettsia prowazekii*)*;* Diarrheagenic *E. coli;* Pathogenic Vibrios (e.g., V. parahaemolyticus, V. vulnificus, V. mimicus, V. hollisae, V. fluvialis, V. alginolyticus, V. metschnikovii, and V. damsela; Shigella spp. -; Salmonella spp.; *Listeria monocytogenes; Campylobacter jejuni; Yersinia enterocolitica*)*;* Multi-drug resistant TB; ;Other Rickettsias (e.g., *R. rickettsii, R. conorii, R. tsutsugamushi, R. typhi,* and *R. akari);* and is tested in the subject assays or is treated using a compound of the invention.

In another embodiment, an organism is potentially important as an agent in bioterrorism which has a Mar-like system is tested in the subject assays or is treated using a compound of the invention. Exemplary organisms include: *E. coli* (enteropathogenic *E.coli* (EPEC), enterotoxigenic *E. coli* (ETEC), enterohemorrhagic (EHEC), enteroaggregative (EAEC), Shiga toxin producing *E*. *coli* (STEC)), *Salmonella enterica* serovar Choleraesuis, *Salmonella enterica* serovar Enteritidis, *Salmonella enterica* serovar Typhimurium, *Salmonella enterica* serovar Typhimurium DT104, *Yersinia* spp. *(Y. pestis, Y. enterocolitica, Y. pseudotuberculosis)* Shigella spp. (*S. flexneri, S. sonnei, S. dysenteriae) Vibrio cholerae,* and *Bacillus* spp.

### VII. Pharmaceutical Compositions

The agents which modulate the activity or expression of transcription factors can be administered to a subject using pharmaceutical compositions suitable for such administration. Such compositions typically comprise the agent (e.g., nucleic acid molecule, protein, or antibody) and a pharmaceutically acceptable carrier.

In one embodiment, such compositions can be administered in combination with a second agent. For example, an agent that modulates the activity or expression of a transcription factor can be administered to a subject along with a second agent that is effective at controlling the growth or virulence of a microbe. Exemplary agents include antibiotics or biocides. Such a second agent can be administered or contacted with a microbe or a surface either separately or as part of the pharmaceutical composition comprising the agent which modulates the activity or expression of the transcription factor.

As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition used in the therapeutic methods of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment oftonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi: The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the agent that modulates the expression and/or activity of a transcription in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer. Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The agents that modulate the activity of transcription factors can also be prepared in the form of suppositories (e.g., with conventional suppository bases such as cocoa butter and other glycerides) or retention enemas for rectal delivery.

In one embodiment, the agents that modulate transcription factor expression and/or activity are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the agent that modulates the expression and/or activity of a transcription factor and the particular therapeutic effect to be achieved, and the limitations inherent in the art of compounding such an agent for the treatment of subjects.

Toxicity and therapeutic efficacy of such agents can be determined by standard pharmaceutical procedures in cell cultures or experimental animals, e.g., for determining the LD50 (the dose lethal to 50% of the population) and the ED50 (the dose therapeutically effective in 50% of the population).

Preliminary *in vitro* cytotoxicity (Tox) assays of all newly synthesized Mar inhibitors will be performed on African green monkey kidney COS-1 and Chinese hamster ovary (CHO-K1) cell lines according to standard methods and in a relatively high-throughput manner using automatic liquid dispensers and robotic instrumentation. Briefly, cell cultures are washed, trypsinized, and harvested. The cell suspensions are then prepared, used to seed 96-well black-walled microtiter plates, and incubated under tissue culture conditions overnight at 37°C. On the following day, serial dilutions of a Mar inhibitor are transferred to the plates that are then incubated for a period of 24 hr. Subsequently, the media/drug is aspirated and 50 *µ*l of Resazurin is added. Resazurin is a soluble non-toxic dye that is used as an indicator of cellular metabolism and is routinely employed for these types of cytotoxicity assays.

Plates are then incubated under tissue culture conditions for 2 hr and then in the dark for an additional 30 min. Fluorescence measurements (excitation 535 nm, emission 590 nm) are recorded and are used to calculate toxicity versus control cells. Ultimately, Tox₅₀ and Tox₁₀₀ values will be determined and these values represent the concentration of compound necessary to inhibit cellular proliferation by 50% and 100%, respectively. Control cytotoxic and non-cytotoxic compounds are routinely included in all assays. The goal of these experiments is to identify compounds with little or no measurable *in vitro* cytotoxicity, defined as compounds with Tox₅₀ and Tox₁₀₀ values ≥:50-100 *µ*g/ml.

Mar inhibitors that perform favorably in the cellular Tox assays will be studied in a mouse model of acute toxicity. Briefly, groups of female CD1 mice (n=5) will be treated with the test compound or a control compound (vehicle) via a subcutaneous route of administration at up to three dose levels for five days. Overt signs of animal distress, e.g., general clinical observations, weight loss, feed consumption, etc., will be monitored daily. Animals will be euthanized, via CO₂/O₂ asphyxiation, upon completion of the study and hematological and pathological tissue analyses and serum chemistries can be performed. The goal will be to identify compounds without detectable (≥15-25 mg/kg) acute toxicity.

The dose ratio between toxic and therapeutic effects is the therapeutic index and can be expressed as the ratio LD50/ED50. Agents which exhibit large therapeutic indices are preferred. While agents that exhibit toxic side effects may be used, care should be taken to design a delivery system that targets such agents to the site of affected tissue in order to minimize potential damage to uninfected cells and, thereby, reduce side effects.

The data obtained from the cell culture assays and animal studies can be used in formulating a range of dosage for use in humans. The dosage of such transcription factor modulating agents lies preferably within a range of circulating concentrations that include the ED50 with little or no toxicity. The dosage may vary within this range depending upon the dosage form employed and the route of administration utilized. For any agent used in the therapeutic methods of the invention, the therapeutically effective dose can be estimated initially from cell culture assays. A dose may be formulated in animal models to achieve a circulating plasma concentration range that includes the IC50 (*i.e.,* the concentration of the test compound which achieves a half-maximal inhibition of symptoms) as determined in cell culture. Such information can be used to more accurately determine useful doses in humans. Levels in plasma may be measured, for example, by high performance liquid chromatography.

As defined herein, a therapeutically effective amount of protein or polypeptide (i.e., an effective dosage) ranges from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The skilled artisan will appreciate that certain factors may influence the dosage required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody can include a single treatment or, preferably, can include a series of treatments. It will also be appreciated that the effective dosage of antibody, protein, or polypeptide used for treatment may increase or decrease over the course of a particular treatment. Changes in dosage may result and become apparent from the results of diagnostic assays as described herein.

The present invention encompasses agents which modulate expression and/or activity. An agent may, for example, be a small molecule. For example, such small molecules include, but are not limited to, peptides, peptidomimetics, amino acids, amino acid analogs, polynucleotides, polynucleotide analogs, nucleotides, nucleotide analogs, organic or inorganic compounds (*i.e.,* including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds. It is understood that appropriate doses of small molecule agents depends upon a number of factors within the ken of the ordinarily skilled physician, veterinarian, or researcher. The dose(s) of the small molecule will vary, for example, depending upon the identity, size, and condition of the subject or sample being treated, further depending upon the route by which the composition is to be administered, if applicable, and the effect which the practitioner desires the small molecule to have upon the nucleic acid or polypeptide of the invention.

Exemplary doses include milligram or microgram amounts of the small molecule per kilogram of subject or sample weight (e.g., about 1 microgram per kilogram to about 500 milligrams per kilogram, about 100 micrograms per kilogram to about 5 milligrams per kilogram, or about 1 microgram per kilogram to about 50 micrograms per kilogram). It is furthermore understood that appropriate doses of a small molecule depend upon the potency of the small molecule with respect to the expression and/or activity to be modulated. Such appropriate doses may be determined using the assays described herein. When one or more of these small molecules is to be administered to an animal (e.g., a human) in order to modulate expression and/or activity of a polypeptide or nucleic acid of the invention, a physician, veterinarian, or researcher may, for example, prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet of the subject, the time of administration, the route of administration, the rate of excretion, any drug combination, and the degree of expression and/or activity to be modulated.

### VIII. Methods of Treatment

The present invention provides for both prophylactic and therapeutic methods of treating a subject, *e.g.,* a human, at risk of (or susceptible to) or having a microbial infection by administering an agent which modulates the expression and/or activity of a transcription factor. The term "treatment", as used herein, is defined as the application or administration of a therapeutic agent to a patient, who has an infection, a symptom of an infection, or a predisposition toward an infection, with the purpose to cure, heal, alleviate, relieve, alter, remedy, ameliorate, improve or affect the infection, the symptoms of the infection, or the predisposition toward an infection, e.g., a bacterial infection.

In one embodiment, the invention provides for a method of treatment, either prophylactic or therapeutic of a subject or a patient population at risk of infection, e.g., individuals in long term care facilities, critical and intensive care units, transplant (kidney) services, post-surgical (urologic) or oncology units, sexually active young females, or postmenopausal women that experience recurrent UTI. In addition, the subject methods and compounds can be used in the prophylactic treatment of asymptomatic bacteriuria in pregnant women and patients undergoing urologic surgery or renal transplants. Immunocompromised or catheterized patients could also be treated using the subject methods and compounds.

In one embodiment, the compounds and methods of the invention can be used to treat genito-urinary tract infections (e.g., cystitis, uncomplicated UTI, acute uncomplicated pyelonephritis, complicated UTI, UTI in women, UTI in men, recurrent UTI, and asymptomatic bacteriuria).

In one embodiment, the invention provides for a method of treatment, either prophylactic or therapeutic treatment, of a subject or a patient population exposed to or at risk of exposure to an organism potentially important as an agent in bioterrorism by modulating the expression and/or activity of a transcription factor.

Exemplary therapeutic agents include, but are not limited to, small molecules, peptides, antibodies, ribozymes and antisense oligonucleotides.

In one aspect, the invention provides a method for preventing in a subject, a microbial infection by administering to the subject an agent which modulates the expression and/or activity of a transcription factor or a combination of such agents. Subjects at risk for an infection can be identified, for example, based on the status of the subject (e.g., determining that a subject is immunocompromised) or based on the environmental conditions to which the subject is exposed, (e.g., determining that there is a possibility that the subject may be exposed to a certain agent). Administration of a prophylactic agent can occur prior to the manifestation of symptoms characteristic of an infection, such that an infection is prevented or, alternatively, delayed in its progression. The appropriate agent can be determined, e.g., based on screening assays described herein.

Another aspect of the invention pertains to methods for treating a subject suffering from an existing microbial infection. These methods involve administering to a subject an agent which modulates (e.g., inhibits) the expression and/or activity of a transcription factor or a combination of such agents.

In one embodiment, a second agent may be administered in conjunction with a transcription factor modulating agent of the invention. For example, the second agent can be one which is used clinically for treatment of the microbe. For example, in one embodiment, an antibiotic is coadministered with a transcription factor modulating agent (e.g., is administered as part of the same treatment protocol) or is present on the same surface as the transcription factor modulating agent.

In one embodiment, such a combination therapy is administered to prevent recurring infections (e.g., recurring urinary tract infections) or biofilm-related infections. In another embodiment, such a combination therapy is administered to reduce the amount of antibiotic or eliminate the need for one or more antibiotics for prophylaxis or treatment. In another embodiment, such a combination treatment prevents resistance to the antibiotic from developing in the microbe.

In one embodiment, the invention pertains to a method for dispersing or preventing the formation of a biofilm on a surface (e.g., an abiotic, i.e., non-living surface, or in an area, by administering an effective amount of a transcription factor modulating compound, e.g., a HTH protein modulating compound, an AraC family polypeptide modulating compound, a MarA family polypeptide modulating compound, or a MarA inhibiting compound.

It has been discovered that the absence of MarA and its homologs has a negative effect on biofilm formation in *E. coli.* In order to confirm this finding genetically, plasmid encoded *marA* was transformed into an *E. coli* strain deleted of *marA, soxS,* and *rob* (triple knockout). The expression of MarA in this triple knockout restored biofilm formation in this host to a level that was comparable to that of the wild type host.

The term "biofilm" includes biological films that develop and persist at interfaces in aqueous and other environments. Biofilms are composed of microorganisms embedded in an organic gelatinous structure composed of one or more matrix polymers which are secreted by the resident microorganisms. The term "biofilm" also includes bacteria that are attached to a surface in sufficient numbers to be detected or communities of microorganisms attached to a surface (Costerton, J. W., et al. (1987) Ann. Rev. Microbiol. 41:435-464; Shapiro, J. A. (1988) Sci Am. 256:82-89; O'Toole, G. et al. (2000) Annu Rev Microbiol. 54:49-79).

In another embodiment, the invention pertains to methods of treating biofilm associated states in a subject, by administering to said subject an effective amount of a transcription factor modulating compound, e.g., a MarA family inhibiting compound, such that the biofilm associated state is treated.

The term "biofilm associated states" includes disorders which are characterized by the presence or potential presence of a bacterial biofilm. Many medically important pathogens form biofihns and biofilm formation is often one component of the infectious process (Costerton, J. W. et al. (1999) Science 284:1318-1322). Examples of biofilm associated states include, but are not limited to, middle ear infections, cystic fibrosis, osteomyelitis, acne, dental cavities, and prostatitis. Biofilm associated states also include infection of the subject by one or more bacteria, *e.g., Pseudomonas aeruginosa.* One consequence of biofilm formation is that bacteria within biofilms are generally less susceptible to a range of different antibiotics relative to their planktonic counterparts.

Furthermore, the invention also pertains to methods for preventing the formation of biofilms on surfaces or in areas, by contacting the area with an effective amount of a transcription factor modulating compound, e.g., a MarA family inhibiting compound, etc.

Industrial facilities employ many methods of preventing biofouling of industrial water systems. Many microbial organisms are involved in biofilm formation in industrial waters. Growth of slime-producing bacteria in industrial water systems causes problems including decreased heat transfer, fouling and blockage of lines and valves, and corrosion or degradation of surfaces. Control of bacterial growth in the past has been accomplished with biocides. Many biocides and biocide formulations are known in the art. However, many of these contain components which may be environmentally deleterious or toxic, and are often resistant to breakdown.

The transcription factor inhibiting compounds, such as but not limited to AraC family inhibiting compounds and MarA family inhibiting compounds, of the present invention are useful in a variety of environments including industrial, clinical, the household, and personal care. The compositions of the invention may comprise one or more compounds of the invention as an active ingredient acting alone, additively, or synergistically against the target organism.

The compounds of the invention may be formulated in a composition suitable for use in environments including industry, pharmaceutics, household, and personal care. In an embodiment, the compounds of the invention are soluble in water. The modulating compounds may be applied or delivered with an acceptable carrier system. The composition may be applied or delivered with a suitable carrier system such that the active ingredient (e.g., transcription factor modulating compound of the invention such as a MarA family modulating compound, e.g., a MarA family polypeptide inhibiting compound) may be dispersed or dissolved in a stable manner so that the active ingredient, when it is administered directly or indirectly, is present in a form in which it is available in a advantageous way.

Also, the separate components of the compositions of the invention may be preblended or each component may be added separately to the same environment according to a predetermined dosage for the purpose of achieving the desired concentration level of the treatment components and so long as the components eventually come into intimate admixture with each other. Further, the present invention may be administered or delivered on a continuous or intermittent basis.

A transcription factor modulating compound when present in a composition will generally be present in an amount from about 0.000001% to about 100%, more preferably from about 0.001% to about 50%, and most preferably from about 0.01% to about 25%.

For compositions of the present invention comprising a carrier, the composition comprises, for example, from about 1% to about 99%, preferably from about 50% to about 99%, and most preferably from about 75% to about 99% by weight of at least one carrier.

The transcription factor modulating compound of the invention may be formulated with any suitable carrier and prepared for delivery in forms, such as, solutions, microemulsions, suspensions or aerosols. Generation of the aerosol or any other means of delivery of the present invention may be accomplished by any of the methods known in the art. For example, in the case of aerosol delivery, the compound is supplied in a finely divided form along with any suitable carrier with a propellant. Liquefied propellants are typically gases at ambient conditions and are condensed under pressure. The propellant may be any acceptable and known in the art including propane and butane, or other lower alkanes, such as those of up to 5 carbons. The composition is held within a container with an appropriate propellant and valve, and maintained at elevated pressure until released by action of the valve.

The compositions of the invention may be prepared in a conventional form suitable for, but not limited to topical or local application such as an ointment, paste, gel, spray and liquid, by including stabilizers, penetrants and the carrier or diluent with the compound according to a known technique in the art. These preparations may be prepared in a conventional form suitable for enteral, parenteral, topical or inhalational applications.

The present invention may be used in compositions suitable for household use. For example, compounds of the present invention are also useful as active antimicrobial ingredients in household products such as cleansers, detergents, disinfectants, dishwashing liquids, soaps and detergents. In an embodiment, the transcription factor modulating compound of the present invention may be delivered in an amount and form effective for the prevention, removal or termination of microbes.

The compositions of the invention for household use comprise, for example, at least one transcription factor modulating compound of the invention and at least one suitable carrier. For example, the composition may comprise from about 0.00001% to about 50%, preferably from about 0.0001% to about 25%, most preferably from about 0.0005% to about 10% by weight of the modulating compound based on the weight percentage of the total composition.

The transcription factor modulating compound of the present invention may also be used in hygiene compositions for personal care. For instance, compounds of the invention can be used as an active ingredient in personal care products such as facial cleansers, astringents, body wash, shampoos, conditioners, cosmetics and other hygiene products. The hygiene composition may comprise any carrier or vehicle known in the art to obtain the desired form (such as solid, liquid, semisolid or aerosol) as long as the effects of the compound of the present invention are not impaired. Methods of preparation of hygiene compositions are not described herein in detail, but are known in the art. For its discussion of such methods, The CTFA Cosmetic Ingredient Handbook, Second Edition, 1992, and pages 5-484 of A Formulary of Cosmetic Preparations (Vol. 2, Chapters 7-16) are incorporated herein by reference.
The hygiene composition for use in personal care comprise generally at least one modulating compound of the present application and at least one suitable carrier. For example, the composition may comprise from about 0.00001% to about 50%, preferably from about 0.0001% to about 25%, more preferably from about 0.0005% to about 10% by weight of the transcription factor modulating compound of the invention based on the weight percentage of the total composition.

The transcription factor modulating compound of the present invention may be used in industry. In the industrial setting, the presence of microbes can be problematic, as microbes are often responsible for industrial contamination and biofouling.

Compositions of the invention for industrial applications may comprise an effective amount of the compound of the present invention in a composition for industrial use with at least one acceptable carrier or vehicle known in the art to be useful in the treatment of such systems. Such carriers or vehicles may include diluents, deflocculating agents, penetrants, spreading agents, surfactants, suspending agents, wetting agents, stabilizing agents, compatibility agents, sticking agents, waxes, oils, co-solvents, coupling agents, foams, antifoaming agents, natural or synthetic polymers, elastomers and synergists. Methods of preparation, delivery systems and carriers for such compositions are not described here in detail, but are known in the art. For its discussion of such methods, U.S. Patent No. 5,939,086 is herein incorporated by reference. Furthermore, the preferred amount of the composition to be used may vary according to the active ingredient(s) and situation in which the composition is being applied.

The transcription factor modulating compounds of the present invention may be useful in nonaqueous environments. Such nonaqueous environments may include, but are not limited to, terrestrial environments, dry surfaces or semi-dry surfaces in which the compound or composition is applied in a manner and amount suitable for the situation.

The transcription factor modulating compounds of the present invention may be used to form contact-killing coatings or layers on a variety of substrates including personal care products (such as toothbrushes, contact lens cases and dental equipment), healthcare products, household products, food preparation surfaces and packaging, and laboratory and scientific equipment. Further, other substrates include medical devices such as catheters, urological devices, blood collection and transfer devices, tracheotomy devices, intraocular lenses, wound dressings, sutures, surgical staples, membranes, shunts, gloves, tissue patches, prosthetic devices (e.g., heart valves) and wound drainage tubes. Still further, other substrates include textile products such as carpets and fabrics, paints and joint cement. A further use is as an antimicrobial soil fumigant.

The transcription factor modulating compounds of the invention may also be incorporated into polymers, such as polysaccharides (cellulose, cellulose derivatives, starch, pectins, alginate, chitin, guar, carrageenan), glycol polymers, polyesters, polyurethanes, polyacrylates, polyacrylonitrile, polyamides (e.g., nylons), polyolefins, polystyrenes, vinyl polymers, polypropylene, silks or biopolymers. The modulating compounds may be conjugated to any polymeric material such as those with the following specified functionality: 1) carboxy acid, 2) amino group, 3) hydroxyl group and/or 4) haloalkyl group.

The composition for treatment of nonaqueous environments may be comprise at least one transcription factor modulating compound of the present application and at least one suitable carrier. In an embodiment, the composition comprises from about 0.001% to about 75%, advantageously from about 0.01% to about 50%, and preferably from about 0.1% to about 25% by weight of a transcription factor modulating compound of the invention based on the weight percentage of the total composition.

The transcription factor modulating compounds and compositions of the invention may also be useful in aqueous environments. "Aqueous environments" include any type of system containing water, including, but not limited to, natural bodies of water such as lakes or ponds; artificial, recreational bodies of water such as swimming pools and hot tubs; and drinking reservoirs such as wells. The compositions of the present invention may be useful in treating microbial growth in these aqueous environments and may be applied, for example, at or near the surface of water.

The compositions of the invention for treatment of aqueous environments may comprise at least one transcription factor modulating compound of the present invention and at least one suitable carrier. In an embodiment, the composition comprises from about 0.001% to about 50%, advantageously from about 0.003% to about 15%, preferably from about 0.01% to about 5% by weight of the compound of the invention based on the weight percentage of the total composition.

The present invention also provides a process for the production of an antibiofouling composition for industrial use. Such process comprises bringing at least one of any industrially acceptable carrier known in the art into intimate admixture with a transcription factor modulating compound of the present invention. The carrier may be any suitable carrier discussed above or known in the art.

The suitable antibiofouling compositions may be in any acceptable form for delivery of the composition to a site potentially having, or having at least one living microbe. The antibiofouling compositions may be delivered with at least one suitably selected carrier as hereinbefore discussed using standard formulations. The mode of delivery may be such as to have a binding inhibiting effective amount of the antibiofouling composition at a site potentially having, or having at least one living microbe. The antibiofouling compositions of the present invention are useful in treating microbial growth that contributes to biofouling, such as scum or slime formation, in these aqueous environments. Examples of industrial processes in which these compounds might be effective include cooling water systems, reverse osmosis membranes, pulp and paper systems, air washer systems and the food processing industry. The antibiofouling composition may be delivered in an amount and form effective for the prevention, removal or termination of microbes.

The antibiofouling composition of the present invention generally comprise at least one compound of the invention. The composition may comprise from about 0.001% to about 50%, more preferably from about 0.003% to about 15%, most preferably from about 0.01% to about 5% by weight of the compound of the invention based on the weight percentage of the total composition.

The amount of antibiofouling composition may be delivered in an amount of about 1 mg/l to about 1000 mg/l, advantageously from about 2 mg/l to about 500 mg/l, and preferably from about 20 mg/l to about 140 mg/l.

Antibiofouling compositions for water treatment generally comprise transcription factor modulating compounds of the invention in amounts from about 0.001% to about 50% by weight of the total composition. Other components in the antibiofouling compositions (used at 0.1% to 50%) may include, for example, 2-bromo-2-nitropropane-1,3-diol (BNPD), β-nitrostyrene (BNS), dodecylguanidine hydrochloride, 2,2-dibromo-3-nitrilopropionamide (DBNPA), glutaraldehyde, isothiazolin, methylene bis(thiocyanate), triazines, n-alkyl dimethylbenzylammonium chloride, trisodium phosphate-based, antimicrobials, tributyltin oxide, oxazolidines, tetrakis (hydroxymethyl)phosphonium sulfate (THPS), phenols, chromated copper arsenate, zinc or copper pyrithione, carbamates, sodium or calcium hypochlorite, sodium bromide, halohydantoins (Br, Cl), or mixtures thereof.

Other possible components in the compositions of the invention include biodispersants (about 0.1% to about 15% by weight of the total composition), water, glycols (about 20-30%) or Pluronic (at approximately 7% by weight of the total composition). The concentration of antibiofouling composition for continuous or semi-continuous use is about 5 to about 70 mg/l.

Antibiofouling compositions for industrial water treatment may comprise compounds of the invention in amounts from about 0.001% to about 50% based on the weight of the total composition. The amount of compound of the invention in antibiofouling compositions for aqueous water treatment may be adjusted depending on the particular environment. Shock dose ranges are generally about 20 to about 140 mg/l; the concentration for semi-continuous use is about 0.5X of these concentrations.

The invention also pertains, at least in part, to a method of regulating biofilm development. The method includes administering a composition which contains a transcription factor modulating compound of the invention. The composition can also include other components which enhance the ability of the composition to degrade biofilms.

The composition can be formulated as a cleaning product, e.g., a household or an industrial cleaner to remove, prevent, inhibit, or modulate biofilm development. Advantageously, the biofilm is adversely affected by the administration of the compound of the invention, e.g., biofilm development is diminished. These compositions may include compounds such as disinfectants, soaps, detergents, as well as other surfactants. Examples of surfactants include, for example, sodium dodecyl sulfate; quaternary ammonium compounds; alkyl pyridinium iodides; TWEEN 80, TWEEN 85, TRITON X-100; BRIJ 56; biological surfactants; rhamnolipid, surfactin, visconsin, and sulfonates. The composition of the invention may be applied in known areas and surfaces where disinfection is required, including but not limited to drains, shower curtains, grout, toilets and flooring. A particular application is on hospital surfaces and medical instruments. The disinfectant of the invention may be useful as a disinfectant for bacteria such as, but not limited to, *Pseudomonadaceae, Azatobacteraceae, Rhizabiaceae, Mthylococcaceae, Halobacteriaceae, Acetobacteraceae, Legionellaceae, Neisseriaceae,* and other genera.

The invention also pertains to a method for cleaning and disinfecting contact lenses. The method includes contacting the contact lenses with a solution of at least one compound of the invention in an acceptable carrier. The invention also pertains to the solution comprising the compound, packaged with directions for using the solution to clean contact lenses.

The invention also includes a method of treating medical indwelling devices. The method includes contacting at least one compound of the invention with a medical indwelling device, such as to prevent or substantially inhibit the formation of a biofilm. Examples of medical indwelling devices include catheters, orthopedic devices and implants.

A dentifrice or mouthwash containing the compounds of the invention may be formulated by adding the compounds of the invention to dentifrice and mouthwash formulations, e.g., as set forth in Remington's Pharmaceutical Sciences, 18th Ed., Mack Publishing Co., 1990, Chapter 109 (incorporated herein by reference in its entirety). The dentifrice may be formulated as a gel, paste, powder or slurry. The dentifrice may include binders, abrasives, flavoring agents, foaming agents and humectants. Mouthwash formulations are known in the art, and the compounds of the invention may be advantageously added to them.

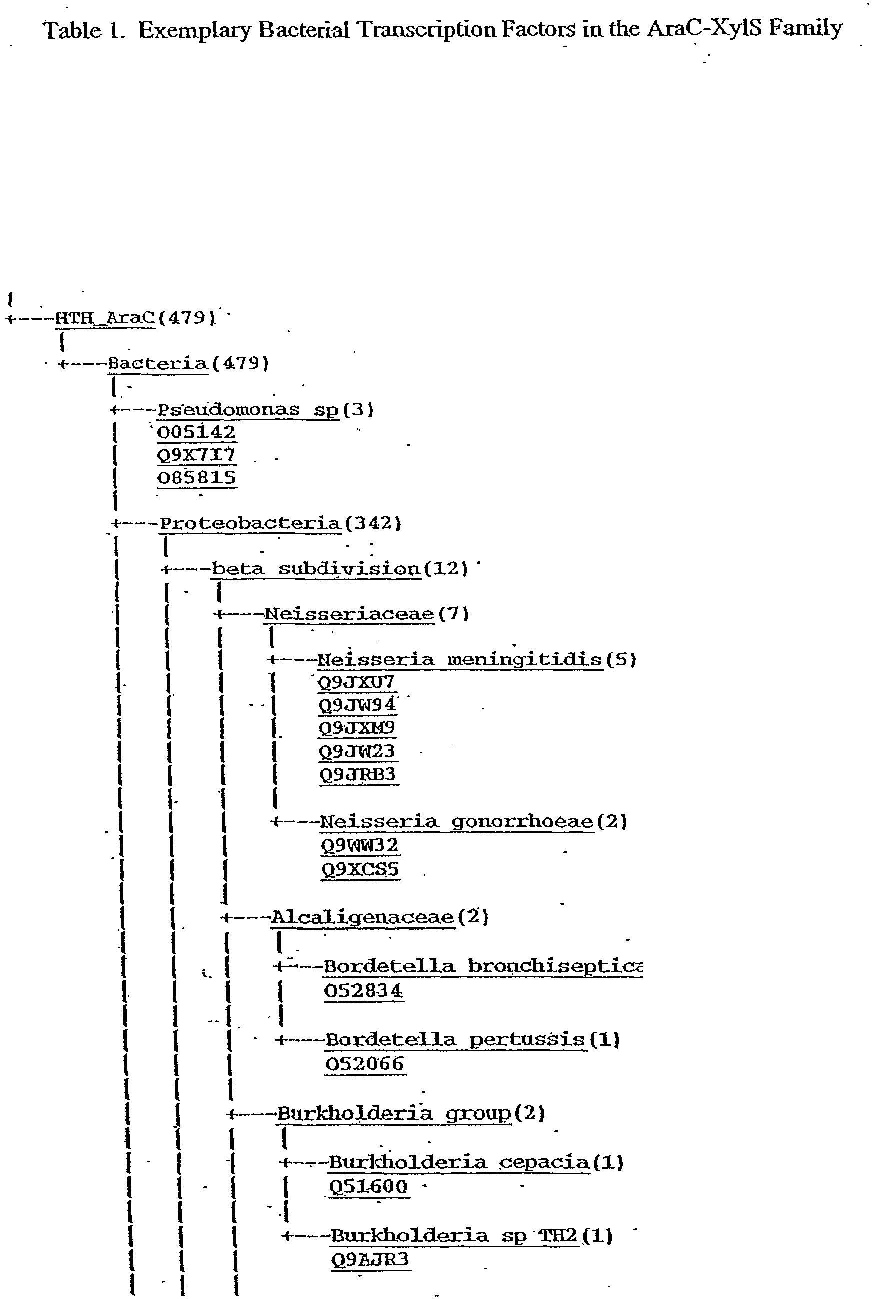

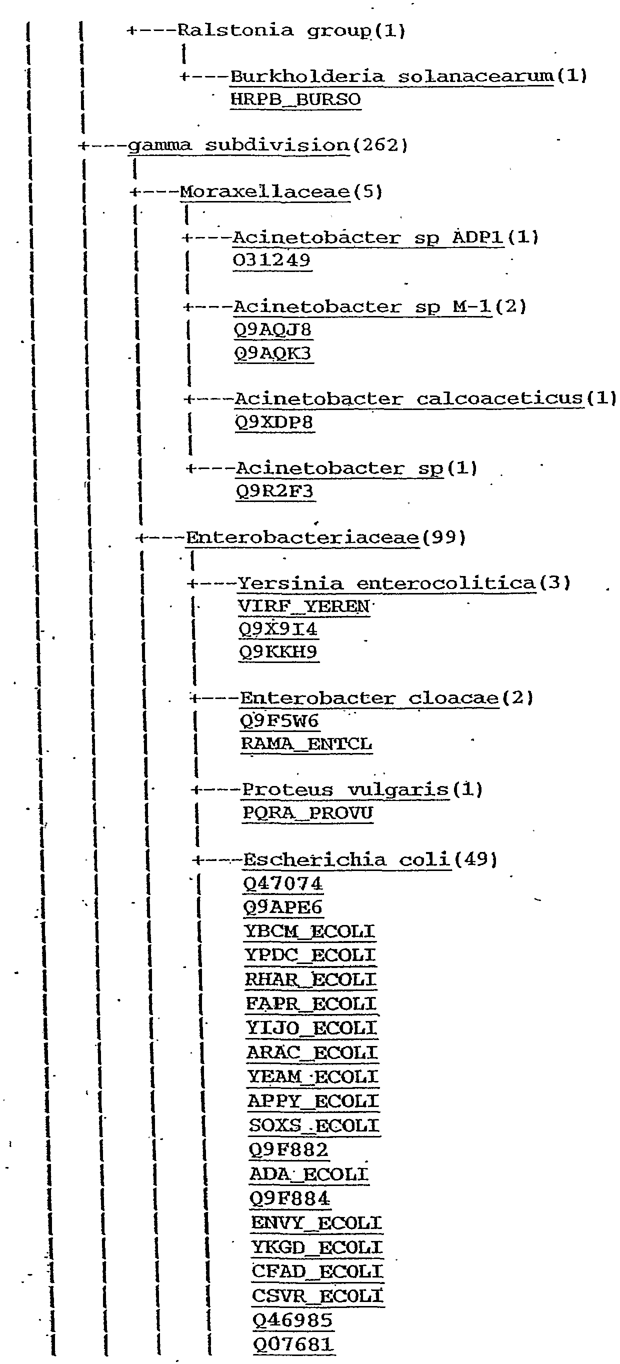

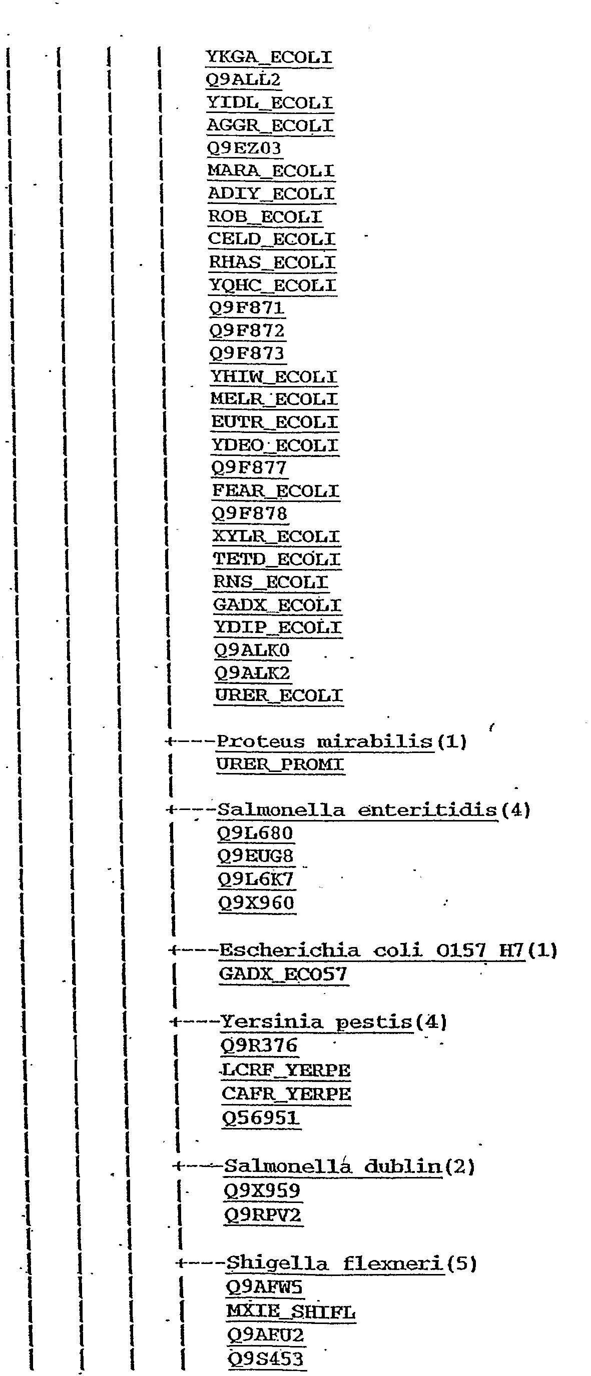

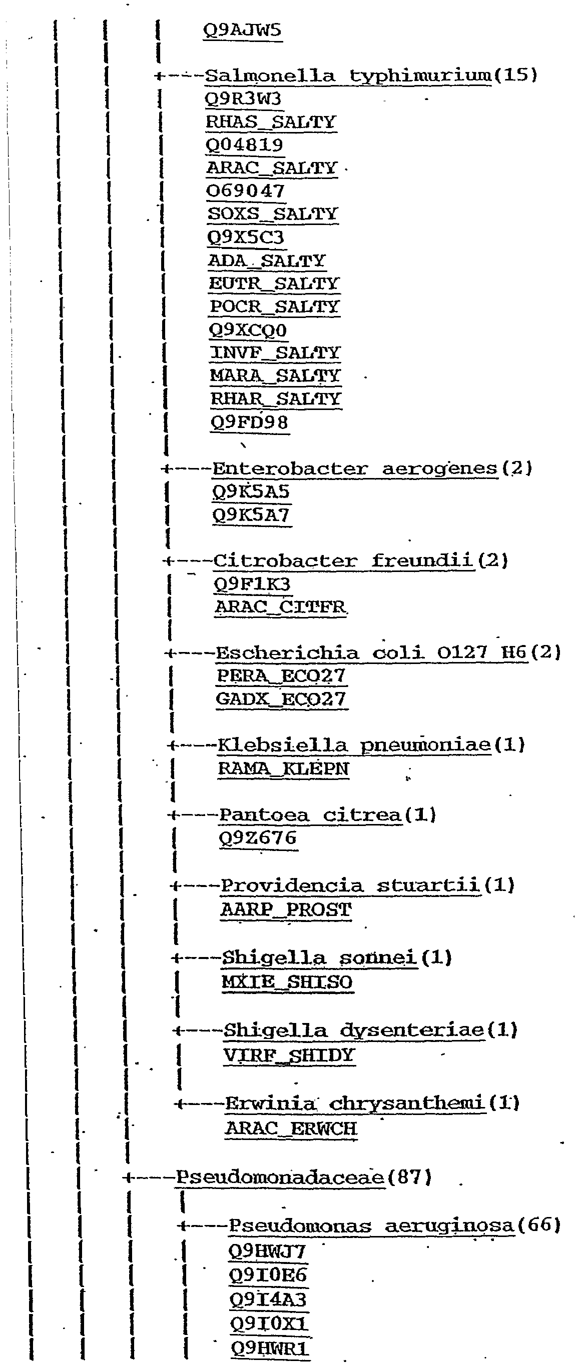

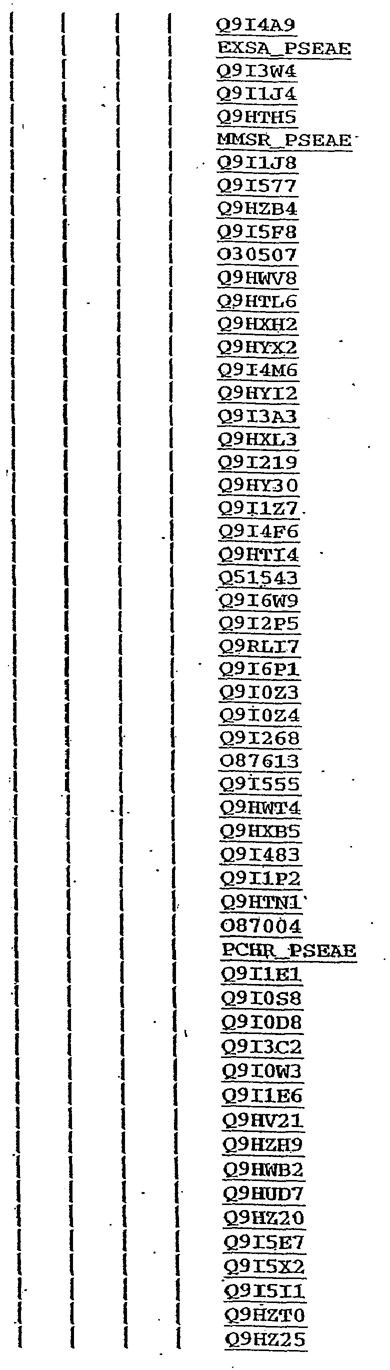

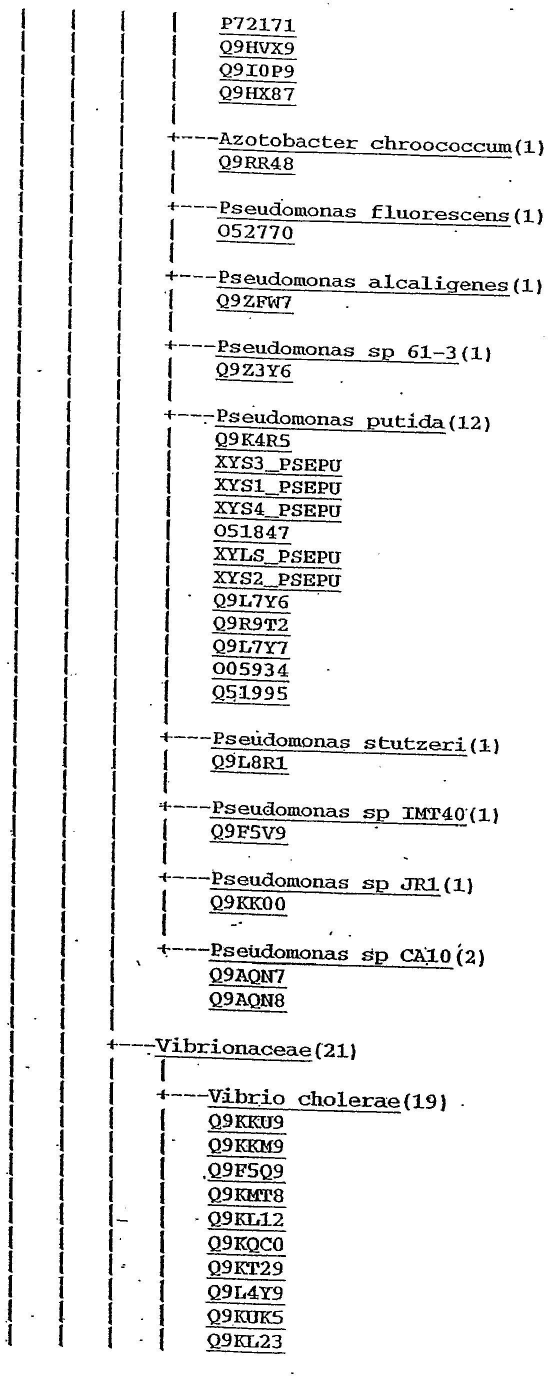

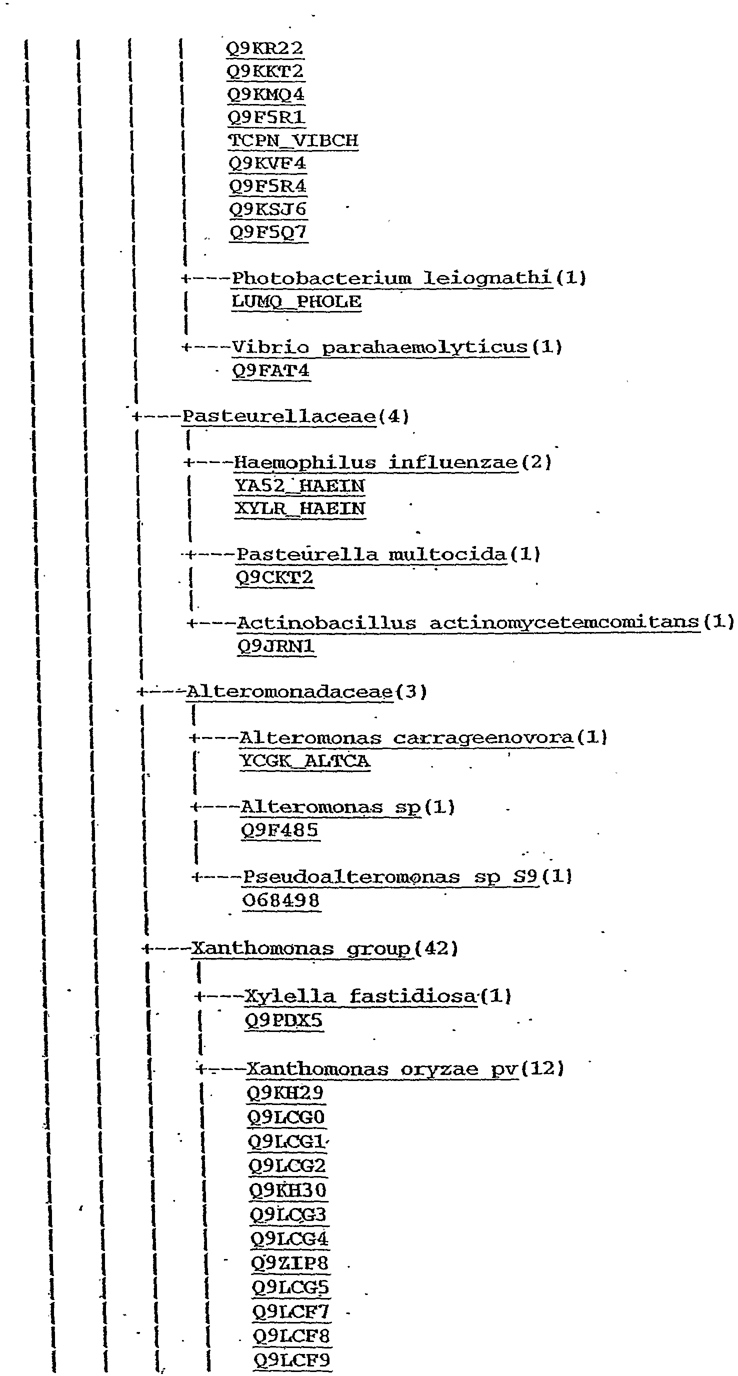

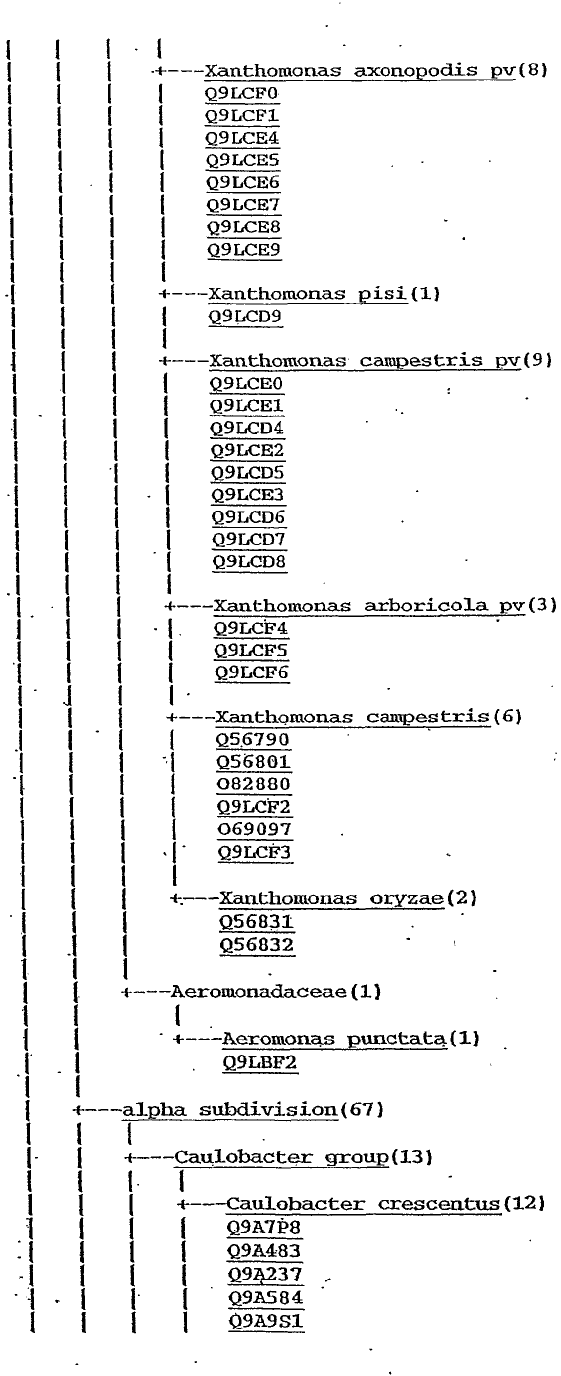

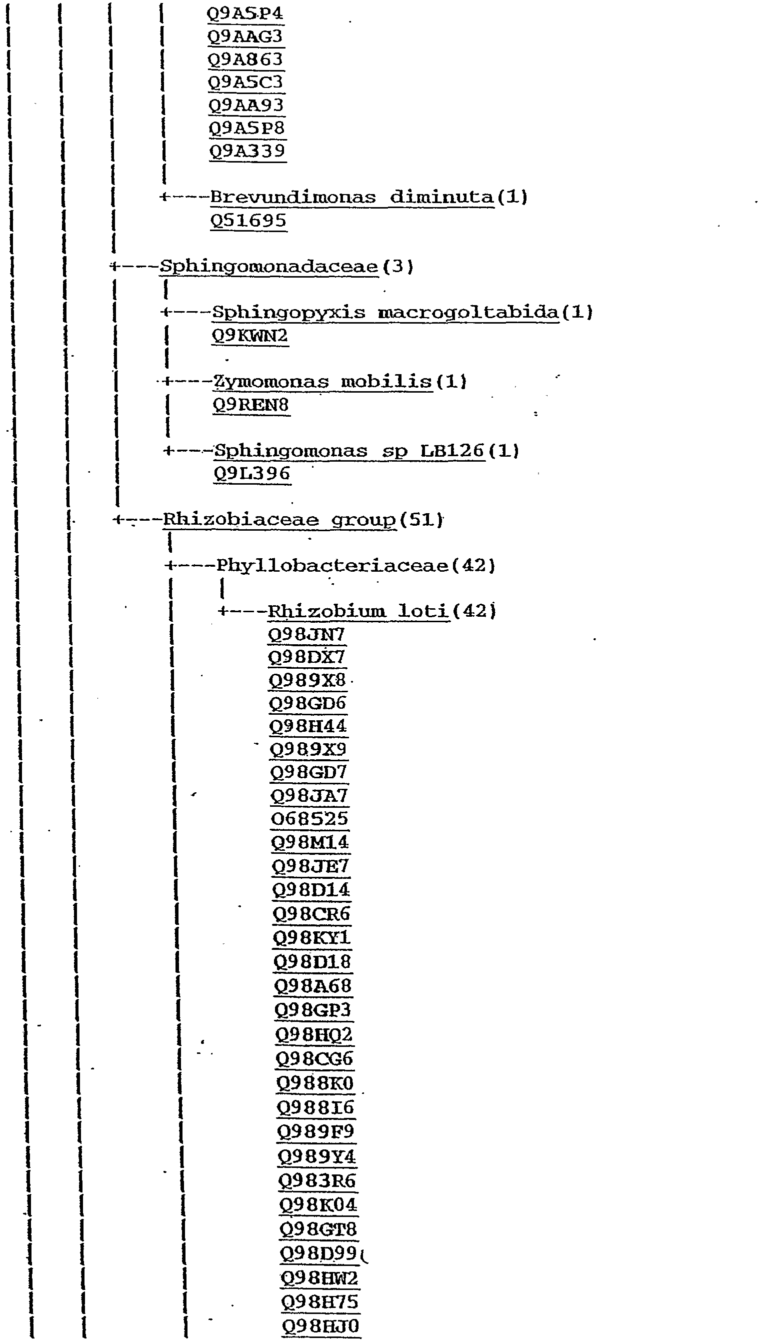

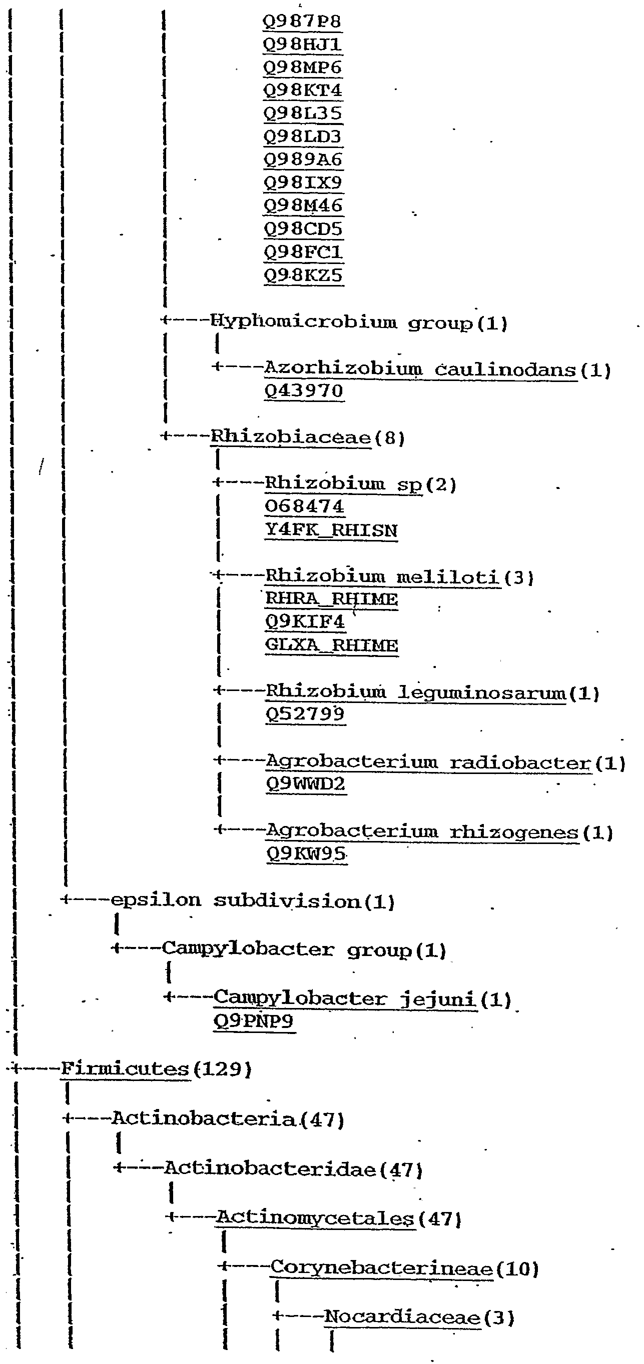

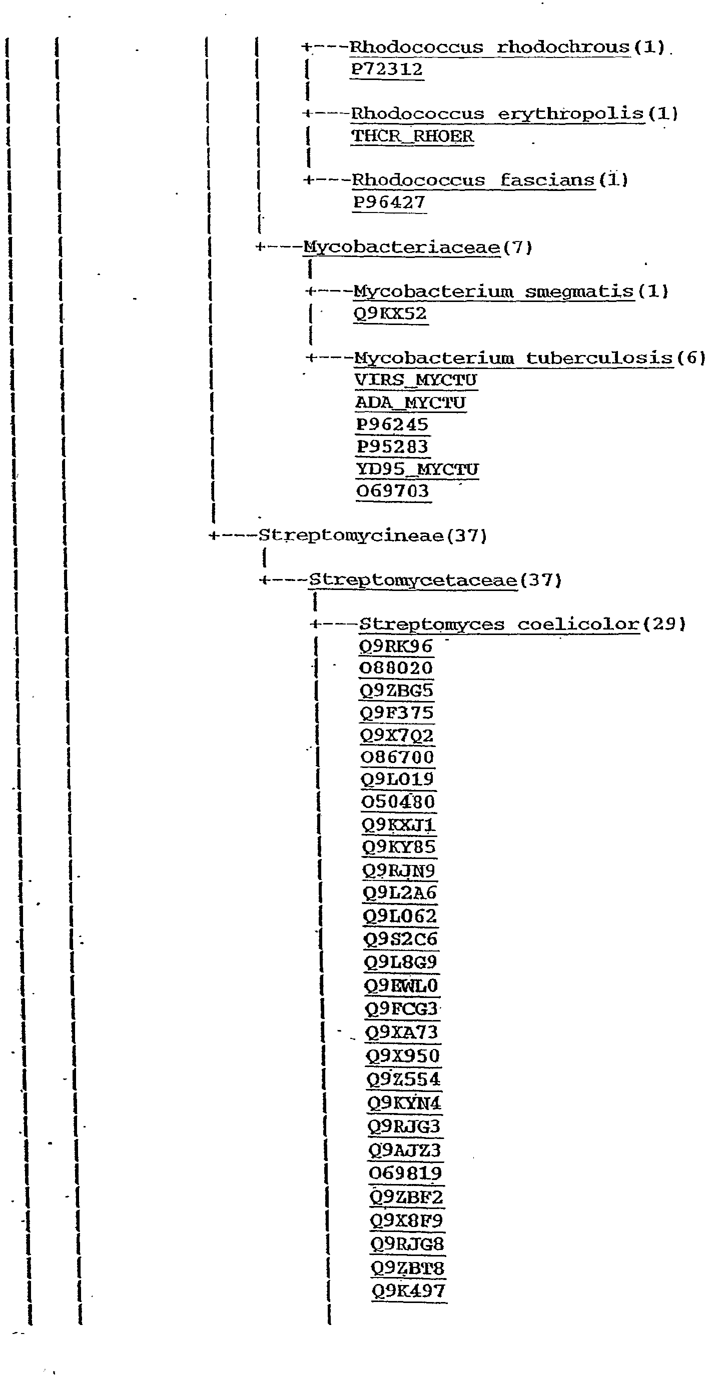

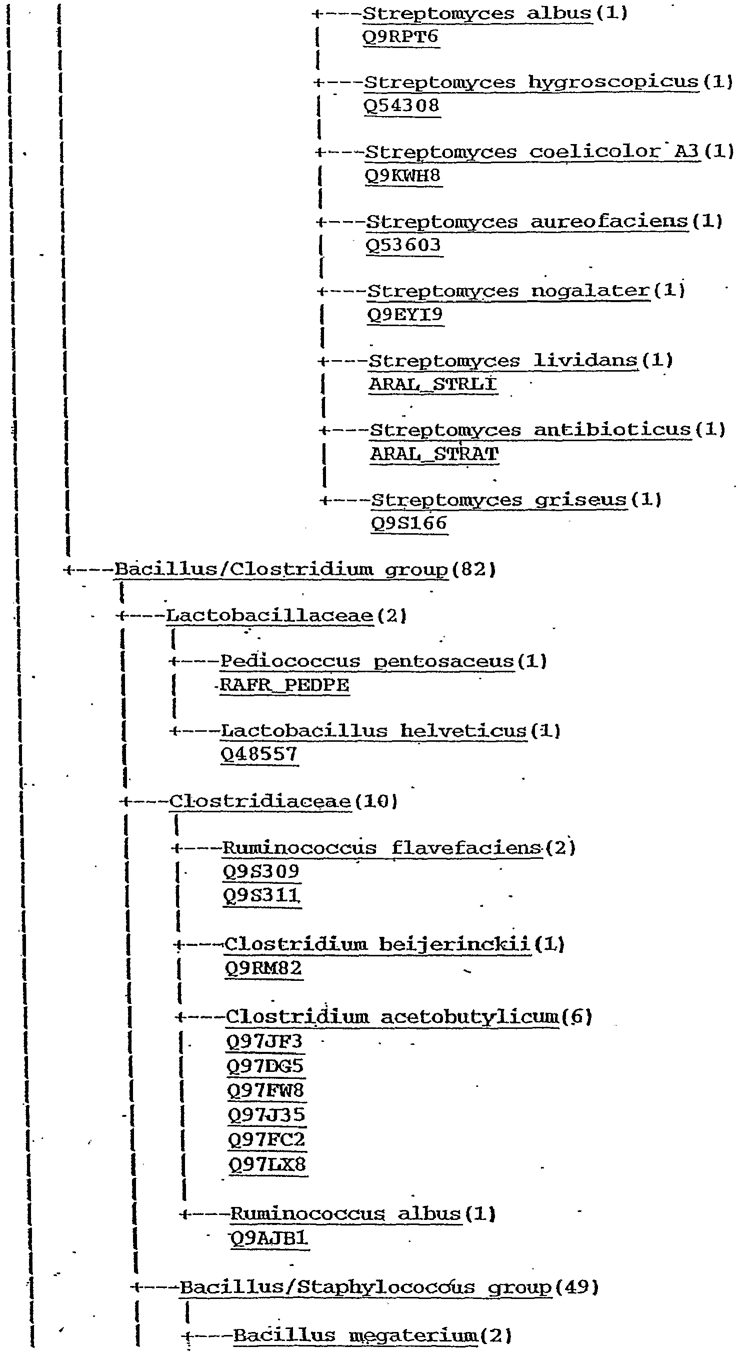

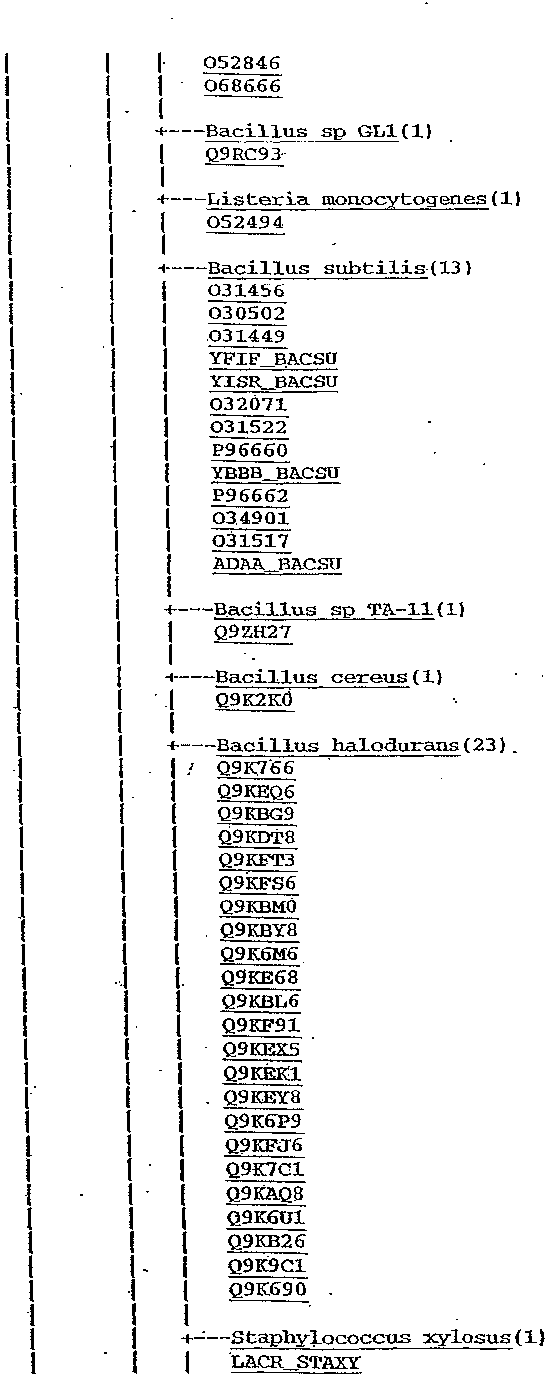

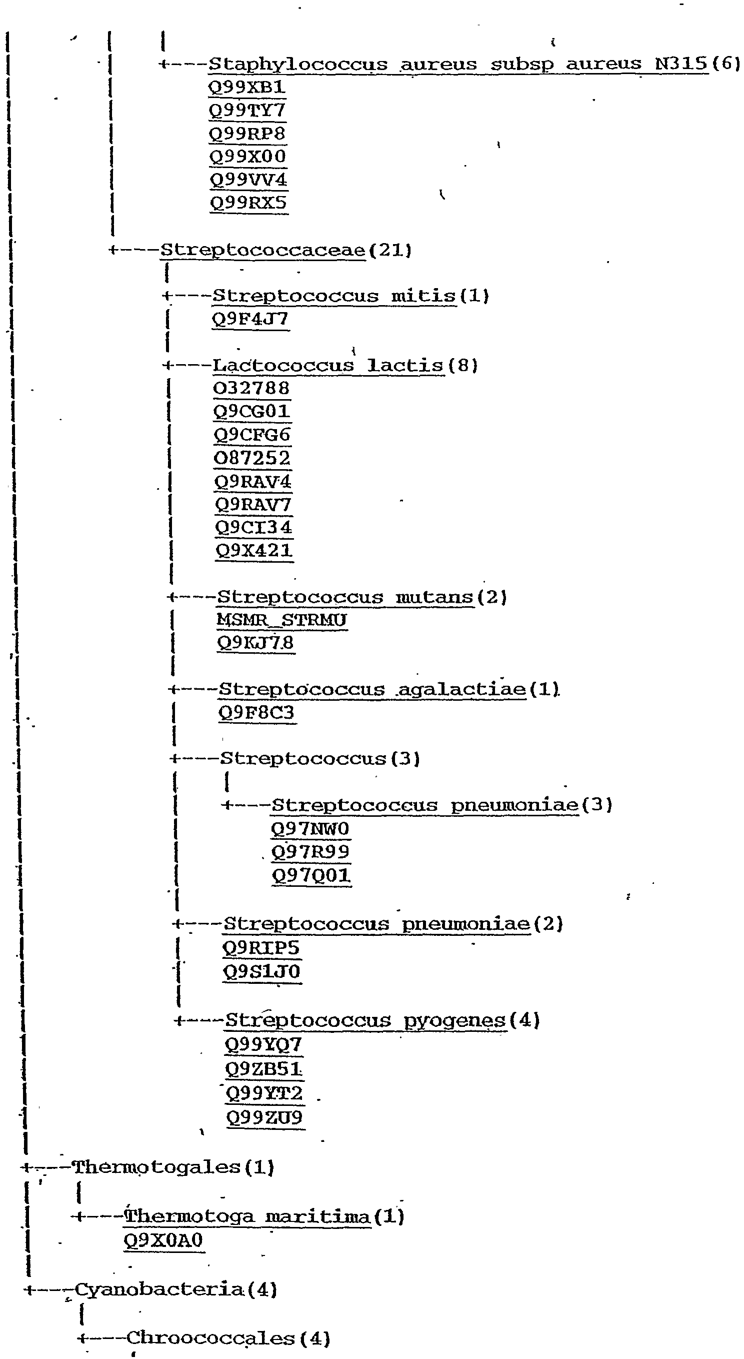

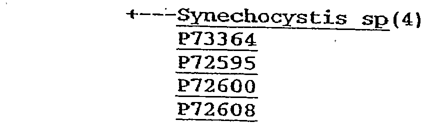

The practice of the present invention will employ, unless otherwise indicated, conventional techniques of cell biology, cell culture, molecular biology, microbiology, recombinant DNA, and immunology, which are within the skill of the art. Such techniques are explained fully in the literature. See, for example, Genetics; Molecular Cloning A Laboratory Manual, 2nd Ed., ed. by Sambrook, J. et al. (Cold Spring Harbor Laboratory Press (1989)); Short Protocols in Molecular Biology, 3rd Ed., ed. by Ausubel, F. et al. (Wiley, NY (1995)); DNA Cloning, Volumes I and II (D. N. Glover ed., 1985); Oligonucleotide Synthesis (M. J. Gait ed. (1984)); Mullis et al. U.S. Patent No: 4,683,195; Nucleic Acid Hybridization (B. D. Hames & S. J. Higgins eds. (1984)); the treatise, Methods In Enzymology (Academic Press, Inc., N.Y.); Immunochemical Methods In Cell And Molecular Biology (Mayer and Walker, eds., Academic Press, London (1987)); Handbook Of Experimental Immunology, Volumes I-IV (D. M. Weir and C. C. Blackwell, eds. (1986)); and Miller, J. Experiments in Molecular Genetics (Cold Spring Harbor Press, Cold Spring Harbor, N.Y. (1972)).

The contents of all references, pending patent applications and published patents, cited throughout this application are hereby expressly incorporated by reference.

The invention is further illustrated by the following examples, which should not be construed as further limiting.

### Examples

### Example 1: Generation of Knockout Bacteria

The parental strains, KM-D and C189, were isolated from an intestinal fistula (Maneewannakul and Levy. 1996. 40:1695) and a patient with a cystitis infection (Rippere-Lampe. 2001 Infect. Immunity 69:3954), respectively. In frame deletions of specific genes in KM-D were constructed by crossover PCR and allelic exchange (Link et al. J. Bacteriol. 1997 179:6228). A 1 kb DNA fragment consisting of 500bp flanking the upstream and downstream portions of the sequence targeted for deletion, separated by a 33 nucleotide spacer, was constructed by crossover PCR and cloned into the NotI-BamHI site of the suicide vector pSR47s. pSR47s contains the R6K origin of replication, rendering it dependent on the π proteins, the kanamycin resistance gene from Pn903 and the *B. subtilis* sacB gene, used as a counterselectable marker. Plasmids with the cloned crossover PCR fragments were transferred from E. Coli S17.1 λpri to KM-D by conjugation, and exconjugants were selected on M9 minimal medium containing 0.2% glucose and 30ug/ml kanamycin. KM-D exconjugants were then grown overnight at 37 C in LB without antibiotics. The overnight cultures were diluted in double distilled water and 105-106 colony forming units were plated on L agar containing 5% sucrose and incubated at 30 C overnight. The resulting colonies were plated on LB plus kanamycin and LB alone. Kanamycin sensitive colonies were tested for the presence or absence of the wild type and deleted alleles by PCR with allele specific primers.

The crossover PCR products used for the in-frame deletion have a 33 nucleotide stuffer sequence containing a SpeI restriction site. In order to restore the deleted genes into their original loci, the wild type genes were amplified from KM-D colonies with primers that created SpeI restriction sites at both ends of the open reading frame. These fragments were restricted with SpeI, and ligated to the plasmids used to make the corresponding in frame deletions. This procedure recreates the original gene with an additional seven amino acids MVINLTG at the amino terminus. This complementation plasmid was recombined into the chromosome of the appropriate mutant strains by allelic exchange as described above, and the presence of the wild type allele was confirmed by PCR.

| Strain | Relevant characteristics/ genotype | Reference |
|---|---|---|
| S17.1λ*pir* | *lamB F supE44 thi-1 thr-1 leuB6 lacY1 tonA21 hsdR hsdM recA pro RP4:2- Tc::Mu::Km:Tn7 λpir* | |
| DH5αλ*pir* | *F-phi80 lacZΔM15 endA1* | |
| | *recA1 hsdR17(r-m+) supE44 thilgyrA96 relA1Δ(lacZYA-argF) U169 λpir* | |
| KMD | Wild type clinical isolate, *marR (mar^{C})* | Maneewannakul and Levy. 1996. 40:1695 |
| PC1012 (SRM) | KMD, *soxS, rob, marA* | This study |
| PC1003 | KMD, *rob* | This study |
| PC1040 | PC1003::*rob* | This study |
| PC1038 | PC1012::*rob* | This study |
| PC1005 | KMD, *soxS* | This study |
| PC1035 | PC1005::*soxS* | This study |
| PC1037 | PC1012::*soxS* | This study |
| PC1033 | PC1012::*marA* | This study |
| C189 | Wild type clinical cyctitis isolate | [Rippere-Lampe (2001) Infect. Immun. 69: 3954] |
| PC0124-90R | C189, *rob* | This study |
| PC0124-90S | C189, *soxS* | This study |
| | | |
| | | |
| Plasmid | | |
| pSR47s | Km^{R} R6*KoriV*RP4*oriT sacB* | |
| *pPCΔrob* | pSR47s with DNA sequences flanking *rob* | |
| *pPCΔsoxS* | pSR47s with DNA sequences flanking *soxS* | |
| pPCΔ*marA* | pSR47s with DNA sequences flanking *marA* | |

### Example 2. Identification of compounds

MarA and Rob-DNA co-crystals suitable for structural analysis have been produced and are available under Protein Data Bank ID codes 1BL0 and 1D5Y, respectively.

A structure-based drug design approach was used to identify inhibitors of these proteins. Briefly, the atomic coordinates of portions of the MarA and Rob DNA binding domains were used as "active site" templates in computer aided small molecule docking experiments. a set of combinatorial chemistry scaffolds was then docked to these templates and a number of high-scoring scaffolds were identified. These scaffolds were then used to identify chemical structures for structurally similar molecules. Five structurally unique classed of Mar inhibitors were identified.

Structures of two classes of these compounds are shown below: 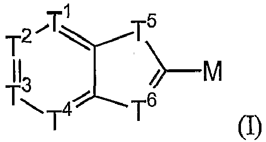 wherein
T¹, T², T³, T⁴, T⁵, and T⁶ are each independently substituted or unsubstituted carbon, oxygen, substituted or unsubstituted nitrogen, or sulfur;
M is hydrogen, alkyl, alkenyl, heterocyclic, alkynyl, or aryl, or pharmaceutically acceptable salts thereof
and 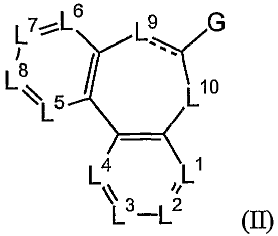 wherein
G is substituted or unsubstituted aromatic moiety, heterocyclic, alkyl, alkenyl, alkynyl, hydroxy, cyano, nitro, amino, carbonyl, or hydrogen; and L¹, L², L³, L⁴, L⁵, L⁶, L⁷, L⁸, L⁹, and L¹⁰ are each independently oxygen, substituted or unsubstituted nitrogen, sulfur and or substituted or unsubstituted carbon, and pharmaceutically acceptable salts thereof.

In a preferred embodiment, structure I is a 2,6-substituted benzoimidazole, such as: 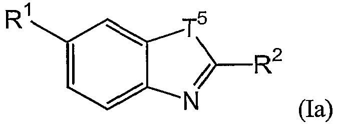 wherein
T⁵ is NOH, NOCOCO₂H, or a substituted or unsubstituted straight or branched C₁-C₅ alkyloxy-substituted nitrogen atom;
R¹ is an electron-donating or electron-withdrawing group, substituted or unsubstituted alkyl group, substituted or unsubstituted aryl group, or substituted or unsubstituted hetetocylic group; and
R² is a substituted or unsubstituted aryl group, substituted or unsubstituted acyl group, or substituted or unsubstituted heterocyclic group. Further examplary R¹ and R² groups are illustrated in Example 5, *infra.*

In another preferred embodiment, structure II is a substituted triazineoxazepine, such as: 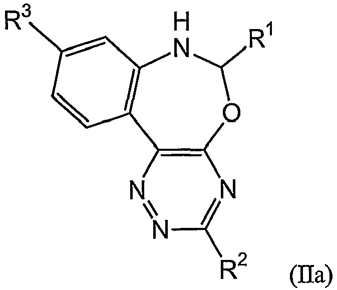 wherein
each of R¹, R², and R³ is an electron-donating or electron-withdrawing group, substituted or unsubstituted alkyl group, substituted or unsubstituted aryl group, or substituted or unsubstituted hetetocylic group.

### Example 3. Modification of Compounds of Formula Ia

The classes of compounds identified will be modified to optimize their activity. For example, the core structure of Formula I contains two main points of diversity (R¹ and R²) as shown in Formula Ia, which can be explored extensively through a variety of chemical modifications (see below). To establish a structure activity relationship, R¹ will be modified by substitution with various electron donating, electron withdrawing, alkyl, aryl, and heterocyclic sidechains. Additional modifications of R² will include a wide variety of substituted aryl groups, heterocycles and acyl sidechains. The large chemical diversity of potential derivatives obtained from this series will greatly facilitate the optimization of some of the preliminary Trancription factor modulators.

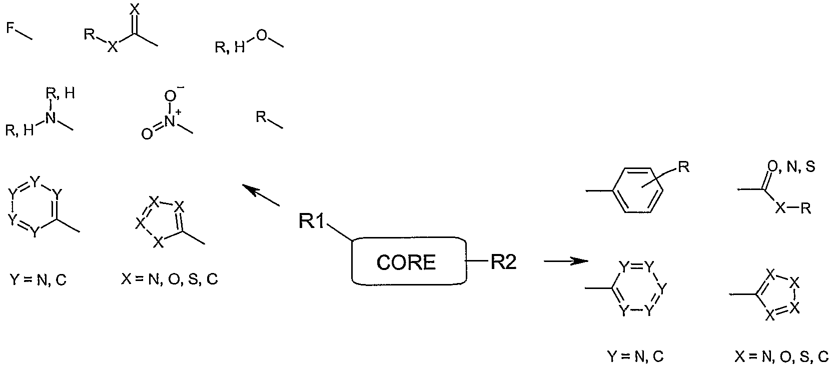

The synthetic medicinal chemistry plan for developing a structure activity relationship around Mar inhibitor structure class I.

### Example 4: Development of DNA-protein binding assays

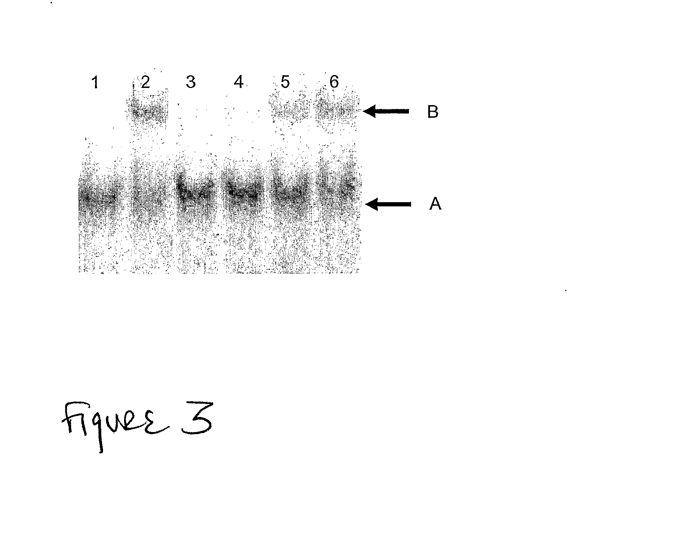

An Electrophoretic mobility shift assay (EMSA) was developed for a qualitative assessment of the activity of our trancription factor modulators to determine if they interrupt DNA-protein interactions *in vitro.* Briefly, 5 nM of a MarA (AraC) family member (or a concentration where ∼50% of a radiolabeled (³³P) double-stranded DNA probe is bound to the protein) is incubated for 30 min at room temperature either in the absence (DMSO (solvent) alone) or presence of a Mar inhibitor. Subsequently, 0.1 nM of the (³³P) labeled DNA probe is added and the mixture is allowed to equilibrate for 15 min at room temperature. The mixture is then resolved on a non-denaturing polyacrylamide gel and the gel is analyzed by autoradiography. As illustrated in figure 3, different Trancription factor modulators have varying activities against SoxS *in vitro* in an EMSA: Compound A is very active, Compound C is moderately active, and Compound D lacks activity (Fig. 3). These data are useful in driving subsequent medicinal chemistry efforts to increase inhibitor potency.

### Example 5: Development of luminescence assays

A quantitative chemiluminescence-based assay is being used to measure the DNA binding activity of various MarA (AraC) family members. With this technique, a biotinylated double-stranded DNA molecule (2 nM) is incubated with a MarA (AraC) protein (20 nM) fused to 6-histidine (6-His) residues in a streptavidin coated 96-well microtiter (white) plate (Pierce Biotechnology, Rockford, IL). Unbound DNA and protein are removed by washing and a primary monoclonal anti-6His antibody is subsequently added. A second washing is performed and a secondary HRP-conjugated antibody is then added to the mixture. Excess antibody is removed by a third wash step and a chemiluminescence substrate (Cell Signaling Technology, Beverly, MA) is added to the plate. Luminescence is read immediately using a Victor V plate reader (PerkinElmer Life Sciences, Wellesley, MA). Compounds that inhibit the binding of the protein to the DNA result in a loss of protein from the plate at the first wash step and are identified by a reduced luminescence signal. The concentration of compound necessary to reduce signal by 50% (EC₅₀/IC₅₀) can be calculated using serial dilutions of the inhibitory compounds. For example, the EC₅₀s of Compound A for SoxS and SlyA (an unrelated protein and MarR family member) are 9.2 and 150 µM, respectively, demonstrating a specificity of the compound. Also, single Trancription factor modulators that affect different transcription factors have been identified as shown below:

**Table X. Activity of selected Trancription factor modulators against disparate MarA (AraC) family members.**

| | | | **EC₅₀ (µM)** | |
|---|---|---|---|---|
| **Host-Protein** | **% Identity to MarA** | **K*_{D}* (nM)** | **Compound E** | **Compound F** |
| E. coli | | | | |
| MarA | 100 | 44 | | |
| SoxS | 42 | 31 | 0.82 | 8.3 |
| Rob | 51 | 8.8 | 1.3 | 28 |

| S. typhimurium | | | | |
|---|---|---|---|---|
| Rma | 38 | 137 | 1.8 | 17 |

| P. mirabilis | | | | |
|---|---|---|---|---|
| PqrA | 40 | 268 | 1.4 | 13.6 |

| P. aeruginosa | | | | |
|---|---|---|---|---|
| ExsA | 24 | 190 | 1.9 | 15.6 |

### Example 6: Development of an Animal model of infection

CD-1 female mice were housed in cages prior to surgery. Mice were diuresed on a diet consisting of water containing 5% glucose and restricted solid food. On the day of the experiment, each mouse was anaesthetized with isoflurance and the abdominal area was shaved and bathed with iodine and alcohol. A small incision (approx. 15 mm) was made through the outer most skin layer just above the urethra. Once the inner skin layer was exposed, another small incision was made through the peritoneum, exposing the inner cavity and the bladder. A small puncture was made in the bladder to aspirate excess urine and to introduce the infectious bacterial inoculum. From an overnight culture bacteria were washed, diluted, and 100ul of this culture (∼10⁷ colony forming units) was used to inoculate the mice.

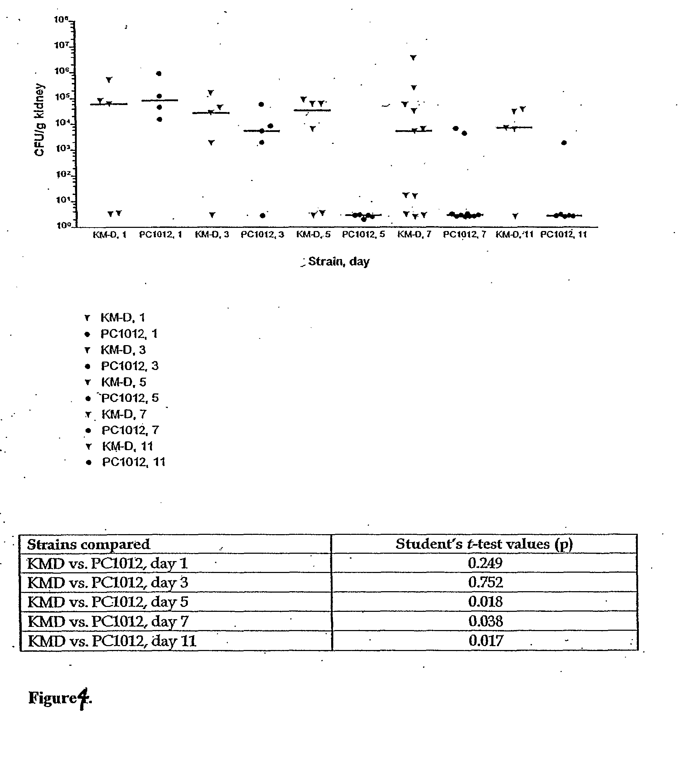

After a designated period of infection, routinely between 24h and 11 days, mice were sacrificed and their kidneys removed. Individual mouse kidney weights were recorded and the kidneys were then suspended in 5 ml of sterile PBS. The plates to determine CFU/gram of kidney. Representative data are presented in Figures 4-6.

First, the infectivity of a wild type clinical isolate lacking all three transcription factors was tested. As illustrated in Figure 4, bacteria lacking soxS, rob, and mar (the PC1012 triple knock out strain) are capable of infecting the host, as indicated by the presence of bacteria in the kidneys of the animals at days 1 and 3, but are unable to maintain the infection (see days 5, 7, and 11). The wild type bacteria (KM-D), in contrast, maintain the infection throughout the course of the study.

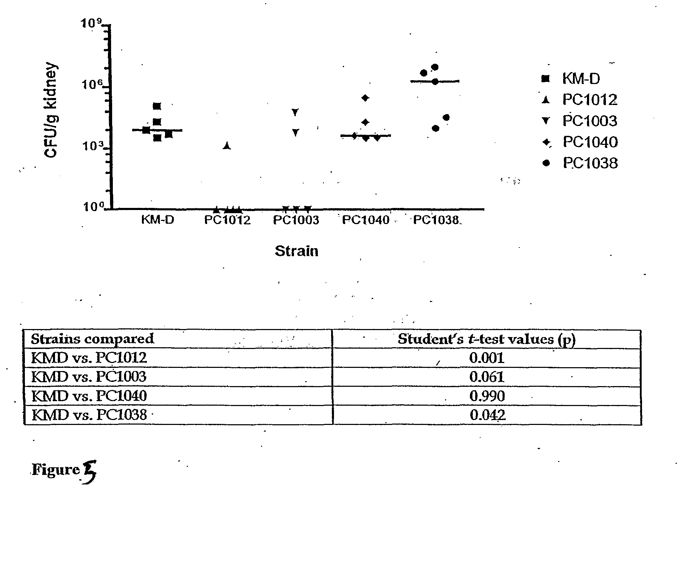

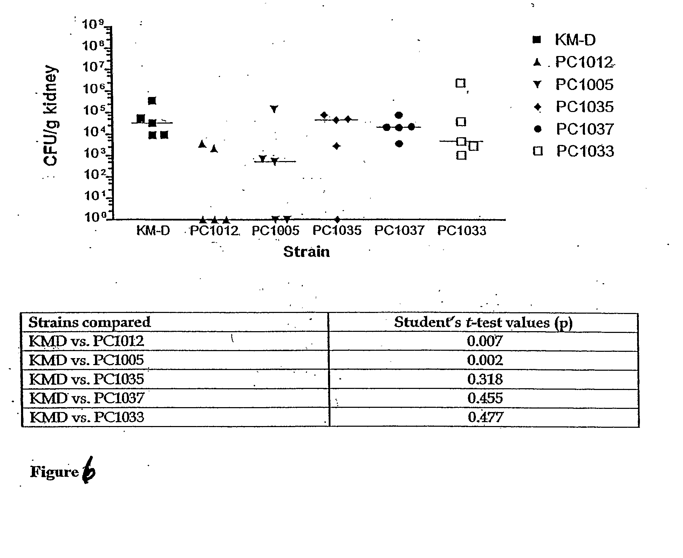

In order to study the effects on virulence following the deletion of a single transcrition factor, i.e., soxS or rob, the appropriate bacterial strains were constructed (see the table in Example 1) and tested in the UTI model. Figure 5 shows that a rob knock out strain (PC1003) is less virulent than the KMD wild type strain. Restoring rob in the rob knock out restores virulence (PC1040) as does restoring rob in the PC1012 triple knock out strain (PC1038). Figure 6 shows similar results for soxS. The KMD soxS knock out strain (PC1005) is less virulent than the KMD wild type strain. Restoring soxS to the soxS knock out (PC1035) or to the triple knock out (PC1037) restores virulence. In addition, Figure 6 also shows that restoring marA to the triple knock out (PC1033) restores virulence.

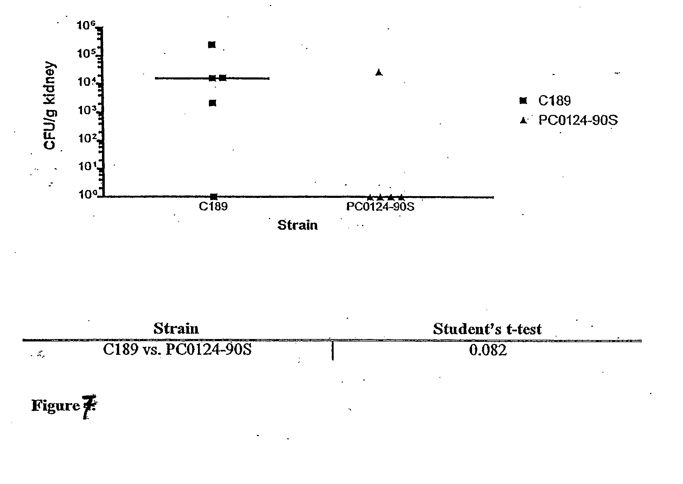

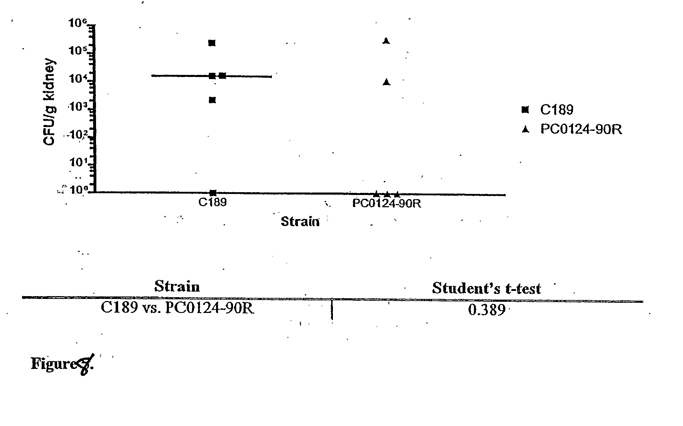

Figures 7 and 8 examine the effect of knocking out soxS or rob in a clinical isolate, C189. Figure 7 shows that the soxS knock out (PC0124-90S) is less virulent than the wild type isolate. Similarly, Figure 8 shows that the rob knock out (PC0124-90R) is less virulent than the C 189 clinical isolate.

Thus, deletion of rob or soxS alone is sufficient to confer the avirulent phenotype. Moreover, supplying either rob or soxS in their original chromosomal locations in either the single (PC1037 and PC1038) or triple (SRM) knockout backgrounds fully restored virulence in these strains. These data convincingly demonstrate that both SoxS and Rob are virulence factors. With respect to marA, when marA is supplied in its original chromosomal location in the triple knockout background (PC1012), virulence is fully restored.

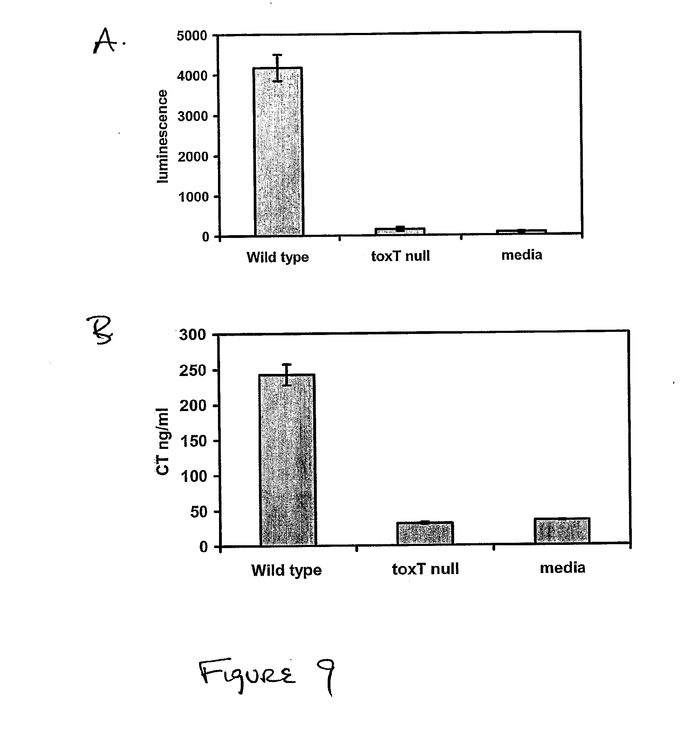

Further, E.coli SRM was used in a pyelonephritis model of infection to show that the triple knockout was significantly less infectious than its parent strain (Figures 9A-B).

Thus, like SoxS and Rob, MarA can be considered a virulence factor in this model.

### Example 7. Activity of Transcription Factor Inhibitors In Vivo

The ability of small organic inhibitors of transcription factors of the AraC family to prevent infection was tested. These organic molecules inhibit MarA, SoxS, Rob, and other MarA family molecules, e.g., Rma from *Salmonella enterica* serovar Typhimurium and PqrA from *Proteus mirabilis.* Two organic molecules from two structurally unrelated classes of inhibitors were found to work well in vitro and one was tested in the in vivo urinary tract infection model. In a first experiment, infected mice were subjected to dosing at time of infection and at 6, 24, 30, 48, 54, 72, and 96 hours post-infection. Mice were sacrificed at 120 hours after infection.

The data for two representative experiments are presented below:

| Dose (mg/kg) | # of Mice infected | Student's t-test (p values) |
|---|---|---|
| 0 | 4/5 (80%) | na |
| 1 | 0/5 (0%) | 0.006 |
| 5 | 1/5 (20%) | 0.034 |
| 10 | 2/5 (40%) | 0.066 |
| 20 | 2/5 (40%) | 0.359 |

| Dose (mg/kg) | # of Mice infected | Student's t-test (p values) |
|---|---|---|
| 0 | 5/6 (83%) | na |
| 1 | 1/5 (20%) | 0.037 |
| 5 | 1/6 (17%) | 0.013 |
| 10 | 2/5 (40%) | 0.256 |
| 20 | 1/5 (20%) | 0.037 |

In a subsequent experiment, mice were treated at 0 and 24 hours post infection. Data from a representative experiment are shown below:

| Dose (mg/kg) | # of Mice infected | Student's t-test (p values) |
|---|---|---|
| 0 | 6/6 (100%) | na |
| 1 | 3/6 (50%) | 0.009 |
| 5 | 5/6 (17%) | 0.314 |
| 10 | 3/5 (60%) | 0.030 |
| 20 | 2/5 (40%) | 0.023 |

In a final experiment, mice were treated once, at the time of infection. Data from a representative experiment are shown below:

| Dose (mg/kg) | # of Mice infected | Student's t-test (p values) |
|---|---|---|
| 0 | 5/6 | na |
| 0.1 | 5/5 | 0.473 |
| 1 | 2/4 | 0.106 |
| 10 | 4/6 | 0.244 |
| 100 | 0/5 | 0.003 |

### Example 8. Effects on Biofilm formation

Previous data indicate that genes within the MarA and SoxS regulons are involved in biofilm formation. The ability of a few exemplary hits to prevent *in vitro* biofilm formation were demonstrated. These assays were performed according to a published protocol (e.g., O'Toole et al. 1999 Methods Enzymol 310:91) and measure the ability of *E*. *coli* to adhere to the walls of a 96-well polystyrene (abiotic) microtiter plate. As illustrated, the compounds which with inhibitory activity in the *in vitro* DNA binding assays and that lack antibacterial activity, all affect biofilm formation in intact cells. Both of these findings also indicate that the Trancription factor modulators can penetrate the intact bacterial cell.

### Example 9. Evaluate the efficacy of the Trancription factor modulators in murine models of infection.

The acute toxicity and preliminary PK data will be used to prioritize and select compounds for efficacy evaluation in mouse models of infection described below. Initially, the 50% lethal dose (LD₅₀) of the infecting organism will be determined (see below). Subsequently, trancription factor modulators will be tested for efficacy using an infectious dose necessary to produce colonization of the target organ(s) and a constant concentration (25 mg/kg dosed orally (p.o.) once a day for the length of the study) of the transcription factor modulators or vehicle alone as a control. Compounds that perform favorably, e.g., produce a ≥2-log decrease in CFU/g of organ, will then be subjected to a dose response analysis. In these experiments, groups of mice (n=6) will be treated with serial 2-fold dilutions (ranging from 0-50 mg/kg) of a Trancription factor modulator and the ED₅₀, drug concentration necessary to prevent infection in 50% of the treatment group, will be calculated from these data. ED₅₀ determinations with an antibiotic will be performed accordingly and these agents will be used a controls in all experiments.

Trancription factor modulators can be subjected to efficacy analysis in the ascending pyelonephritis mouse model of infection (see above). Briefly, groups of female CD1 mice (n=6) will be diuresed and infected with *E. coli* UPEC strain C189 via intravesicular inoculation. Subsequently, mice will be dosed with a Trancription factor modulator (25 mg/kg), a control compound, e.g., SXT (Qualitest Pharmaceuticals, Huntsville, AL), or vehicle alone (0 mg/kg), via an oral route of administration at the time of infection and once a day for 4 days thereafter, to maintain a constant level of drug in the mice. After a 5-day period of infection and prior to sacrifice via CO₂/O₂ asphyxiation, a urine sample will be taken by gentle compression of the abdomen. Following asphyxiation, the bladder and kidneys will be removed aseptically as previously described. Urine volumes and individual organ weights will be recorded, the organs will be suspended in sterile PBS containing 0.025% Triton X-100, and then homogenized. Serial 10-fold dilutions of the urine samples and homogenates will be plated onto McConkey agar plates to determine CFU/ml of urine or CFU/gram of organ.

Efficacy in these experiments will be defined as a ≥2-log decrease in CFU/ml of urine or CFU/g organ. These values are in accord with previous experiments investigating the treatment of UTI in mice.

Trancription factor modulators that perform favorably, e.g., produce a ≥2-log decrease in CFU/g of organ, will be subjected to a dose response analysis. In these experiments, groups of mice (n=6) mice will be treated with serial2-fold dilutions (ranging from 0-50 mg/kg and using the dosing scheme described above) of a Trancription factor modulator and the ED₅₀, drug concentration necessary to cure infection in 50% of the treatment group, will be calculated from these data. ED₅₀ determinations with a standard antibiotic, e.g., SXT, will be performed accordingly. It is expected that a maximum of 5 compounds would be evaluated in this infection model. In addition, a similar model can be used for *S*. *saprophyticus* and *P. mirabilis* to evaluate a broader spectrum of the lead compounds.
*C. rodentium. C. rodentium* (MPEC) produces a disease in mice that is equivalent to the human infections caused by EPEC and EHEC. This organism is the only A/E lesion producing bacterium that causes infections in mice and is therefore commonly used as a surrogate for studies that investigate the pathogenesis of EPEC and EHEC.

The efficacy of our trancription factor modulators against MPEC will be examined. The LD₅₀ of *C*. *rodentium* DBS100 (ATCC 51459) will be determined using methods known in the art following oral (p.o.) infection of groups of Swiss Webster mice (Taconic Laboratories, Germantown, NY) (*n*=7) with serial 10-fold dilutions of the organism. Once the LD₅₀ is ascertained, mice will be infected with an inoculum sufficient to produce colonization of the colon as described. Feces will be collected at 3, 5, and 7 days post-infection (p.i.), weighed, and homogenized in sterile phosphate buffer saline (PBS) and bacterial load will be determined by serial dilution onto selective media. At 10 days p.i., mice will be sacrificed and entire colons will be removed aseptically, homogenized in PBS, and the bacterial loads will be subsequently determined. Efficacy evaluations will then be performed.
*S. **flexneri.*** Since mice do not develop intestinal disease following infection with *S*. *flexneri,* a murine pulmonary infection model has been used to assess virulence of this organism. The use of small rodents, while not a direct mimic of human infection, is less cumbersome than using the rabbit Sereny or ligated ileal loop models or Macaque monkeys.

In this model, groups of 4-6 week old BALB/cJ mice (The Jackson Laboratory, Bar Harbor, ME) (*n=*7) will be anesthetized and infected with serial 10-fold dilutions (up to ∼10⁸ CFU/ml) of *S. flexneri* through an intranasal route as described previous1. Mice will be sacrificed at 24, 48, and 72 hr post-infection, the lungs will be removed aseptically, homogenized, and the bacterial load will be enumerated via plating on selective media according to an established procedure. An infectious dose that yields a suitable lung infection will be determined from these preliminary experiments and used for subsequent analyses. Efficacy evaluations will then be performed.
*S. **typhimurium.*** It is well established that inbred mice exhibit varying susceptibilities to infection by *Salmonella* spp.. This property is attributed to the absence (i.e., in BALB/c mice [Charles River Labs, Wilmington, MA] which are extremely susceptible to infection) or presence (i.e., in Sv129 mice [The Jackson Laboratory, Bar Harbor, ME] which are moderately resistant to infection) of the natural resistance associated macrophage protein 1 (Nramp1). Nonetheless, murine models of salmonellosis are routinely used to study systemic *Salmonella* infections. Therefore, initial assessments of Trancription factor modulator efficacy will be performed using both strains of mice.

LD₅₀ determinations will be calculated as described above following p.o. infection of BALB/c (8-9 weeks old) or Sv129 mice *(n*=7*)* with *S. typhimurium* SL1344. Once the LD₅₀ is determined, mice will be infected with an inoculum sufficient to produce a systemic model of infection. In these studies, mice will be monitored for weight loss and other gross abnormalities during the course of the infection. Three and six days post-infection, the mice will be sacrificed and tissues, including caecum, Peyer's patches, mesenteric lymph nodes, spleen, and liver will be examined for bacterial load according to published protocols. Depending on the outcome of these studies, a single mouse strain will be chosen for subsequent experiments. The overall goal will be to find an inoculum and host, i.e., a combination that will not rapidly lead to death, which will permit efficacy evaluation of the Trancription factor modulators. Efficacy evaluations will then be performed.
***V. cholerae.*** In order to evaluate the efficacy *in vivo* of the Trancription factor modulators against *V. cholerae,* colonization and lethal infection models will be used. *V. cholerae* 0395 (classical biotype) and E7946 (E1 Tor biotype) and infant (3- to 5-day old) CD-1 and BALB/c mice will initially be used in both models as previously described. For the LD₅₀ determinations, groups of infant mice (*n*=7) will be orally infected with serial 10-fold dilutions (∼10⁴-10⁸ CFU/ml) of overnight cultures of *V. cholera.* The infected mice will be monitored for a period of 5 days and the LD₅₀s will be calculated as described previously. In the colonization model, groups of infant mice *(n=7)* will be pre-starved and then intragastrically infected with an inoculum sufficient to produce colonization of the intestines. Following a period of colonization (∼24-36 hr), the intestines will be aseptically removed, homogenized, and serial dilutions will be plated onto selective media to enumerate the bacterial load. Efficacy evaluations will then be performed.

### Example 10: Whole cell Y. pseudotuberculosis YopH virulence assay.

In order to study the effects of trancription factor modulators on the intact bacterial cell, an assay was developed to measure the effects of inhibiting the activity of LcrF (VirF), a MarA (AraC) family member, on YopH activity in whole cells. YopH is a tyrosine phosphatase and *Yersinia* spp. virulence factor that is secreted by a TTSS in the pathogen. The activity of YopH on *p*-nitrophenyl phosphate (pNPP, an indicator of phosphatase activity) results in the formation of a colored substrate that can be measured spectrophotometrically. *Y. pseudotuberculosis* were incubated in the presence and absence of a Trancription factor modulator and controls were included to measure the inhibitory effects of the compounds themselves on the phosphatase activity of YopH. Compounds that had an effect were excluded from further analysis. This assay identified a number of compounds that adversely affect YopH (expression or secretion of the protein) presumably at the level of LcrF (VirF). These findings also indicate that the trancription factor modulators can penetrate the intact bacterial cell.

### Example 11. Measurement of the effects of the trancription factor modulators in a Y. pseudotuberculosis mouse model of infection.

The acute toxicity and preliminary pharmokinetic data generated will allow selection of compounds for efficacy evaluation in the mouse model of systemic Y. *pseudotuberculosis* infection. Briefly, 8- to 10-week-old BALB/c female mice will be used for all infections and will be housed for a week prior to infection in a BL-2 facility. All mice will be denied food for 16 hr prior to orogastric infection. Two treatment groups (n=6) will be infected orally with a sub-lethal dose (5x10¹⁰ CFU/ml) of *Y. pseudotuberculosis* strain YPIIIpIBI {Mecsas, 2001 #.1233}. Following infection, mice will be dosed via an oral route with 0 (vehicle alone) or 25 mg/kg of a trancription factor modulator once a day for the duration of the study, to maintain a constant level of drug in the mice. Mice will be monitored for weight loss and other gross abnormalities during the course of the infection. Five days post-infection, the mice will be sacrificed and tissues, including small intestine lumen, cecal lumen, large intestine lumen, Peyer's patches, mesenteric lymph nodes, spleen, liver, lungs, and kidneys, and blood will be examined for bacterial load according to an established protocol.

Trancription factor modulators that perform favorably, e.g., produce a ≥2-log decrease in CFU/g of organ, will be subjected to a dose response analysis. In these experiments, groups of mice (n=6) mice will be treated with serial 2-fold dilutions (ranging from 0-50 mg/kg) of a Trancription factor modulator and the ED₅₀, drug concentration necessary to prevent infection in 50% of the treatment group, will be calculated from these data. ED₅₀ determinations with a standard antibiotic, e.g., streptomycin or doxycycline, will be performed accordingly and these agents will be used a controls in all experiments.

### Example 12. Analysis of transcription factor modulators function in a mouse model of infection.

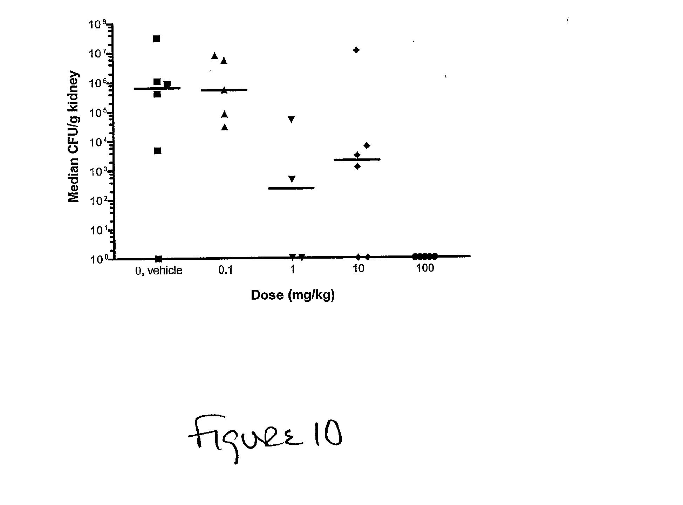

The efficacy of one prototypic inhibitor was investigated in the ascending pyelonephritis model of infection (see above). As illustrated, the administration of a single subcutaneous dose of the inhibitor at the time of infection was sufficient to prevent infection in this *in vivo* model (Fig. 10). Results similar to those obtained with the single 100 mg/kg dose (Fig. 10) were observed using smaller doses with multiple dose regimens (bid x4 d, data not shown). These data are a small molecule proof-of-principle demonstration that our approach is feasible. More recently, preliminary PK data indicate that this compound and other similar molecules are orally bioavailable.

### Example 13. Pharmokinetic studies.

The PK parameters of non-toxic trancription factor modulators will then be evaluated. Briefly, groups of female CD1 mice (n=3) will be fasted overnight prior to dosing and then weighed to calculate dose levels. On the day of the experiment, mice will be given 100 µl of a test article solution containing an exemplary Trancription factor modulator, without detectable acute toxicity, via an oral and/or subcutaneous route of administration. As a control, one additional group of mice treated with the vehicle alone will be used to determine baseline urine and serum levels. Following treatment, mice will be given unrestricted access to both food and water. Plasma and urine samples and individual organs, e.g., kidneys, lungs, spleen, etc., will be collected at various time points and compound concentrations will be determined using standard bioanalytical LC/MS/MS procedures. PK parameters, i.e., maximum drug concentration (Cₘₐₓ), (Tₘₐₓ), drug area under the curve (AUC), drug half-life (T_{1/2}), will be calculated from these data. Any animal(s) removed from the study because of bad injection will be replaced with a new animal from a group of "extra" mice. Animals that die spontaneously after dosing and before 5 hours will be dropped from the study altogether and will not be replaced.

For the avoidance of doubt, the subject-matter of the present invention includes subject-matter as defined in the following numbered paragraphs (hereafter "para."):
1. A method for preventing infection of a subject by a microbe comprising:
   administering a compound that modulates the expression or activity of a microbial transcription factor to a subject at risk of developing an infection such that infection of the subject is prevented.
2. The method of para. 1, wherein the transcription factor is a member of the AraC-XylS family of transcription factors.
3. The method of para. 1, wherein the transcription factor is a member of the MarA family of transcription factors.
4. The method of para. 1, further comprising administering an antibiotic.
5. A method for preventing urinary tract infection of a subject by a microbe comprising: administering a compound that modulates the expression or activity of a microbial transcription factor to a subject at risk of developing a urinary tract infection such that infection of the subject is prevented.
6. A method for preventing prostatitis in a subject by a microbe comprising:
   administering a compound that modulates the expression or activity of a microbial transcription factor to a subject at risk of developing prostatitis such that infection of the subject is prevented.
7. A method for reducing virulence of a microbe comprising: administering a compound that modulates the expression or activity of a microbial transcription factor to a subject at risk of developing an infection with the microbe such that virulence of the microbe is reduced.
8. The method of para. 7, wherein the transcription factor is a member of the AraC-XylS family of transcription factors.
9. The method of para. 7, wherein the transcription factor is a member of the MarA family of transcription factors.
10. The method of para. 7, further comprising administering an antibiotic.
11. A method for treating a microbial infection in a subj ect comprising:
   administering a compound that modulates the expression or activity of a transcription factor to a subject having a microbial infection such that infection of the subject is treated.
12. The method of para. 11, wherein the transcription factor is a member of the AraC-XylS family of transcription factors.
13. The method of para. 11, wherein the transcription factor is a member of the MarA family of transcription factors.
14. The method of para. 11, further comprising administering an antibiotic.
15. A method for treating a urinary tract infection in a subject comprising:
   administering a compound that modulates the expression or activity of a transcription factor to a subject having a urinary tract infection such that infection of the subject is treated.
16. A method for treating prostatitis in a subject comprising: administering a compound that modulates the expression or activity of a transcription factor to a subject having prostatitis such that infection of the subject is treated.
17. The method of para. 15, wherein the transcription factor is a member of the AraC-XylS family of transcription factors.
18. The method of para. 15, wherein the transcription factor is a member of the MarA family of transcription factors.
19. The method of para. 15, further comprising administering an antibiotic.
20. A method for reducing virulence in a microbe comprising: administering a compound that inhibits the expression or activity of a transcription factor to a subject having a microbial infection such that virulence of the microbe is reduced.
21. The method of para. 20, wherein the transcription factor is a member of the AraC-XylS family of transcription factors.
22. The method of para. 20, wherein the transcription factor is a member of the MarA family of transcription factors.
23. The method of para. 20, further comprising administering an antibiotic.
24. A method for evaluating the effectiveness of a compound that modulates the expression or activity of a microbial transcription factor at inhibiting microbial virulence comprising: infecting a non-human animal with a microbe, wherein the ability of the microbe to establish an infection in the non-human animal requires that the microbe colonize the animal; administering the compound that modulates the expression or activity of the microbial transcription factor to the non-human animal; and determining the level of infection of the non-human animal, wherein the ability of the compound to reduce the level of infection of the animal indicates that the compound is effective at inhibiting microbial virulence.
25. The method of para. 24, wherein the transcription factor is a member of the AraC-XylS family of transcription factors.
26. The method of para. 24, wherein the transcription factor is a member of the MarA family of transcription factors.
27. The method of para. 24, further comprising administering an antibiotic.
28. The method of para. 24, wherein the level of infection of the non-human animal is determined by measuring the ability of the microbe to colonize the tissue of the non-human animal.
29. The method of para. 24, wherein the level of infection of the non-human animal is determined by enumerating the number of microbes present in the tissue of the non-human animal.
30.A method for identifying a compound for treating microbial infection, comprising: innoculating a non-human animal with a microbe, wherein the ability of the microbe to establish an infection in the non-human animal requires that the microbe colonize the animal; administering a compound which reduces the expression or activity of a microbial transcription factor to the animal, and determining the effect of the test compound on the ability of the microbe to colonize the animal, such that a compound for treating microbial infection is identified.
31. The method of para. 30, wherein the transcription factor is a member of the AraC-XylS family of transcription factors.
32. The method of para. 30, wherein the transcription factor is a member of the MarA family of transcription factors.
33. The method of para. 30, wherein the level of infection of the non-human animal is determined by measuring the ability of the microbe to colonize the tissue of the non-human animal.
34. The method of para. 30, wherein the level of infection of the non-human animal is determined by enumerating the number of microbes present in the tissue of the non-human animal.
35.A method for identifying a compound for reducing microbial virulence, comprising: inoculating a non-human animal with a microbe, wherein the ability of the microbe to establish an infection in the non-human animal requires that the microbe colonize the animal; administering a compound which reduces the expression or activity of a microbial transcription factor to the animal, and determining the effect of the test compound on the ability of the microbe to colonize the animal, such that a compound for reducing microbial virulence is identified.
36. The method of para. 35, wherein the transcription factor is a member of the AraC-XylS family of transcription factors.
37. The method of para. 35, wherein the transcription factor is a member of the MarA family of transcription factors.
38. The method of para. 35, wherein the level of infection of the non-human animal is determined by measuring the ability of the microbe to colonize the tissue of the non-human animal.
39. The method of para. 35, wherein the level of infection of the non-human animal is determined by enumerating the number of microbes present in the tissue of the non-human animal.
40.A method for identifying transcription factors which promote microbial virulence comprising: creating a microbe in which a transcription factor to be tested is misexpressed; introducing the microbe into a non-human animal; wherein the ability of the microbe to establish an infection in the non-human animal requires that the microbe colonize the animal; and determining the ability of the microbe to colonize the animal, wherein a reduced ability of the microbe to colonize the animal as compared to a wild-type microbial cell identifies the transcription factor as a transcription factor which promotes microbial virulence.
41. The method of para. 40, wherein the transcription factor is a member of the AraC-XylS family of transcription factors.
42. The method of para. 40, wherein the transcription factor is a member of the MarA family of transcription factors.
43. The method of para. 40, wherein the level of infection of the non-human animal is determined by measuring the ability of the microbe to colonize the tissue of the non-human animal.
44. The method of para. 40, wherein the level of infection of the non-human animal is determined by enumerating the number of microbes present in the tissue of the non-human animal.
45. A method for reducing the ability of a microbe to adhere to an abiotic surface comprising: contacting the abiotic surface or the microbe with a compound that modulates the activity of a transcription factor such that the ability of the microbe to adhere to the abiotic surface is reduced.
46. The method of para. 45, wherein the transcription factor is a member of the AraC-XylS family of transcription factors.
47. The method of para. 45, wherein the transcription factor is a member of the MarA family of transcription factors.
48. The method of para. 45, further comprising contacting the abiotic surface or the microbe with a second agent that is effective at controlling the growth of the microbe.
49. The method of para. 45, wherein the abiotic surface is selected from the group consisting of: stents, catheters, and prosthetic devices.
50. A pharmaceutical composition comprising a compound that modulates the activity or expression of a microbial transcription factor and a pharmaceutically acceptable carrier, wherein the compound reduces microbial virulence.
51. A pharmaceutical composition comprising a compound that modulates the activity or expression of a microbial transcription factor and an antibiotic in a pharmaceutically acceptable carrier.

## Claims

1. The use of a compound that modulates the expression or activity of a microbial transcription factor in one of the following methods:
a method for preventing or treating infection of a subject by a microbe, which method comprises administering the compound to a subject at risk of developing an infection such that infection of the subject is prevented or treated;
a method for reducing virulence of a microbe, which method comprises administering the compound to a subject at risk of developing an infection with the microbe such that virulence of the microbe is reduced;
a method for reducing virulence in a microbe, which method comprises administering the compound to a subject having a microbial infection such that virulence of the microbe is reduced.

2. The use according to claim 1, wherein the method is a method for preventing or treating infection of a subject by a microbe, and wherein the infection is urinary tract infection or prostatitis.

3. The use according to claim 1 or 2, wherein the transcription factor is a member of the AraCXylS family of transcription factors or a member of the MarA family of transcription factors.

4. The use according to claim 1, 2 or 3, further comprising administering an antibiotic.

5. A method comprising:
(a) infecting or inoculating a nonhuman animal with a microbe, wherein the ability of the microbe to establish an infection in the nonhuman animal requires that the microbe colonize the animal;
(b) administering a compound that modulates the expression or activity of the microbial transcription factor to the nonhuman animal; and
(c1) determining the level of infection of the nonhuman animal, wherein the ability of the compound to reduce the level of infection of the animal indicates that the compound is effective at inhibiting microbial virulence; or
(c2) determining the effect of the test compound on the ability of the microbe to colonize the animal, such that a compound for treating microbial infection is identified; or
(c3) determining the effect of the test compound on the ability of the microbe to colonize the animal, such that a compound for reducing microbial virulence is identified.

6. The method of claim 5, further comprising administering an antibiotic.

7. A method for identifying transcription factors which promote microbial virulence comprising: creating a microbe in which a transcription factor to be tested is misexpressed; introducing the microbe into a nonhuman animal; wherein the ability of the microbe to establish an infection in the nonhuman animal requires that the microbe colonize the animal; and determining the ability of the microbe to colonize the animal, wherein a reduced ability of the microbe to colonize the animal as compared to a wildtype microbial cell identifies the transcription factor as a transcription factor which promotes microbial virulence.

8. The method of claim 5 or 7, wherein the transcription factor is a member of the AraCXylS family of transcription factors or a member of the MarA family of transcription factors..

9. The method of claim 5 or 7, wherein the level of infection of the nonhuman animal is determined by measuring the ability of the microbe to colonize the tissue of the nonhuman animal or by enumerating the number of microbes present in the tissue of the nonhuman animal.

10. A method for reducing the ability of a microbe to adhere to an abiotic surface comprising: contacting the abiotic surface or the microbe with a compound that modulates the activity of a transcription factor such that the ability of the microbe to adhere to the abiotic surface is reduced.

11. The method of claim 10, wherein the transcription factor is a member of the AraCXylS family of transcription factors or a member of the MarA family of transcription factors.

12. The method of claim 10, further comprising contacting the abiotic surface or the microbe with a second agent that is effective at controlling the growth of the microbe.

13. A pharmaceutical composition comprising:
a compound that modulates the activity or expression of a microbial transcription factor and a pharmaceutically acceptable carrier, wherein the compound reduces microbial virulence; or
a compound that modulates the activity or expression of a microbial transcription factor and an antibiotic in a pharmaceutically acceptable carrier.


SEQUENCE LISTING

<110> PARATEK PHARMACEUTICALS, INC.

<120> METHODS FOR PREVENTING AND TREATING MICROBIAL
      INFECTIONS BY MODULATING TRANSCRIPTION FACTORS

<130> PAZ-190PC

<140>
<141>

<150> 60/391345
<151> 2002-06-24

<150> 60/421218
<151> 2002-10-25

<150> 60/429142
<151> 2002-11-26

<150> 60/458935
<151> 2003-03-31

<160> 4

<170> FastSEQ for Windows Version 4.0

<210> 1
<211> 7878
<212> DNA
<213> Echerichia coli

<220>
<221> CDS
<222> (4124)...(4843)

<400> 1
gttaactgtg gtggttgtca ccgcccatta cacggcatac agctatatcg agccttttgt 60
acaaaacatt gcgggattca gcgccaactt tgccacggca ttactgttat tactcggtgg 120
tgcgggcatt attggcagcg tgattttcgg taaactgggt aatcagtatg cgtctgcgtt 180
ggtgagtacg gcgattgcgc tgttgctggt gtgcctggca ttgctgttac ctgcggcgaa 240
cagtgaaata cacctcgggg tgctgagtat tttctggggg atcgcgatga tgatcatcgg 300
gcttggtatg caggttaaag tgctggcgct ggcaccagat gctaccgacg tcgcgatggc 360
gctattctcc ggcatattta atattggaat cggggcgggt gcgttggtag gtaatcaggt 420
gagtttgcac tggtcaatgt cgatgattgg ttatgtgggc gcggtgcctg cttttgccgc 480
gttaatttgg tcaatcatta tatttcgccg ctggccagtg acactcgaag aacagacgca 540
atagttgaaa ggcccattcg ggcctttttt aatggtacgt tttaatgatt tccaggatgc 600
cgttaataat aaactgcaca cccatacata ccagcaggaa tcccatcaga cgggagatcg 660
cttcaatgcc acccttgccc accagccgca taattgcgcc ggagctgcgt aggcttcccc 720
acaaaataac cgccaccagg aaaaagatca gcggcggcgc aaccatcagt acccaatcag 780
cgaaggttga actctgacgc actgtggacg ccgagctaat aatcatcgct atggttcccg 840
gaccggcagt acttggcatt gccagcggca caaaggcaat attggcactg ggttcatctt 900
ccagctcttc cgacttgctt ttcgcctccg gtgaatcaat cgctttctgt tgcggaaaga 960
gcatccgaaa accgataaac gcgacgatta agccgcctgc aattcgcaga ccgggaatcg 1020
aaatgccaaa tgtatccatc accagttgcc cggcgtaata cgccaccatc atgatggcaa 1080

atacgtacac cgaggccatc aacgactgac gattacgttc ggcactgttc atgttgcctg 1140
ccaggccaag aaataacgcg acagttgtta atgggttagc taacggcagc aacaccacca 1200
gccccaggcc aattgcttta aacaaatcta acattggtgg ttgttatcct gtgtatctgg 1260
gttatcagcg aaaagtataa ggggtaaaca aggataaagt gtcactcttt agctagcctt 1320

gcatcgcatt gaacaaaact tgaaccgatt tagcaaaacg tggcatcggt caattcattc 1380
atttgactta tacttgcctg ggcaatatta tcccctgcaa ctaattactt gccagggcaa 1440
ctaatgtgaa aagtaccagc gatctgttca atgaaattat tccattgggt cgcttaatcc 1500
atatggttaa tcagaagaaa gatcgcctgc ttaacgagta tctgtctccg ctggatatta 1560
ccgcggcaca gtttaaggtg ctctgctcta tccgctgcgc ggcgtgtatt actccggttg 1620
aactgaaaaa ggtattgtcg gtcgacctgg gagcactgac ccgtatgctg gatcgcctgg 1680
tctgtaaagg ctgggtggaa aggttgccga acccgaatga caagcgcggc gtactggtaa 1740
aacttaccac cggcggcgcg gcaatatgtg aacaatgcca tcaattagtt ggccaggacc 1800
tgcaccaaga attaacaaaa aacctgacgg cggacgaagt ggcaacactt gagtatttgc 1860
ttaagaaagt cctgccgtaa acaaaaaaga ggtatgacga tgtccagacg caatactgac 1920
gctattacca ttcatagcat tttggactgg atcgaggaca acctggaatc gccactgtca 1980
ctggagaaag tgtcagagcg ttcgggttac tccaaatggc acctgcaacg gatgtttaaa 2040
aaagaaaccg gtcattcatt aggccaatac atccgcagcc gtaagatgac ggaaatcgcg 2100
caaaagctga aggaaagtaa cgagccgata ctctatctgg cagaacgata tggcttcgag 2160
tcgcaacaaa ctctgacccg aaccttcaaa aattactttg atgttccgcc gcataaatac 2220
cggatgacca atatgcaggg cgaatcgcgc tttttacatc cattaaatca ttacaacagc 2280
tagttgaaaa cgtgacaacg tcactgaggc aatcatgaaa ccactttcat ccgcaatagc 2340
agctgcgctt attctctttt ccgcgcaggg cgttgcggaa caaaccacgc agccagttgt 2400
tacttcttgt gccaatgtcg tggttgttcc cccatcgcag gaacacccac cgtttgattt 2460
aaatcacatg ggtactggca gtgataagtc ggatgcgctc ggcgtgccct attataatca 2520
acacgctatg tagtttgttc tggccccgac atctcggggc ttattaactt cccaccttta 2580
ccgctttacg ccaccgcaag ccaaatacat tgatatacag cccggtcata atgagcaccg 2640
cacctaaaaa ttgcagaccc gttaagcgtt catccaacaa tagtgccgca cttgccagtc 2700
ctactacggg caccagtaac gataacggtg caacccgcca ggtttcatag cgtcccagta 2760
acgtccccca gatcccataa ccaacaattg tcgccacaaa cgccagatac atcagagaca 2820
agatggtggt catatcgata gtaaccagac tgtgaatcat ggttgcggaa ccatcgagaa 2880
tcagcgaggc aacaaagaag ggaatgattg ggattaaagc gctccagatt accagcgaca 2940
tcaccgccgg acgcgttgag tgcgacatga tctttttatt gaagatgttg ccacacgccc 3000
aactaaatgc tgccgccagg gtcaacataa agccgagcat cgccacatgc tgaccgttca 3060
gactatcttc gattaacacc agtacgccaa aaatcgctaa ggcgatcccc gccaattgtt 3120
tgccatgcag tcgctccccg aaagtaaacg cgccaagcat gatagtaaaa aacgcctgtg 3180
cctgtaacac cagcgaagcc agtccagcag gcataccgaa gttaatggca caaaaaagaa 3240
aagcaaactg cgcaaaactg atggttaatc cataccccag cagcaaattc agtggtactt 3300
tcggtcgtgc gacaaaaaag atagccggaa aagcgaccag cataaagcgc aaaccggcca 3360
gcatcagcgt ggcatgttat gaagccccac tttgatgacc acaaaattta gcccccatac 3420
gaccactacc agtagcgcca acaccccatc ttttcgcgac attctaccgc ctctgaattt 3480
catcttttgt aagcaatcaa cttagctgaa tttacttttc tttaacagtt gattcgttag 3540
tcgccggtta cgacggcatt aatgcgcaaa taagtcgcta tacttcggat ttttgccatg 3600
ctatttcttt acatctctaa aacaaaacat aacgaaacgc actgccggac agacaaatga 3660
acttatccct acgacgctct accagcgccc ttcttgcctc gtcgttgtta ttaaccatcg 3720
gacgcggcgc taccgtgcca tttatgacca tttacttgag tcgccagtac agcctgagtg 3780
tcgatctaat cggttatgcg atgacaattg cgctcactat tggcgtcgtt tttagcctcg 3840
gttttggtat cctggcggat aagttcgaca agaaacgcta tatgttactg gcaattaccg 3900
ccttcgccag cggttttatt gccattactt tagtgaataa cgtgacgctg gttgtgctct 3960
tttttgccct cattaactgc gcctattctg tttttgctac cgtgctgaaa gcctggtttg 4020
ccgacaatct ttcgtccacc agcaaaacga aaatcttctc aatcaactac accatgctaa 4080
acattggctg accatcggtc cgccgctcgg cacgctgttg gta atg cag agc atc 4135
Met Gln Ser Ile
1

aat ctg ccc ttc tgg ctg gca gct atc tgt tcc gcg ttt ccc atg ctt 4183
Asn Leu Pro Phe Trp Leu Ala Ala Ile Cys Ser Ala Phe Pro Met Leu
5                   10                  15                  20

ttc att caa att tgg gta aag cgc agc gag aaa atc atc gcc acg gaa 4231
Phe Ile Gln Ile Trp Val Lys Arg Ser Glu Lys Ile Ile Ala Thr Glu
                25                  30                  35

aca ggc agt gtc tgg tcg ccg aaa gtt tta tta caa gat aaa gca ctg 4279
Thr Gly Ser Val Trp Ser Pro Lys Val Leu Leu Gln Asp Lys Ala Leu
            40                  45                  50

ttg tgg ttt acc tgc tct ggt ttt ctg gct tct ttt gta agc ggc gca 4327
Leu Trp Phe Thr Cys Ser Gly Phe Leu Ala Ser Phe Val Ser Gly Ala
        55                  60                  65

ttt gct tca tgc att tca caa tat gtg atg gtg att gct gat ggg gat 4375
Phe Ala Ser Cys Ile Ser Gln Tyr Val Met Val Ile Ala Asp Gly Asp
    70                  75                  80

ttt gcc gaa aag gtg gtc gcg gtt gtt ctt ccg gtg aat gct gcc atg 4423
Phe Ala Glu Lys Val Val Ala Val Val Leu Pro Val Asn Ala Ala Met
85                  90                  95                  100

gtg gtt acg ttg caa tat tcc gtg ggc cgc cga ctt aac ccg gct aac 4471
Val Val Thr Leu Gln Tyr Ser Val Gly Arg Arg Leu Asn Pro Ala Asn
                105                 110                 115

atc cgc gcg ctg atg aca gca ggc acc ctc tgt ttc gtc atc ggt ctg 4519
Ile Arg Ala Leu Met Thr Ala Gly Thr Leu Cys Phe Val Ile Gly Leu
            120                 125                 130

gtc ggt ttt att ttt tcc ggc aac agc ctg cta ttg tgg ggt atg tca 4567
Val Gly Phe Ile Phe Ser Gly Asn Ser Leu Leu Leu Trp Gly Met Ser
        135                 140                 145

gct gcg gta ttt act gtc ggt gaa atc att tat gcg ccg ggc gag tat 4615
Ala Ala Val Phe Thr Val Gly Glu Ile Ile Tyr Ala Pro Gly Glu Tyr
    150                 155                 160

atg ttg att gac cat att gcg ccg cca gaa atg aaa gcc agc tat ttt 4663
Met Leu Ile Asp His Ile Ala Pro Pro Glu Met Lys Ala Ser Tyr Phe
165                 170                 175                 180

tcc gcc cag tct tta ggc tgg ctt ggt gcc gcg att aac cca tta gtg 4711
Ser Ala Gln Ser Leu Gly Trp Leu Gly Ala Ala Ile Asn Pro Leu Val
                185                 190                 195

agt ggc gta gtg cta acc agc ctg ccg cct tcc tcg ctg ttt gtc atc 4759
Ser Gly Val Val Leu Thr Ser Leu Pro Pro Ser Ser Leu Phe Val Ile
            200                 205                 210

tta gcg ttg gtg atc att gct gcg tgg gtg ctg atg tta aaa ggg att 4807
Leu Ala Leu Val Ile Ile Ala Ala Trp Val Leu Met Leu Lys Gly Ile
        215                 220                 225

cga gca aga ccg tgg ggg cag ccc gcg ctt tgt tga tttaagtcga 4853
Arg Ala Arg Pro Trp Gly Gln Pro Ala Leu Cys *
230 235

acacaataaa gatttaattc agccttcgtt taggttacct ctgctaatat ctttctcatt 4913
gagatgaaaa ttaaggtaag cgaggaaaca caccacacca taaacggagg caaataatgc 4973
tgggtaatat gaatgttttt atggccgtac tgggaataat ttatttttct ggttttctgg 5033
ccgcgtattt cagccacaaa tgggatgact aatgaacgga gataatccct cacctaaccg 5093
gccccttgtt acagttgtgt acaaggggcc tgatttttat gacggcgaaa aaaaaccgcc 5153
agtaaaccgg cggtgaatgc ttgcatggat agatttgtgt tttgctttta cgctaacagg 5213
cattttcctg cactgataac gaatcgttga cacagtagca tcagttttct caatgaatgt 5273
taaacggagc ttaaactcgg ttaatcacat tttgttcgtc aataaacatg cagcgatttc 5333

ttccggtttg cttaccctca tacattgccc ggtccgctct tccaatgacc acatccagag 5393
gctcttcagg aaatgcgcga ctcacacctg ctgtcacggt aatgttgata tgcccttcag 5453
aatgtgtgat ggcatggtta tcgactaact ggcaaattct gacacctgca cgacatgctt 5513
cttcatcatt agccgctttg acaataatga taaattcttc gcccccgtag cgataaaccg 5573
tttcgtaatc acgcgtccaa ctggctaagt aagttgccag ggtgcgtaat actacatcgc 5633
cgattaaatg cccgtagtat cattaaccaa tttaaatcgg tcaatatcca acaacattaa 5693
ataaagattc agaggctcag cgttgcgtaa ctgatgatca aaggattcat caagaacccg 5753
acgacccggc aatcccgtca aaacatccat attgctacgg atcgtcagca aataaatttt 5813
gtaatcggtt aatgccgcag taaaagaaag caacccctcc tgaaaggcgt cgaaatgcgc 5873
gtcctgccag tgattttcaa caatagccag cattaattcc cgaccacagt tatgcatatg 5933
ttgatgggca gaatccatta gccgaacgta aggtaattca tcgttatcga gtggccccag 5993
atgatcaatc caccgaccaa actggcacag tccataagaa tggttatccg ttatttctgg 6053
cttactggca tctctcgcga ccacgctgtg aaacatactc accagccact ggtagtgggc 6113
atcgatagcc ttattgagat ttaacaagat ggcatcaatt tccgttgtct tcttgatcat 6173
tgccactcct ttttcacagt tccttgtgcg cgctattcta acgagagaaa agcaaattta 6233
cgtcaatatt ttcatagaaa tccgaagtta tgagtcatct ctgagataac attgtgattt 6293
aaaacaaaat cagcggataa aaaagtgttt aattctgtaa attacctctg cattatcgta 6353
aataaaagga tgacaaatag cataacccaa taccctaatg gcccagtagt tcaggccatc 6413
aggctaattt atttttattt ctgcaaatga gtgacccgaa cgacggccgg cgcgcttttc 6473
ttatccagac tgccactaat gttgatcatc tggtccggct gaacttctcg tccatcaaag 6533
acggccgcag gaataacgac attaatttca ccgctcttat cgcgaaaaac gtaacggtcc 6593
tctcctttgt gagaaatcaa attaccgcgt agtgaaaccg aagcgccatc gtgcatggtt 6653
tttgcgaaat caacggtcat tttttttgca tcatcggttc cgcgatagcc atcttctatt 6713
gcatgaggcg gcggtggcgc tgcatcctgt tttaaaccgc cctggtcatc tgccaacgca 6773
taaggcatga caagaaaact tgctaataca atggcctgaa atttcatact aactccttaa 6833
ttgcgtttgg tttgacttat taagtctggt tgctattttt ataattgcca aataagaata 6893
ttgccaattg ttataaggca tttaaaatca gccaactagc tgtcaaatat acagagaatt 6953
taactcacta aagttaagaa gattgaaaag tcttaaacat attttcagaa taatcggatt 7013
tatatgtttg aaaattatta tattggacga gcatacagaa aaagcaaatc acctttacat 7073
ataaaagcgt ggacaaaaaa cagtgaacat taatagagat aaaattgtac aacttgtaga 7133
taccgatact attgaaaacc tgacatccgc gttgagtcaa agacttatcg cggatcaatt 7193
acgcttaact accgccgaat catgcaccgg cggtaagttg gctagcgccc tgtgtgcagc 7253
tgaagataca cccaaatttt acggtgcagg ctttgttact ttcaccgatc aggcaaagat 7313
gaaaatcctc agcgtaagcc agcaatctct tgaacgatat tctgcggtga gtgagaaagt 7373
ggcagcagaa atggcaaccg gtgccataga gcgtgcggat gctgatgtca gtattgccat 7433
taccggctac ggcggaccgg agggcggtga agatggtacg ccagcgggta ccgtctggtt 7493
tgcgtggcat attaaaggcc agaactacac tgcggttatg cattttgctg gcgactgcga 7553
aacggtatta gctttagcgg tgaggtttgc cctcgcccag ctgctgcaat tactgctata 7613
accaggctgg cctggcgata tctcaggcca gccattggtg gtgtttatat gttcaagcca 7673
cgatgttgca gcatcggcat aatcttaggt gccttaccgc gccattgtcg atacaggcgt 7733
tccagatctt cgctgttacc tctggaaagg atcgcctcgc gaaaacgcag cccattttca 7793
cgcgttaatc cgccctgctc aacaaaccac tgataaccat catcggccaa catttgcgtc 7853
cacagataag cgtaataacc tgcag 7878

<210> 2
<211> 239
<212> PRT
<213> Echerichia coli

<400> 2

Met Gln Ser Ile Asn Leu Pro Phe Trp Leu Ala Ala Ile Cys Ser Ala
1               5                   10                  15
Phe Pro Met Leu Phe Ile Gln Ile Trp Val Lys Arg Ser Glu Lys Ile
            20                  25                  30
Ile Ala Thr Glu Thr Gly Ser Val Trp Ser Pro Lys Val Leu Leu Gln
        35                  40                  45
Asp Lys Ala Leu Leu Trp Phe Thr Cys Ser Gly Phe Leu Ala Ser Phe
    50                  55                  60

Val Ser Gly Ala Phe Ala Ser Cys Ile Ser Gln Tyr Val Met Val Ile
65                  70                  75                  80
Ala Asp Gly Asp Phe Ala Glu Lys Val Val Ala Val Val Leu Pro Val
                85                  90                  95
Asn Ala Ala Met Val Val Thr Leu Gln Tyr Ser Val Gly Arg Arg Leu
            100                 105                 110
Asn Pro Ala Asn Ile Arg Ala Leu Met Thr Ala Gly Thr Leu Cys Phe
        115                 120                 125
Val Ile Gly Leu Val Gly Phe Ile Phe Ser Gly Asn Ser Leu Leu Leu
    130                 135                 140
Trp Gly Met Ser Ala Ala Val Phe Thr Val Gly Glu Ile Ile Tyr Ala
145                 150                 155                 160
Pro Gly Glu Tyr Met Leu Ile Asp His Ile Ala Pro Pro Glu Met Lys
                165                 170                 175
Ala Ser Tyr Phe Ser Ala Gln Ser Leu Gly Trp Leu Gly Ala Ala Ile
            180                 185                 190
Asn Pro Leu Val Ser Gly Val Val Leu Thr Ser Leu Pro Pro Ser Ser
        195                 200                 205
Leu Phe Val Ile Leu Ala Leu Val Ile Ile Ala Ala Trp Val Leu Met
    210                 215                 220
Leu Lys Gly Ile Arg Ala Arg Pro Trp Gly Gln Pro Ala Leu Cys
225                 230                 235

<210> 3
<211> 870
<212> DNA
<213> Echerichia coli

<220>
<221> CDS

<222> (1)...(870)

<400> 3
atg gat cag gcc ggc att att cgc gac ctt tta atc tgg ctg gaa ggt     48
Met Asp Gln Ala Gly Ile Ile Arg Asp Leu Leu Ile Trp Leu Glu Gly
 1               5                  10                  15

cat ctg gat cag ccc ctg tcg ctc gac aat gta gcg gcg aaa gca ggt     96
His Leu Asp Gln Pro Leu Ser Leu Asp Asn Val Ala Ala Lys Ala Gly
            20                  25                  30

tat tcc aag tgg cac tta cag aga atg ttt aaa gat gtc act ggc cat    144
Tyr Ser Lys Trp His Leu Gln Arg Met Phe Lys Asp Val Thr Gly His
        35                  40                  45

gct att ggc gcg tat att cgt gct cgt cgt ttg tcg aaa tcg gcg gtc    192
Ala Ile Gly Ala Tyr Ile Arg Ala Arg Arg Leu Ser Lys Ser Ala Val
    50                  55                  60

gca cta cgc ctg act gcg cgt ccg att ctg gac atc gcg ctg caa tac    240
Ala Leu Arg Leu Thr Ala Arg Pro Ile Leu Asp Ile Ala Leu Gln Tyr
65                  70                  75                  80

cgc ttc gac tct caa cag aca ttt acc cgc gca ttc aag aag cag ttt    288
Arg Phe Asp Ser Gln Gln Thr Phe Thr Arg Ala Phe Lys Lys Gln Phe
                85                  90                  95

gcc cag act cct gca ctt tac cgc cgt tct cct gaa tgg agc gcc ttt     336
Ala Gln Thr Pro Ala Leu Tyr Arg Arg Ser Pro Glu Trp Ser Ala Phe
            100                 105                 110

ggt att cgc ccg ccg cta cgc ctg ggt gaa ttc act atg cca gag cac     384
Gly Ile Arg Pro Pro Leu Arg Leu Gly Glu Phe Thr Met Pro Glu His
        115                 120                 125

aaa ttt gtc acc ctg gaa gat acg ccg ctg att ggt gtt acc cag agc     432
Lys Phe Val Thr Leu Glu Asp Thr Pro Leu Ile Gly Val Thr Gln Ser
    130                 135                 140

tac tcc tgt tcg ctg gag caa atc tct gat ttc cgc cat gaa atg cgt     480
Tyr Ser Cys Ser Leu Glu Gln Ile Ser Asp Phe Arg His Glu Met Arg
145                 150                 155                 160

tat cag ttc tgg cac gat ttt ctc ggc aac gcg ccg acc att ccg ccg     528
Tyr Gln Phe Trp His Asp Phe Leu Gly Asn Ala Pro Thr Ile Pro Pro
                165                 170                 175

gtg ctc tac ggc ctg aat gaa acg cgt ccg agt cag gat aaa gac gac     576
Val Leu Tyr Gly Leu Asn Glu Thr Arg Pro Ser Gln Asp Lys Asp Asp
            180                 185                 190

gag caa gag gta ttc tat acc acc gcg tta gcc cag gat cag gca gat     624
Glu Gln Glu Val Phe Tyr Thr Thr Ala Leu Ala Gln Asp Gln Ala Asp
        195                 200                 205

ggc tat gta ctg acg ggg cat ccg gtg atg ctg cag ggc ggc gaa tat     672
Gly Tyr Val Leu Thr Gly His Pro Val Met Leu Gln Gly Gly Glu Tyr
    210                 215                 220

gtg atg ttt acc tat gaa ggt ctg gga acc ggc gtg cag gag ttt atc     720
Val Met Phe Thr Tyr Glu Gly Leu Gly Thr Gly Val Gln Glu Phe Ile
225                 230                 235                 240

ctg acg gta tac gga acg tgc atg cca atg ctc aac ctg acg cgc cgt     768
Leu Thr Val Tyr Gly Thr Cys Met Pro Met Leu Asn Leu Thr Arg Arg
                245                 250                 255

aaa ggt cag gat att gag cga tac tac ccg gca gaa gat gcc aaa gcg     816
Lys Gly Gln Asp Ile Glu Arg Tyr Tyr Pro Ala Glu Asp Ala Lys Ala
            260                 265                 270

gga gat cgc cca att aat cta cgc tgt gaa ctg ctg att ccg atc cgt     864
Gly Asp Arg Pro Ile Asn Leu Arg Cys Glu Leu Leu Ile Pro Ile Arg
        275                 280                 285

cgt taa                                                             870
Arg  *

<210> 4
<211> 289
<212> PRT
<213> Echerichia coli

<400> 4

Met Asp Gln Ala Gly Ile Ile Arg Asp Leu Leu Ile Trp Leu Glu Gly
1               5                   10                  15
His Leu Asp Gln Pro Leu Ser Leu Asp Asn Val Ala Ala Lys Ala Gly
            20                  25                  30
Tyr Ser Lys Trp His Leu Gln Arg Met Phe Lys Asp Val Thr Gly His
        35                  40                  45
Ala Ile Gly Ala Tyr Ile Arg Ala Arg Arg Leu Ser Lys Ser Ala Val
    50                  55                  60
Ala Leu Arg Leu Thr Ala Arg Pro Ile Leu Asp Ile Ala Leu Gln Tyr
65                  70                  75                  80
Arg Phe Asp Ser Gln Gln Thr Phe Thr Arg Ala Phe Lys Lys Gln Phe
                85                  90                  95
Ala Gln Thr Pro Ala Leu Tyr Arg Arg Ser Pro Glu Trp Ser Ala Phe
            100                 105                 110
Gly Ile Arg Pro Pro Leu Arg Leu Gly Glu Phe Thr Met Pro Glu His
        115                 120                 125
Lys Phe Val Thr Leu Glu Asp Thr Pro Leu Ile Gly Val Thr Gln Ser
    130                 135                 140
Tyr Ser Cys Ser Leu Glu Gln Ile Ser Asp Phe Arg His Glu Met Arg
145                 150                 155                 160
Tyr Gln Phe Trp His Asp Phe Leu Gly Asn Ala Pro Thr Ile Pro Pro
                165                 170                 175
Val Leu Tyr Gly Leu Asn Glu Thr Arg Pro Ser Gln Asp Lys Asp Asp
            180                 185                 190
Glu Gln Glu Val Phe Tyr Thr Thr Ala Leu Ala Gln Asp Gln Ala Asp
        195                 200                 205
Gly Tyr Val Leu Thr Gly His Pro Val Met Leu Gln Gly Gly Glu Tyr
    210                 215                 220
Val Met Phe Thr Tyr Glu Gly Leu Gly Thr Gly Val Gln Glu Phe Ile
225                 230                 235                 240
Leu Thr Val Tyr Gly Thr Cys Met Pro Met Leu Asn Leu Thr Arg Arg
                245                 250                 255
Lys Gly Gln Asp Ile Glu Arg Tyr Tyr Pro Ala Glu Asp Ala Lys Ala
            260                 265                 270

Gly Asp Arg Pro Ile Asn Leu Arg Cys Glu Leu Leu Ile Pro Ile Arg
        275                 280                 285
Arg

SEQUENCE LISTING

<110> PARATEK PHARMACEUTICALS, INC.

<120> METHODS FOR PREVENTING AND TREATING MICROBIAL
      INFECTIONS BY MODULATING TRANSCRIPTION FACTORS

<130> PAZ-190PC

<140>
<141>

<150> 60/391345
<151> 2002-06-24

<150> 60/421218
<151> 2002-10-25

<150> 60/429142
<151> 2002-11-26

<150> 60/458935
<151> 2003-03-31

<160> 4

<170> FastSEQ for Windows Version 4.0

<210> 1
<211> 7878
<212> DNA
<213> Echerichia coli

<220>
<221> CDS
<222> (4124)...(4843)

<400> 1
gttaactgtg gtggttgtca ccgcccatta cacggcatac agctatatcg agccttttgt 60
acaaaacatt gcgggattca gcgccaactt tgccacggca ttactgttat tactcggtgg 120
tgcgggcatt attggcagcg tgattttcgg taaactgggt aatcagtatg cgtctgcgtt 180
ggtgagtacg gcgattgcgc tgttgctggt gtgcctggca ttgctgttac ctgcggcgaa 240
cagtgaaata cacctcgggg tgctgagtat tttctggggg atcgcgatga tgatcatcgg 300
gcttggtatg caggttaaag tgctggcgct ggcaccagat gctaccgacg tcgcgatggc 360
gctattctcc ggcatattta atattggaat cggggcgggt gcgttggtag gtaatcaggt 420
gagtttgcac tggtcaatgt cgatgattgg ttatgtgggc gcggtgcctg cttttgccgc 480
gttaatttgg tcaatcatta tatttcgccg ctggccagtg acactcgaag aacagacgca 540
atagttgaaa ggcccattcg ggcctttttt aatggtacgt tttaatgatt tccaggatgc 600
cgttaataat aaactgcaca cccatacata ccagcaggaa tcccatcaga cgggagatcg 660
cttcaatgcc acccttgccc accagccgca taattgcgcc ggagctgcgt aggcttcccc 720
acaaaataac cgccaccagg aaaaagatca gcggcggcgc aaccatcagt acccaatcag 780
cgaaggttga actctgacgc actgtggacg ccgagctaat aatcatcgct atggttcccg 840
gaccggcagt acttggcatt gccagcggca caaaggcaat attggcactg ggttcatctt 900
ccagctcttc cgacttgctt ttcgcctccg gtgaatcaat cgctttctgt tgcggaaaga 960
gcatccgaaa accgataaac gcgacgatta agccgcctgc aattcgcaga ccgggaatcg 1020
aaatgccaaa tgtatccatc accagttgcc cggcgtaata cgccaccatc atgatggcaa 1080

atacgtacac cgaggccatc aacgactgac gattacgttc ggcactgttc atgttgcctg 1140
ccaggccaag aaataacgcg acagttgtta atgggttagc taacggcagc aacaccacca 1200
gccccaggcc aattgcttta aacaaatcta acattggtgg ttgttatcct gtgtatctgg 1260
gttatcagcg aaaagtataa ggggtaaaca aggataaagt gtcactcttt agctagcctt 1320

gcatcgcatt gaacaaaact tgaaccgatt tagcaaaacg tggcatcggt caattcattc 1380
atttgactta tacttgcctg ggcaatatta tcccctgcaa ctaattactt gccagggcaa 1440
ctaatgtgaa aagtaccagc gatctgttca atgaaattat tccattgggt cgcttaatcc 1500
atatggttaa tcagaagaaa gatcgcctgc ttaacgagta tctgtctccg ctggatatta 1560
ccgcggcaca gtttaaggtg ctctgctcta tccgctgcgc ggcgtgtatt actccggttg 1620
aactgaaaaa ggtattgtcg gtcgacctgg gagcactgac ccgtatgctg gatcgcctgg 1680
tctgtaaagg ctgggtggaa aggttgccga acccgaatga caagcgcggc gtactggtaa 1740
aacttaccac cggcggcgcg gcaatatgtg aacaatgcca tcaattagtt ggccaggacc 1800
tgcaccaaga attaacaaaa aacctgacgg cggacgaagt ggcaacactt gagtatttgc 1860
ttaagaaagt cctgccgtaa acaaaaaaga ggtatgacga tgtccagacg caatactgac 1920
gctattacca ttcatagcat tttggactgg atcgaggaca acctggaatc gccactgtca 1980
ctggagaaag tgtcagagcg ttcgggttac tccaaatggc acctgcaacg gatgtttaaa 2040
aaagaaaccg gtcattcatt aggccaatac atccgcagcc gtaagatgac ggaaatcgcg 2100
caaaagctga aggaaagtaa cgagccgata ctctatctgg cagaacgata tggcttcgag 2160
tcgcaacaaa ctctgacccg aaccttcaaa aattactttg atgttccgcc gcataaatac 2220
cggatgacca atatgcaggg cgaatcgcgc tttttacatc cattaaatca ttacaacagc 2280
tagttgaaaa cgtgacaacg tcactgaggc aatcatgaaa ccactttcat ccgcaatagc 2340
agctgcgctt attctctttt ccgcgcaggg cgttgcggaa caaaccacgc agccagttgt 2400
tacttcttgt gccaatgtcg tggttgttcc cccatcgcag gaacacccac cgtttgattt 2460
aaatcacatg ggtactggca gtgataagtc ggatgcgctc ggcgtgccct attataatca 2520
acacgctatg tagtttgttc tggccccgac atctcggggc ttattaactt cccaccttta 2580
ccgctttacg ccaccgcaag ccaaatacat tgatatacag cccggtcata atgagcaccg 2640
cacctaaaaa ttgcagaccc gttaagcgtt catccaacaa tagtgccgca cttgccagtc 2700
ctactacggg caccagtaac gataacggtg caacccgcca ggtttcatag cgtcccagta 2760
acgtccccca gatcccataa ccaacaattg tcgccacaaa cgccagatac atcagagaca 2820
agatggtggt catatcgata gtaaccagac tgtgaatcat ggttgcggaa ccatcgagaa 2880
tcagcgaggc aacaaagaag ggaatgattg ggattaaagc gctccagatt accagcgaca 2940
tcaccgccgg acgcgttgag tgcgacatga tctttttatt gaagatgttg ccacacgccc 3000
aactaaatgc tgccgccagg gtcaacataa agccgagcat cgccacatgc tgaccgttca 3060
gactatcttc gattaacacc agtacgccaa aaatcgctaa ggcgatcccc gccaattgtt 3120
tgccatgcag tcgctccccg aaagtaaacg cgccaagcat gatagtaaaa aacgcctgtg 3180
cctgtaacac cagcgaagcc agtccagcag gcataccgaa gttaatggca caaaaaagaa 3240
aagcaaactg cgcaaaactg atggttaatc cataccccag cagcaaattc agtggtactt 3300
tcggtcgtgc gacaaaaaag atagccggaa aagcgaccag cataaagcgc aaaccggcca 3360
gcatcagcgt ggcatgttat gaagccccac tttgatgacc acaaaattta gcccccatac 3420
gaccactacc agtagcgcca acaccccatc ttttcgcgac attctaccgc ctctgaattt 3480
catcttttgt aagcaatcaa cttagctgaa tttacttttc tttaacagtt gattcgttag 3540
tcgccggtta cgacggcatt aatgcgcaaa taagtcgcta tacttcggat ttttgccatg 3600
ctatttcttt acatctctaa aacaaaacat aacgaaacgc actgccggac agacaaatga 3660
acttatccct acgacgctct accagcgccc ttcttgcctc gtcgttgtta ttaaccatcg 3720
gacgcggcgc taccgtgcca tttatgacca tttacttgag tcgccagtac agcctgagtg 3780
tcgatctaat cggttatgcg atgacaattg cgctcactat tggcgtcgtt tttagcctcg 3840
gttttggtat cctggcggat aagttcgaca agaaacgcta tatgttactg gcaattaccg 3900
ccttcgccag cggttttatt gccattactt tagtgaataa cgtgacgctg gttgtgctct 3960
tttttgccct cattaactgc gcctattctg tttttgctac cgtgctgaaa gcctggtttg 4020
ccgacaatct ttcgtccacc agcaaaacga aaatcttctc aatcaactac accatgctaa 4080
acattggctg accatcggtc cgccgctcgg cacgctgttg gta atg cag agc atc 4135
Met Gln Ser Ile
1

aat ctg ccc ttc tgg ctg gca gct atc tgt tcc gcg ttt ccc atg ctt 4183
Asn Leu Pro Phe Trp Leu Ala Ala Ile Cys Ser Ala Phe Pro Met Leu
5                   10                  15                  20

ttc att caa att tgg gta aag cgc agc gag aaa atc atc gcc acg gaa 4231
Phe Ile Gln Ile Trp Val Lys Arg Ser Glu Lys Ile Ile Ala Thr Glu
                25                  30                  35

aca ggc agt gtc tgg tcg ccg aaa gtt tta tta caa gat aaa gca ctg 4279
Thr Gly Ser Val Trp Ser Pro Lys Val Leu Leu Gln Asp Lys Ala Leu
            40                  45                  50

ttg tgg ttt acc tgc tct ggt ttt ctg gct tct ttt gta agc ggc gca 4327
Leu Trp Phe Thr Cys Ser Gly Phe Leu Ala Ser Phe Val Ser Gly Ala
        55                  60                  65

ttt gct tca tgc att tca caa tat gtg atg gtg att gct gat ggg gat 4375
Phe Ala Ser Cys Ile Ser Gln Tyr Val Met Val Ile Ala Asp Gly Asp
    70                  75                  80

ttt gcc gaa aag gtg gtc gcg gtt gtt ctt ccg gtg aat gct gcc atg 4423
Phe Ala Glu Lys Val Val Ala Val Val Leu Pro Val Asn Ala Ala Met
85                  90                  95                  100

gtg gtt acg ttg caa tat tcc gtg ggc cgc cga ctt aac ccg gct aac 4471
Val Val Thr Leu Gln Tyr Ser Val Gly Arg Arg Leu Asn Pro Ala Asn
                105                 110                 115

atc cgc gcg ctg atg aca gca ggc acc ctc tgt ttc gtc atc ggt ctg 4519
Ile Arg Ala Leu Met Thr Ala Gly Thr Leu Cys Phe Val Ile Gly Leu
            120                 125                 130

gtc ggt ttt att ttt tcc ggc aac agc ctg cta ttg tgg ggt atg tca 4567
Val Gly Phe Ile Phe Ser Gly Asn Ser Leu Leu Leu Trp Gly Met Ser
        135                 140                 145

gct gcg gta ttt act gtc ggt gaa atc att tat gcg ccg ggc gag tat 4615
Ala Ala Val Phe Thr Val Gly Glu Ile Ile Tyr Ala Pro Gly Glu Tyr
    150                 155                 160

atg ttg att gac cat att gcg ccg cca gaa atg aaa gcc agc tat ttt 4663
Met Leu Ile Asp His Ile Ala Pro Pro Glu Met Lys Ala Ser Tyr Phe
165                 170                 175                 180

tcc gcc cag tct tta ggc tgg ctt ggt gcc gcg att aac cca tta gtg 4711
Ser Ala Gln Ser Leu Gly Trp Leu Gly Ala Ala Ile Asn Pro Leu Val
                185                 190                 195

agt ggc gta gtg cta acc agc ctg ccg cct tcc tcg ctg ttt gtc atc 4759
Ser Gly Val Val Leu Thr Ser Leu Pro Pro Ser Ser Leu Phe Val Ile
            200                 205                 210

tta gcg ttg gtg atc att gct gcg tgg gtg ctg atg tta aaa ggg att 4807
Leu Ala Leu Val Ile Ile Ala Ala Trp Val Leu Met Leu Lys Gly Ile
        215                 220                 225

cga gca aga ccg tgg ggg cag ccc gcg ctt tgt tga tttaagtcga 4853
Arg Ala Arg Pro Trp Gly Gln Pro Ala Leu Cys *
230 235

acacaataaa gatttaattc agccttcgtt taggttacct ctgctaatat ctttctcatt 4913
gagatgaaaa ttaaggtaag cgaggaaaca caccacacca taaacggagg caaataatgc 4973
tgggtaatat gaatgttttt atggccgtac tgggaataat ttatttttct ggttttctgg 5033
ccgcgtattt cagccacaaa tgggatgact aatgaacgga gataatccct cacctaaccg 5093
gccccttgtt acagttgtgt acaaggggcc tgatttttat gacggcgaaa aaaaaccgcc 5153
agtaaaccgg cggtgaatgc ttgcatggat agatttgtgt tttgctttta cgctaacagg 5213
cattttcctg cactgataac gaatcgttga cacagtagca tcagttttct caatgaatgt 5273
taaacggagc ttaaactcgg ttaatcacat tttgttcgtc aataaacatg cagcgatttc 5333

ttccggtttg cttaccctca tacattgccc ggtccgctct tccaatgacc acatccagag 5393
gctcttcagg aaatgcgcga ctcacacctg ctgtcacggt aatgttgata tgcccttcag 5453
aatgtgtgat ggcatggtta tcgactaact ggcaaattct gacacctgca cgacatgctt 5513
cttcatcatt agccgctttg acaataatga taaattcttc gcccccgtag cgataaaccg 5573
tttcgtaatc acgcgtccaa ctggctaagt aagttgccag ggtgcgtaat actacatcgc 5633
cgattaaatg cccgtagtat cattaaccaa tttaaatcgg tcaatatcca acaacattaa 5693
ataaagattc agaggctcag cgttgcgtaa ctgatgatca aaggattcat caagaacccg 5753
acgacccggc aatcccgtca aaacatccat attgctacgg atcgtcagca aataaatttt 5813
gtaatcggtt aatgccgcag taaaagaaag caacccctcc tgaaaggcgt cgaaatgcgc 5873
gtcctgccag tgattttcaa caatagccag cattaattcc cgaccacagt tatgcatatg 5933
ttgatgggca gaatccatta gccgaacgta aggtaattca tcgttatcga gtggccccag 5993
atgatcaatc caccgaccaa actggcacag tccataagaa tggttatccg ttatttctgg 6053
cttactggca tctctcgcga ccacgctgtg aaacatactc accagccact ggtagtgggc 6113
atcgatagcc ttattgagat ttaacaagat ggcatcaatt tccgttgtct tcttgatcat 6173
tgccactcct ttttcacagt tccttgtgcg cgctattcta acgagagaaa agcaaattta 6233
cgtcaatatt ttcatagaaa tccgaagtta tgagtcatct ctgagataac attgtgattt 6293
aaaacaaaat cagcggataa aaaagtgttt aattctgtaa attacctctg cattatcgta 6353
aataaaagga tgacaaatag cataacccaa taccctaatg gcccagtagt tcaggccatc 6413
aggctaattt atttttattt ctgcaaatga gtgacccgaa cgacggccgg cgcgcttttc 6473
ttatccagac tgccactaat gttgatcatc tggtccggct gaacttctcg tccatcaaag 6533
acggccgcag gaataacgac attaatttca ccgctcttat cgcgaaaaac gtaacggtcc 6593
tctcctttgt gagaaatcaa attaccgcgt agtgaaaccg aagcgccatc gtgcatggtt 6653
tttgcgaaat caacggtcat tttttttgca tcatcggttc cgcgatagcc atcttctatt 6713
gcatgaggcg gcggtggcgc tgcatcctgt tttaaaccgc cctggtcatc tgccaacgca 6773
taaggcatga caagaaaact tgctaataca atggcctgaa atttcatact aactccttaa 6833
ttgcgtttgg tttgacttat taagtctggt tgctattttt ataattgcca aataagaata 6893
ttgccaattg ttataaggca tttaaaatca gccaactagc tgtcaaatat acagagaatt 6953
taactcacta aagttaagaa gattgaaaag tcttaaacat attttcagaa taatcggatt 7013
tatatgtttg aaaattatta tattggacga gcatacagaa aaagcaaatc acctttacat 7073
ataaaagcgt ggacaaaaaa cagtgaacat taatagagat aaaattgtac aacttgtaga 7133
taccgatact attgaaaacc tgacatccgc gttgagtcaa agacttatcg cggatcaatt 7193
acgcttaact accgccgaat catgcaccgg cggtaagttg gctagcgccc tgtgtgcagc 7253
tgaagataca cccaaatttt acggtgcagg ctttgttact ttcaccgatc aggcaaagat 7313
gaaaatcctc agcgtaagcc agcaatctct tgaacgatat tctgcggtga gtgagaaagt 7373
ggcagcagaa atggcaaccg gtgccataga gcgtgcggat gctgatgtca gtattgccat 7433
taccggctac ggcggaccgg agggcggtga agatggtacg ccagcgggta ccgtctggtt 7493
tgcgtggcat attaaaggcc agaactacac tgcggttatg cattttgctg gcgactgcga 7553
aacggtatta gctttagcgg tgaggtttgc cctcgcccag ctgctgcaat tactgctata 7613
accaggctgg cctggcgata tctcaggcca gccattggtg gtgtttatat gttcaagcca 7673
cgatgttgca gcatcggcat aatcttaggt gccttaccgc gccattgtcg atacaggcgt 7733
tccagatctt cgctgttacc tctggaaagg atcgcctcgc gaaaacgcag cccattttca 7793
cgcgttaatc cgccctgctc aacaaaccac tgataaccat catcggccaa catttgcgtc 7853
cacagataag cgtaataacc tgcag 7878

<210> 2
<211> 239
<212> PRT
<213> Echerichia coli

<400> 2

Met Gln Ser Ile Asn Leu Pro Phe Trp Leu Ala Ala Ile Cys Ser Ala
1               5                   10                  15
Phe Pro Met Leu Phe Ile Gln Ile Trp Val Lys Arg Ser Glu Lys Ile
            20                  25                  30
Ile Ala Thr Glu Thr Gly Ser Val Trp Ser Pro Lys Val Leu Leu Gln
        35                  40                  45
Asp Lys Ala Leu Leu Trp Phe Thr Cys Ser Gly Phe Leu Ala Ser Phe
    50                  55                  60

Val Ser Gly Ala Phe Ala Ser Cys Ile Ser Gln Tyr Val Met Val Ile
65                  70                  75                  80
Ala Asp Gly Asp Phe Ala Glu Lys Val Val Ala Val Val Leu Pro Val
                85                  90                  95
Asn Ala Ala Met Val Val Thr Leu Gln Tyr Ser Val Gly Arg Arg Leu
            100                 105                 110
Asn Pro Ala Asn Ile Arg Ala Leu Met Thr Ala Gly Thr Leu Cys Phe
        115                 120                 125
Val Ile Gly Leu Val Gly Phe Ile Phe Ser Gly Asn Ser Leu Leu Leu
    130                 135                 140
Trp Gly Met Ser Ala Ala Val Phe Thr Val Gly Glu Ile Ile Tyr Ala
145                 150                 155                 160
Pro Gly Glu Tyr Met Leu Ile Asp His Ile Ala Pro Pro Glu Met Lys
                165                 170                 175
Ala Ser Tyr Phe Ser Ala Gln Ser Leu Gly Trp Leu Gly Ala Ala Ile
            180                 185                 190
Asn Pro Leu Val Ser Gly Val Val Leu Thr Ser Leu Pro Pro Ser Ser
        195                 200                 205
Leu Phe Val Ile Leu Ala Leu Val Ile Ile Ala Ala Trp Val Leu Met
    210                 215                 220
Leu Lys Gly Ile Arg Ala Arg Pro Trp Gly Gln Pro Ala Leu Cys
225                 230                 235

<210> 3
<211> 870
<212> DNA
<213> Echerichia coli

<220>
<221> CDS

<222> (1)...(870)

<400> 3
atg gat cag gcc ggc att att cgc gac ctt tta atc tgg ctg gaa ggt     48
Met Asp Gln Ala Gly Ile Ile Arg Asp Leu Leu Ile Trp Leu Glu Gly
 1               5                  10                  15

cat ctg gat cag ccc ctg tcg ctc gac aat gta gcg gcg aaa gca ggt     96
His Leu Asp Gln Pro Leu Ser Leu Asp Asn Val Ala Ala Lys Ala Gly
            20                  25                  30

tat tcc aag tgg cac tta cag aga atg ttt aaa gat gtc act ggc cat    144
Tyr Ser Lys Trp His Leu Gln Arg Met Phe Lys Asp Val Thr Gly His
        35                  40                  45

gct att ggc gcg tat att cgt gct cgt cgt ttg tcg aaa tcg gcg gtc    192
Ala Ile Gly Ala Tyr Ile Arg Ala Arg Arg Leu Ser Lys Ser Ala Val
    50                  55                  60

gca cta cgc ctg act gcg cgt ccg att ctg gac atc gcg ctg caa tac    240
Ala Leu Arg Leu Thr Ala Arg Pro Ile Leu Asp Ile Ala Leu Gln Tyr
65                  70                  75                  80

cgc ttc gac tct caa cag aca ttt acc cgc gca ttc aag aag cag ttt    288
Arg Phe Asp Ser Gln Gln Thr Phe Thr Arg Ala Phe Lys Lys Gln Phe
                85                  90                  95

gcc cag act cct gca ctt tac cgc cgt tct cct gaa tgg agc gcc ttt     336
Ala Gln Thr Pro Ala Leu Tyr Arg Arg Ser Pro Glu Trp Ser Ala Phe
            100                 105                 110

ggt att cgc ccg ccg cta cgc ctg ggt gaa ttc act atg cca gag cac     384
Gly Ile Arg Pro Pro Leu Arg Leu Gly Glu Phe Thr Met Pro Glu His
        115                 120                 125

aaa ttt gtc acc ctg gaa gat acg ccg ctg att ggt gtt acc cag agc     432
Lys Phe Val Thr Leu Glu Asp Thr Pro Leu Ile Gly Val Thr Gln Ser
    130                 135                 140

tac tcc tgt tcg ctg gag caa atc tct gat ttc cgc cat gaa atg cgt     480
Tyr Ser Cys Ser Leu Glu Gln Ile Ser Asp Phe Arg His Glu Met Arg
145                 150                 155                 160

tat cag ttc tgg cac gat ttt ctc ggc aac gcg ccg acc att ccg ccg     528
Tyr Gln Phe Trp His Asp Phe Leu Gly Asn Ala Pro Thr Ile Pro Pro
                165                 170                 175

gtg ctc tac ggc ctg aat gaa acg cgt ccg agt cag gat aaa gac gac     576
Val Leu Tyr Gly Leu Asn Glu Thr Arg Pro Ser Gln Asp Lys Asp Asp
            180                 185                 190

gag caa gag gta ttc tat acc acc gcg tta gcc cag gat cag gca gat     624
Glu Gln Glu Val Phe Tyr Thr Thr Ala Leu Ala Gln Asp Gln Ala Asp
        195                 200                 205

ggc tat gta ctg acg ggg cat ccg gtg atg ctg cag ggc ggc gaa tat     672
Gly Tyr Val Leu Thr Gly His Pro Val Met Leu Gln Gly Gly Glu Tyr
    210                 215                 220

gtg atg ttt acc tat gaa ggt ctg gga acc ggc gtg cag gag ttt atc     720
Val Met Phe Thr Tyr Glu Gly Leu Gly Thr Gly Val Gln Glu Phe Ile
225                 230                 235                 240

ctg acg gta tac gga acg tgc atg cca atg ctc aac ctg acg cgc cgt     768
Leu Thr Val Tyr Gly Thr Cys Met Pro Met Leu Asn Leu Thr Arg Arg
                245                 250                 255

aaa ggt cag gat att gag cga tac tac ccg gca gaa gat gcc aaa gcg     816
Lys Gly Gln Asp Ile Glu Arg Tyr Tyr Pro Ala Glu Asp Ala Lys Ala
            260                 265                 270

gga gat cgc cca att aat cta cgc tgt gaa ctg ctg att ccg atc cgt     864
Gly Asp Arg Pro Ile Asn Leu Arg Cys Glu Leu Leu Ile Pro Ile Arg
        275                 280                 285

cgt taa                                                             870
Arg  *

<210> 4
<211> 289
<212> PRT
<213> Echerichia coli

<400> 4

Met Asp Gln Ala Gly Ile Ile Arg Asp Leu Leu Ile Trp Leu Glu Gly
1               5                   10                  15
His Leu Asp Gln Pro Leu Ser Leu Asp Asn Val Ala Ala Lys Ala Gly
            20                  25                  30
Tyr Ser Lys Trp His Leu Gln Arg Met Phe Lys Asp Val Thr Gly His
        35                  40                  45
Ala Ile Gly Ala Tyr Ile Arg Ala Arg Arg Leu Ser Lys Ser Ala Val
    50                  55                  60
Ala Leu Arg Leu Thr Ala Arg Pro Ile Leu Asp Ile Ala Leu Gln Tyr
65                  70                  75                  80
Arg Phe Asp Ser Gln Gln Thr Phe Thr Arg Ala Phe Lys Lys Gln Phe
                85                  90                  95
Ala Gln Thr Pro Ala Leu Tyr Arg Arg Ser Pro Glu Trp Ser Ala Phe
            100                 105                 110
Gly Ile Arg Pro Pro Leu Arg Leu Gly Glu Phe Thr Met Pro Glu His
        115                 120                 125
Lys Phe Val Thr Leu Glu Asp Thr Pro Leu Ile Gly Val Thr Gln Ser
    130                 135                 140
Tyr Ser Cys Ser Leu Glu Gln Ile Ser Asp Phe Arg His Glu Met Arg
145                 150                 155                 160
Tyr Gln Phe Trp His Asp Phe Leu Gly Asn Ala Pro Thr Ile Pro Pro
                165                 170                 175
Val Leu Tyr Gly Leu Asn Glu Thr Arg Pro Ser Gln Asp Lys Asp Asp
            180                 185                 190
Glu Gln Glu Val Phe Tyr Thr Thr Ala Leu Ala Gln Asp Gln Ala Asp
        195                 200                 205
Gly Tyr Val Leu Thr Gly His Pro Val Met Leu Gln Gly Gly Glu Tyr
    210                 215                 220
Val Met Phe Thr Tyr Glu Gly Leu Gly Thr Gly Val Gln Glu Phe Ile
225                 230                 235                 240
Leu Thr Val Tyr Gly Thr Cys Met Pro Met Leu Asn Leu Thr Arg Arg
                245                 250                 255
Lys Gly Gln Asp Ile Glu Arg Tyr Tyr Pro Ala Glu Asp Ala Lys Ala
            260                 265                 270

Gly Asp Arg Pro Ile Asn Leu Arg Cys Glu Leu Leu Ile Pro Ile Arg
        275                 280                 285
Arg